# EUROPEAN PATENT SPECIFICATION

(11) **EP 2 718 428 B1**
(45) Date of publication and mention of the grant of the patent: **07.03.2018**
(21) Application number: 12801020.4
(22) Date of filing: 13.06.2012
(51) Int. Cl.: C12N 15/82, A61K 39/205, A61K 39/12

(54) **RABIES VIRUS LIKE PARTICLE PRODUCTION IN PLANTS**
HERSTELLUNG RABIESVIRUS-ÄHNLICHER PARTIKEL IN PFLANZEN
PRODUCTION DE PARTICULES DE TYPE VIRUS DE LA RAGE DANS DES PLANTES

(30) Priority: 13.06.2011 US 201161496371 P; 21.12.2011 US 201161578787 P
(43) Date of publication of application: 16.04.2014
(73) Proprietor: Medicago Inc., Québec, Québec G1V 3V9 (CA)
(72) Inventor: D'AOUST, Marc-Andre, Quebec Quebec G1X 4N4 (CA); LAVOIE, Pierre-Olivier, Quebec Quebec G1N 2I7 (CA); VEZINA, Louis-Philippe, Neuville Quebec G0A 2R0 (CA); COUTURE, Manon, St. Augustin de Desmaures Quebec G3C 2A5 (CA)
(74) Representative: HGF Limited
(86) International application number: PCT/CA2012/000581
(87) International publication number: WO 2012/171104

(56) References cited:
- WO-A1-97/43428
- WO-A1-2009/009876
- WO-A1-2011/035422
- WO-A1-2012/083445
- WO-A2-2006/016380
- WO-A2-2006/016380
- WO-A2-2012/061815
- WO-A2-2012/061815
- D'AOUST MARC-ANDRÉ ET AL: "Influenza virus-like particles produced by transient expression in Nicotiana benthamiana induce a protective immune response against a lethal viral challenge in mice", PLANT BIOTECHNOLOGY JOURNAL, BLACKWELL, OXFORD, GB, vol. 6, no. 9, 1 December 2008 (2008-12-01), pages 930-940, XP002598510, ISSN: 1467-7644
- HAYNES JOEL R: "Influenza virus-like particle vaccines", EXPERT REVIEW OF VACCINES ENGLAND, EXPERT REVIEWS LTD, GB, vol. 8, no. 4, 1 April 2009 (2009-04-01), pages 435-445, XP009128169, ISSN: 1744-8395, DOI: 10.1586/ERV.09.8
- D'AOUST MARC-ANDRE ET AL: "The production of hemagglutinin-based virus-like particles in plants: a rapid, efficient and safe response to pandemic influenza", PLANT BIOTECHNOLOGY JOURNAL, BLACKWELL, OXFORD, GB, vol. 8, no. 5, 1 June 2010 (2010-06-01), pages 607-619, XP002598511, ISSN: 1467-7644, DOI: 10.1111/J.1467-7652.2009.00496.X
- GRGACIC E V L ET AL: "Virus-like particles: Passport to immune recognition", METHODS, ACADEMIC PRESS, vol. 40, no. 1, 1 September 2006 (2006-09-01), pages 60-65, XP024908472, ISSN: 1046-2023, DOI: 10.1016/J.YMETH.2006.07.018 [retrieved on 2006-09-01]
- MODELSKA ET AL.: 'Immunization against rabies with plant-derived antigen' PROCEEDINGS OF THE NATIONAL ACADEMY OF SCIENCES USA vol. 95, March 1998, pages 2481 - 2485, XP002921640

## Description

### FIELD OF INVENTION

The present invention relates to producing native viral proteins in plants. More specifically, the present invention also relates to producing virus-like particles comprising native rabies virus structural protein in plants.

### BACKGROUND OF THE INVENTION

Vaccination provides protection against disease caused by an infectious agent by inducing a subject to mount a defense prior to infection. Conventionally, this has been accomplished through the use of live attenuated or whole inactivated forms of the infectious agents as immunogens. To avoid the danger of using the whole virus (such as killed or attenuated viruses) as a vaccine, recombinant viral proteins, for example subunits, have been pursued as vaccines. Both peptide and subunit vaccines are subject to a number of potential limitations. Subunit vaccines may exhibit poor immunogenicity, owing to incorrect folding, poor antigen presentation, or differences in carbohydrate and lipid composition. A major problem is the difficulty of ensuring that the conformation of the engineered proteins mimics that of the antigens in their natural environment. Suitable adjuvants and, in the case of peptides, carrier proteins, must be used to boost the immune response. In addition these vaccines elicit primarily humoral responses, and thus may fail to evoke effective immunity. Subunit vaccines are often ineffective for diseases in which whole inactivated virus can be demonstrated to provide protection.

Virus-like particles (VLPs) are potential candidates for inclusion in immunogenic compositions. VLPs closely resemble mature virions, but they do not contain viral genomic material. Therefore, VLPs are nonreplicative in nature, which make them safe for administration as a vaccine. In addition, VLPs can be engineered to express viral glycoproteins on the surface of the VLP, which is their most native physiological configuration. Moreover, since VLPs resemble intact virions and are multivalent particulate structures, VLPs may be more effective in inducing neutralizing antibodies to the glycoprotein than soluble envelope protein antigens.

To date, VLPs have been produced for more than 30 different viruses that infect humans and other animals. One of the most striking features of this group is that it is extremely diverse in terms of the structure of the individual viruses. It includes viruses that have a single capsid protein, multiple capsid proteins, and those with and without lipid envelopes.

The formation of VLPs for viruses with a lipid envelope has a different type of technical challenge than those produced for viruses with multiple capsids. For these viruses, the choice of expression system can be important for the efficiency of VLP formation. For example, Hantaan virus readily forms VLPs when expressed in mammalian cells from a vaccinia-virus-based vector, but VLP formation is relatively inefficient in insect cells (Betenbaugh M et al. 1995, Virus Res. 38, 111-124).

The rabies virus (RV) is a member of the family Rhabdoviridae. Like most members of this family, RV is a non-segmented, negative stranded RNA virus whose genome codes for five viral proteins: RNA-dependent RNA polymerase (L); a nucleoprotein (N); a phosphorylated protein (P); a matrix protein (M) located on the inner side of the viral protein envelope; and an external surface glycoprotein (G). (Dietzschold B et al., 1991, Crit. Rev. Immunol. 10: 427-439.)

Cell-cultured based vaccines for rabies are limited to growing inactivated strains of the virus in cell cultures. These vaccines comprise the virus grown in cell cultures. Current biotechnological approaches aim at expressing the coat protein gene of the rabies virus to develop a safe recombinant protein that could be deployed as an active vaccine. Stable expression of rabies virus glycoprotein has been shown in Chinese Hamster Ovary cells (Burger et al., 1991, J Gen Virol., Feb; 72 (Pt 2):359-67). A full length, glycosylated protein of 67 K that co-migrated with the G-protein isolated from virus-infected cells, was obtained.

Expression of rabies glycoprotein gene by baculoviral vectors in insect cells gives yields of protein to the extent of 18% of total cellular protein, 48 h post infection. Prehaud D H et al, (1989, Virology, Dec;173(2):390-9) describe placing a sequence encoding the G protein of CVS strain under control of the AcNPV polyhedrin promoter and expressing the construct using a *Spodoptera fugiperda* cell-line. The insect derived protein exhibited altered electrophoretic mobility compared to the wild type due to differences in the glycan components.

Rupprecht et al (1993, Vaccine, 11(9):925-8) demonstrate that a glycoprotein (ERA strain) derived from recombinant baculovirus-infected insect cells was efficacious as an oral vaccine in raccoons. WO/1993/001833 teaches the production of a virus like particles (VLPs) in a baculovirus expression system containing an RNA genome including a 3' domain and a filler domain surrounded by a sheath of rabies N protein and rabies M protein. The VLP also includes a lipid envelope of rabies G protein. In view of relatively high costs of the insect and mammalian cell-systems these are not the systems of choice for G protein expression as a strategy to develop vaccine against rabies.

McGarvey et al. (1995, Bio/Technology Vol. 13, No. 13, pp. 1484-1487) describe the transformation of tomato cotyledons using a full length cDNA encoding glycoprotein G of rabies virus (ERA strain) under the control of the 35S' promoter of cauliflower mosaic virus. The protein was expressed in tomato and was characterized as having a molecular weight of 62 and 60 kDa in western blot after immunoprecipitation, as compared to 66 kDa observed for G protein from virus grown in BHK cells. The difference in molecular weight compared to the natural glycoprotein was suggested to result from post-translational modification of the protein (proteolytic cleavage and/or modified glycosylation). The amount of G protein immunoprecipitated was found to be approximately 1-10 ng/mg of soluble protein i.e. at 0.0001% to 0.001% of soluble protein. The low expression level may have been due to using a poorly designed gene. For example, a native G protein coding gene was used along with its native signal peptide.

Plant derived immune response against diseases such as mink enteritis and rabies were reported by expressing viral epitopes on the surface of plant viruses, followed by infection of susceptible host with the recombinant modified virus (Modelska et al., 1998, Proc. Natl. Acad. Sci. USA 95: 2481-2485; Yusibov et al., 2002, Vaccine 20:3155-3164). The size of the antigen polypeptide expressed on surface of a vector virus was limited to 37 amino acids, and required epitope mapping of the antigen. Such thorough knowledge of the antigen is not generally available, especially with newly discovered diseases where the expression of full-length proteins may be the only option. I some cases, multiple epitopes may be required to give acceptable protection against challenge by the pathogenic virus. Furthermore, containment could be considered as a significant problem at the agricultural level, especially when environmentally stable plant viruses for example, Tobacco Mosaic Virus, are used.

WO 97/43428 teaches a method for producing the glycoprotein G of the rabies virus, or a virus related to the rabies virus, in plants. The construct comprised a sequence encoding a chimeric G protein comprising a mature viral protein G with a N-terminal signal peptide other than that naturally associated with the viral protein G. The glycoprotein had a molecular weight of approximately 66 kDa and was highly insoluble. Detergents, such as SDS, or Triton X-100, were necessary to extract and solubilize the glycoprotein. The authors concluded that the "insoluble" glycoprotein was related to the presence of the C-terminal transmembrane domain (the region located about 40 to 60 amino acids of the carboxy terminus of the glycoprotein) which is important for a protective response when the glycoprotein is used as vaccine.

Enveloped viruses may obtain their lipid envelope when 'budding' out of the infected cell and obtain the membrane from the plasma membrane, or from that of an internal organelle. For example, during the assembly process in rhabdoviruses, the N-P-L complex encapsulates negative-stranded genomic RNA to form the RNP core. The M protein forms a capsule, or matrix, around the RNP, and the RNP-M complex migrates to an area of the plasma membrane containing glycoprotein inserts. The M-protein initiates coiling, and the M-RNP complex binds with the glycoprotein, whereupon the completed virus buds from the plasma membrane.

Within the central nervous system (CNS), there is preferential viral budding from plasma membranes. Conversely, virus in the salivary glands buds primarily from the cell membrane into the acinar lumen. Viral budding into the salivary gland and virus-induced aggressive biting-behavior in the host animal maximize chances of viral infection of a new host. In mammalian or baculovirus cell systems, for example, rabies buds from the plasma membrane. Only a few enveloped viruses are known to infect plants (for example, members of the Topoviruses and Rhabdoviruses). Of the known plant enveloped viruses, they are characterized by budding from internal membranes of the host cell, and not from the plasma membrane. However, recombinant VLPs have been produced in plant hosts from the plasma membrane (WO 2011/035422).

Assembly/budding in rhabdoviruses is driven largely by the matrix (M) protein. The M protein contains a late budding domain that mediates the recruitment of host proteins linked to the vacuolar protein sorting pathway of the cell to facilitate virus-cell separation. Without wishing to be bound by theory, budding of enveloped viruses from cellular membranes is believed to depend on the presence of transmembrane spike proteins interacting with cytoplasmic virus components. For example, cells infected with rabies virus mutants that were deficient for the glycoprotein G, or the G cytoplasmic tail, released spikeless rhabdovirus particles, demonstrating that the viral surface protein is not required to drive the budding process. (Mebatsion T. et al., 1996, Cell, Mar 22:84(6):941-51). In contrast, infectious particles produced within M-protein deficient rabies virus mutants were mainly cell associated, and the yield of cell-free infectious virus was reduced by as much as 500,000-fold. This demonstrates the significant role of the M protein in virus budding. Supernatants from cells infected with the M-deficient rabies virus comprised long, rod-shaped virions, rather than the typical bullet-shaped rhabdovirus particles, further confirming impairment of the virus formation process. Complementation with M protein expressed from plasmids rescued rhabdovirus formation. The M protein therefore appears to play an important role in condensing and targeting the RNP to the plasma membrane as well as in incorporation of G protein into budding virions. (Mebatsion T. et al., 1999, J Virol Jan; 73(1):242-50).

### SUMMARY OF THE INVENTION

The present invention relates to producing native viral proteins in plants. More specifically, the present invention also relates to producing virus-like particles comprising native rabies virus structural protein in plants.
According to the present invention there is provided a method of producing a rabies virus like particle (VLP) in a plant according to appended claims 1 to 5 and 11 to 16; a plant derived VLP produced by the method of any one of claims 1-5, as set out in appended claims 6 and 7; a composition comprising an effective dose of the plant derived VLP of claim 6 or 7 for inducing an immune response in a subject, and a pharmaceutically acceptable carrier, as set out in appended claim 8; the plant derived VLP of claim 6 or 7 for use in inducing immunity to a rabies virus infection in a subject, as set out in claim 9; and a food supplement comprising the plant derived VLP of claim 6 or 7, as set out in claim 10.

The first regulatory region active in the plant, and the second regulatory region active in the plant may be the same or different.

In the method of the invention, the one or more than one geminivirus amplification element may be selected from a Bean Yellow Dwarf Virus long intergenic region (BeYDV LIR), and a BeYDV short intergenic region (BeYDV SIR).

In the methods of the invention plant or portion of the plant may further comprise another nucleic acid sequence encoding a suppressor of silencing, for example HcPro or p19, a geminivirus replicase or both. Alternatively, the plant or portion of the plant may comprise yet another nucleic acid encoding the geminivirus replicase.

The present invention also includes the method as described above, wherein the plant or portion of the plant transiently expressed the first nucleic acid. Alternatively, the first nucleic acid is stably expressed in the plant or portion of the plant.
In the VLP of the present invention the one or more native rabies virus structural protein the VLP may comprise plant-specific N-glycans, or modified N-glycans. There is also provided a polyclonal antibody prepared using the VLP.

There is also provided a method of inducing immunity to a rabies virus infection in a subject, comprising administering the VLP as just described, to the subject. The VLP may be administered to a subject orally, intradermally, intranasally, intramusclarly, intraperitoneally, intravenously, or subcutaneously.

There is also provided plant matter comprising a VLP produced by the method (A) and/or (B) described above. The plant matter may be used in inducing immunity to a rabies virus infection in a subject. The plant matter may also be admixed as a food supplement.

This summary of the invention does not necessarily describe all features of the invention.

### BRIEF DESCRIPTION OF THE DRAWINGS

These and other features of the invention will become more apparent from the following description in which reference is made to the appended drawings wherein:

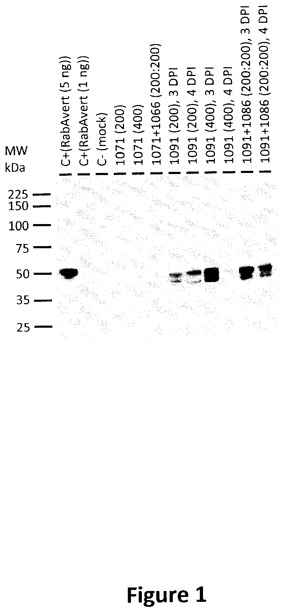

**Figure 1** shows a Western blot analysis of transient rabies G protein expression in *Nicotiana benthamiana.* Rabies G protein was expressed under the control of CPMV-HT with (1091) or without (1071) BeYDV-based DNA amplification system (see table 2 in the Examples for constructs). Numbers in parenthesis refer to the amount of *Agrobacterium* culture, in milliliters, used in the preparation of the bacterial inoculum. Plants infiltrated with AGL1/1091 were harvest 3 or 4 days post-infiltration (DPI). Leaves of infiltrated plants were harvested and extracted mechanically. Protein extracts were separated by SDS-PAGE and analyzed by western blot using anti-rabies G mouse monoclonal antibodies (Santa-Cruz SC-57995).

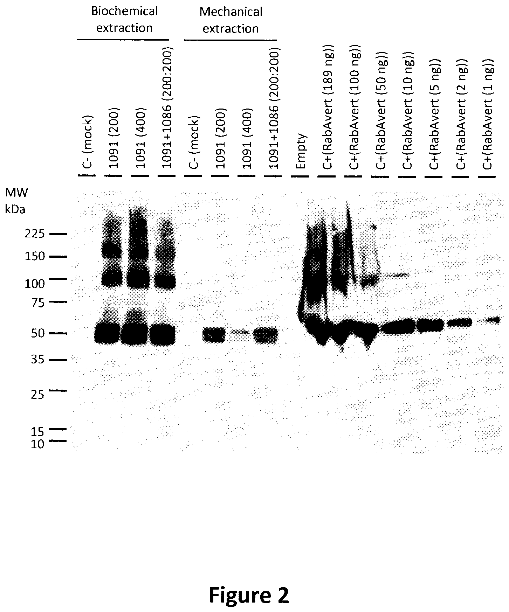

**Figure 2** shows a comparison of rabies G protein content in protein extracts from biochemical and mechanical extraction methods for rabies G protein. Protein extracts were separated by SDS-PAGE and analyzed by western blot using anti-rabies G mouse monoclonal antibodies (Santa-Cruz SC-57995). Numbers in parenthesis refer to the amount of *Agrobacterium* culture, in milliliters, used in the preparation of the bacterial inoculum (see table 2 for constructs).

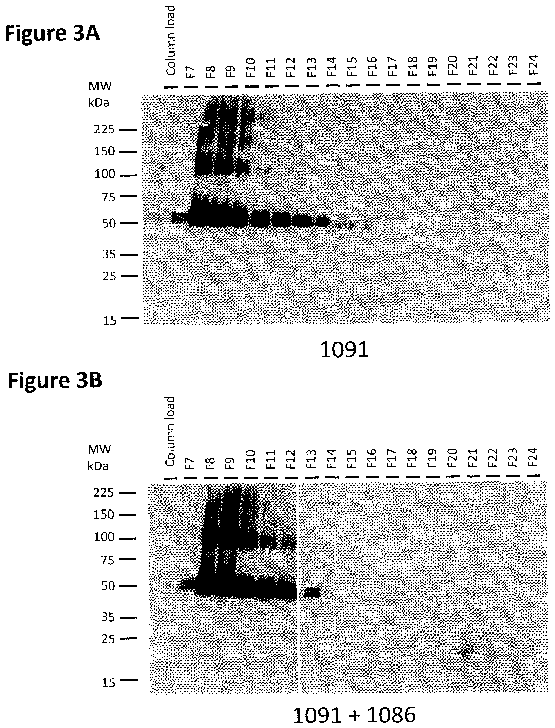

**Figure 3A** shows a western blot analysis of rabies G protein content after separation, by size exclusion chromatography (SEC), of concentrated protein extracts from plants infiltrated with AGL1/1091. Elution fractions from SEC were separated by SDS-PAGE and analyzed by western blot using anti-rabies protein G mouse monoclonal antibodies (Santa-Cruz SC-57995). **Figure 3B** shows a western blot analysis of rabies G protein content after separation, by size exclusion chromatography (SEC), of concentrated protein extracts from plants infiltrated with AGL1/1091 +AGL1/1086. Elution fractions from SEC were separated by SDS-PAGE and analyzed by western blot using anti-rabies protein G mouse monoclonal antibodies (Santa-Cruz SC-57995).

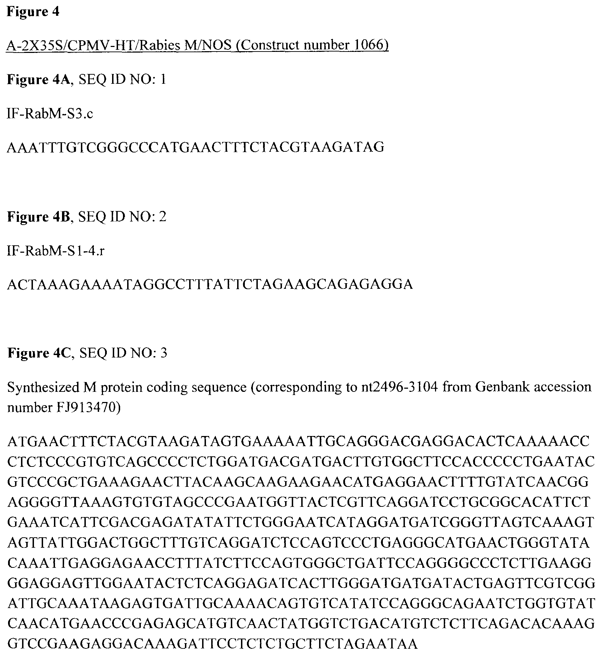

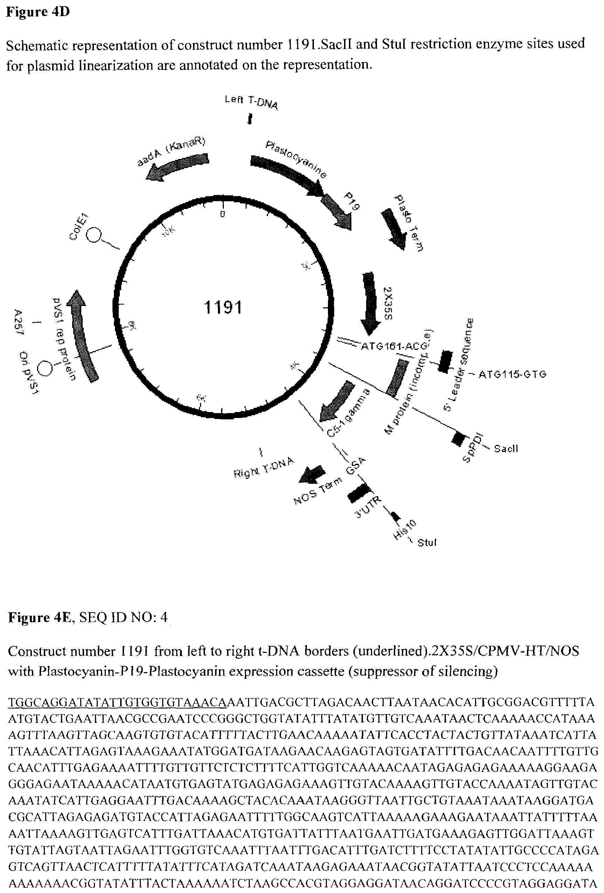

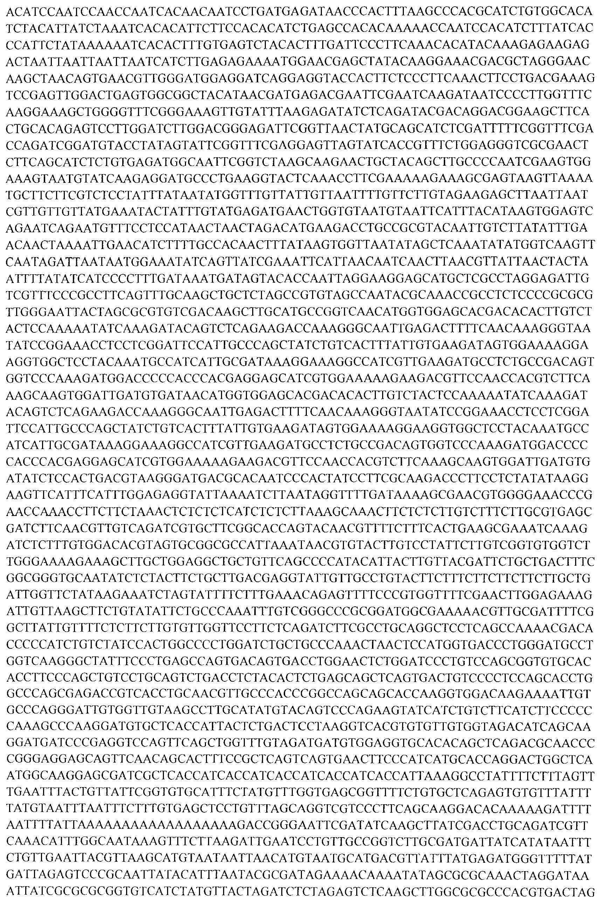

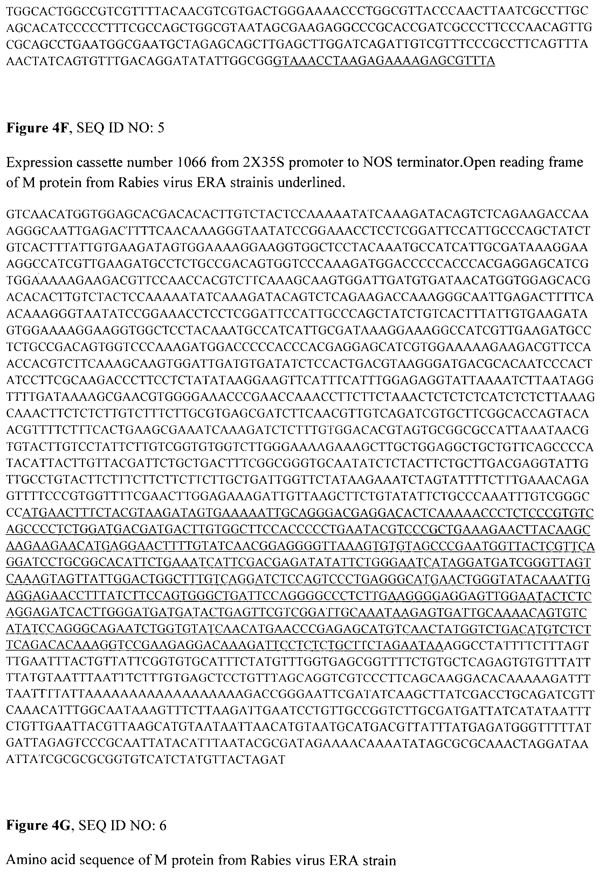

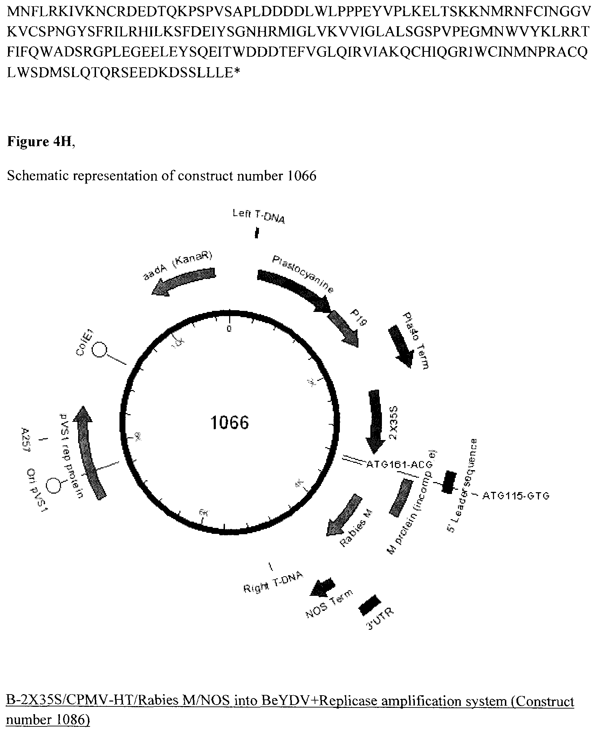

**Figure 4A** shows primer IF-RabM-S3.c (SEQ ID NO:1). **Figure 4B** shows primer IF-RabM-S1-4.r (SEQ ID NO: 2). **Figure 4C** shows synthesized M protein coding sequence (corresponding to nt 2496-3104 from Genbank accession number FJ913470) (SEQ ID NO. 3). **Figure 4D** shows a schematic representation of construct number 1191. SacII and StuI restriction enzyme sites used for plasmid linearization are annotated on the representation. **Figure 4E** shows construct number 1191 from left to right t-DNA borders (underlined). 2X35S/CPMV-HT/NOS with Plastocyanin-P19-Plastocyanin expression cassette (suppressor of silencing) (SEQ ID NO: 4). **Figure 4F** shows expression cassette number 1066 from 2X35S promoter to NOS terminator. The open reading frame of M protein from Rabies virus ERA strains is underlined (SEQ ID NO: 5). **Figure 4G** shows the amino acid sequence of M protein from Rabies virus ERA strain (SEQ ID NO: 6). **Figure 4H** shows the schematic representation of construct number 1066.

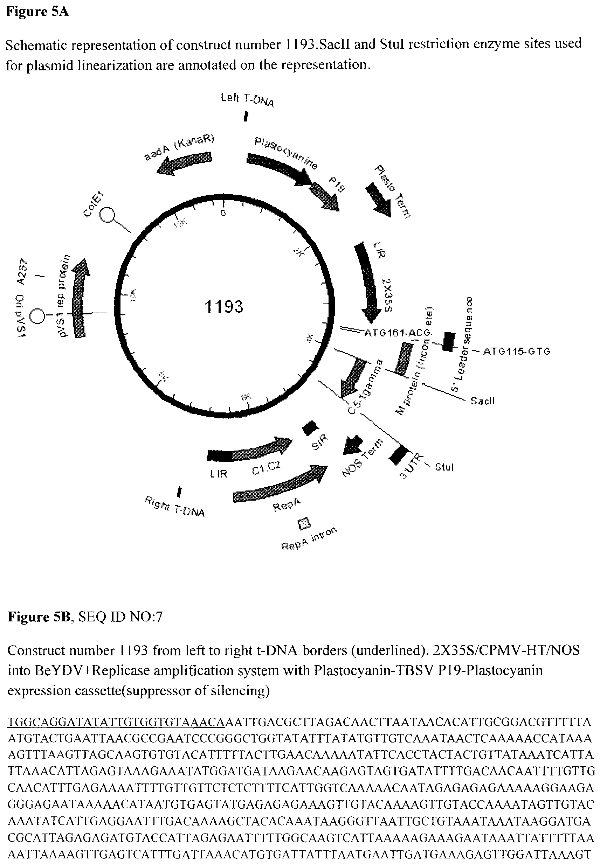

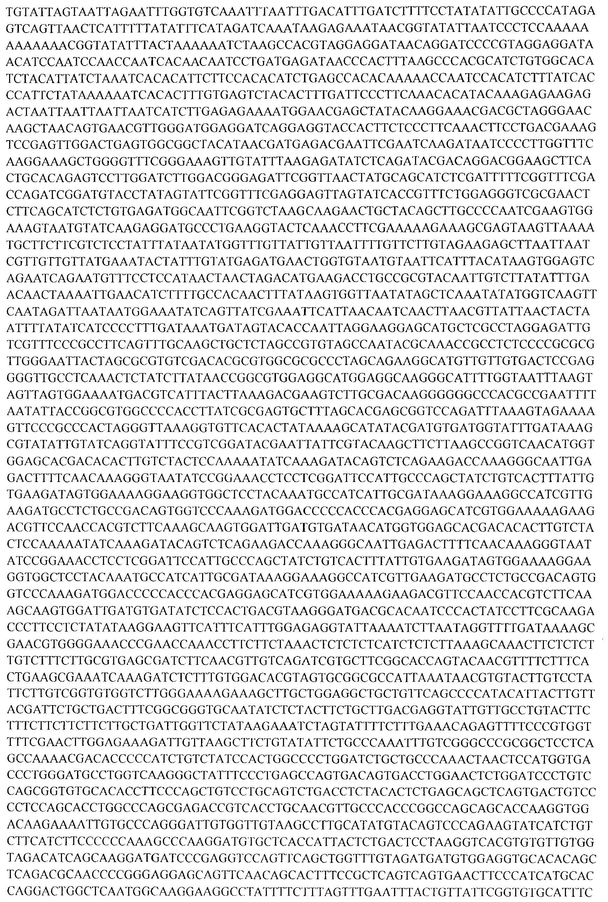

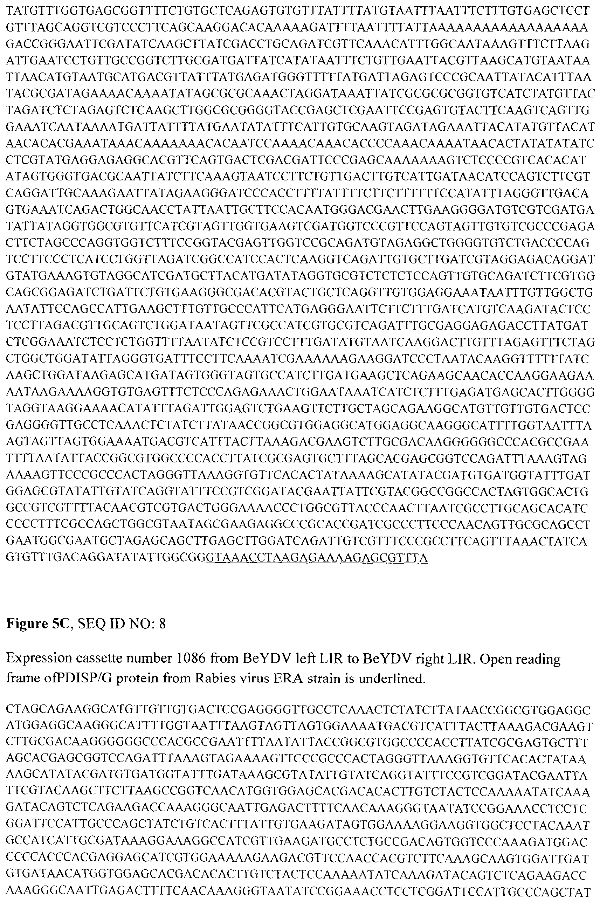

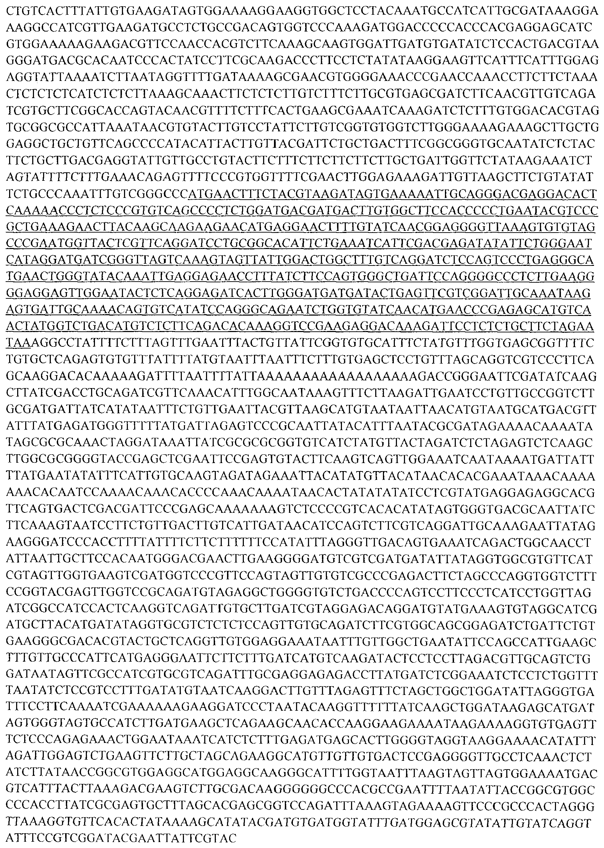

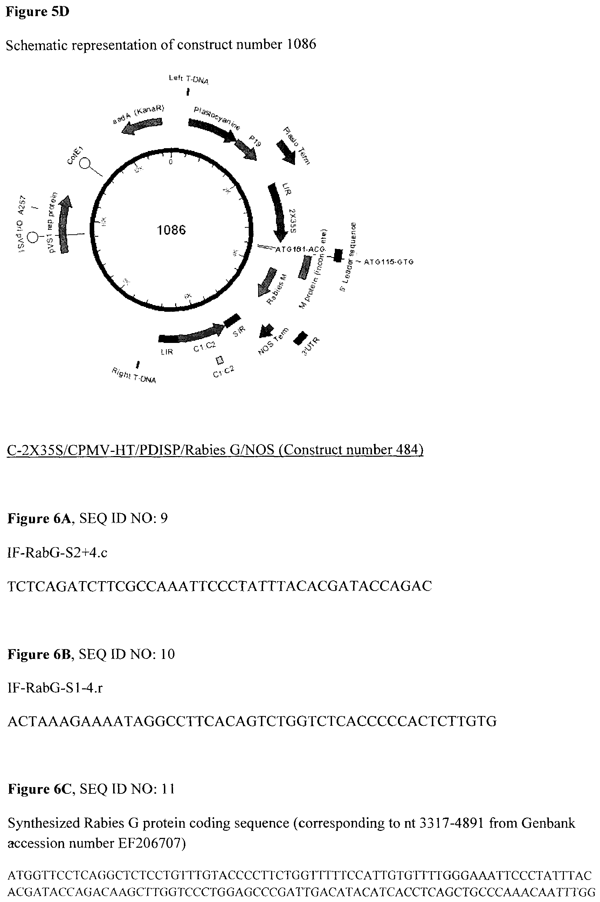

**Figure 5A** shows a schematic representation of construct number 1193. SacII and StuI restriction enzyme sites used for plasmid linearization are annotated on the representation. **Figure 5B** shows construct number 1193 from left to right t-DNA borders (underlined). 2X35S/CPMV-HT/NOS into BeYDV+Replicase amplification system with Plastocyanin-TBSV P19-Plastocyanin expression cassette (suppressor of silencing) (SEQ ID NO:7). **Figure 5C** shows expression cassette number 1086 from BeYDV left LIR to BeYDV right LIR. Open reading frame of PDISP/G protein from Rabies virus ERA strain is underlined. (SEQ ID NO: 8). **Figure 5D** shows a schematic representation of construct number 1086.

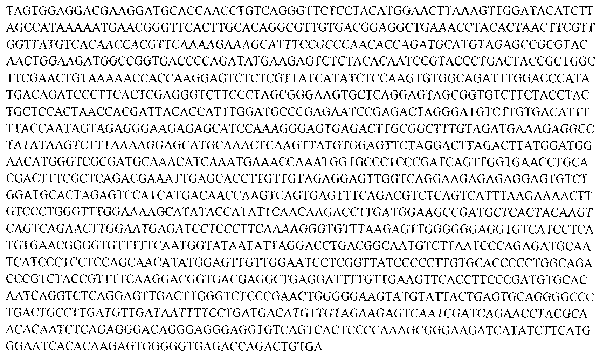

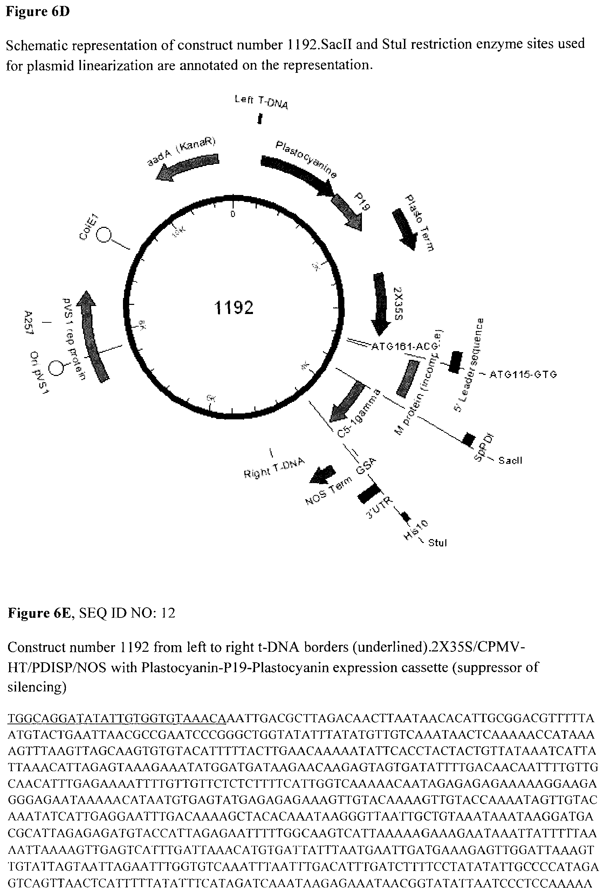

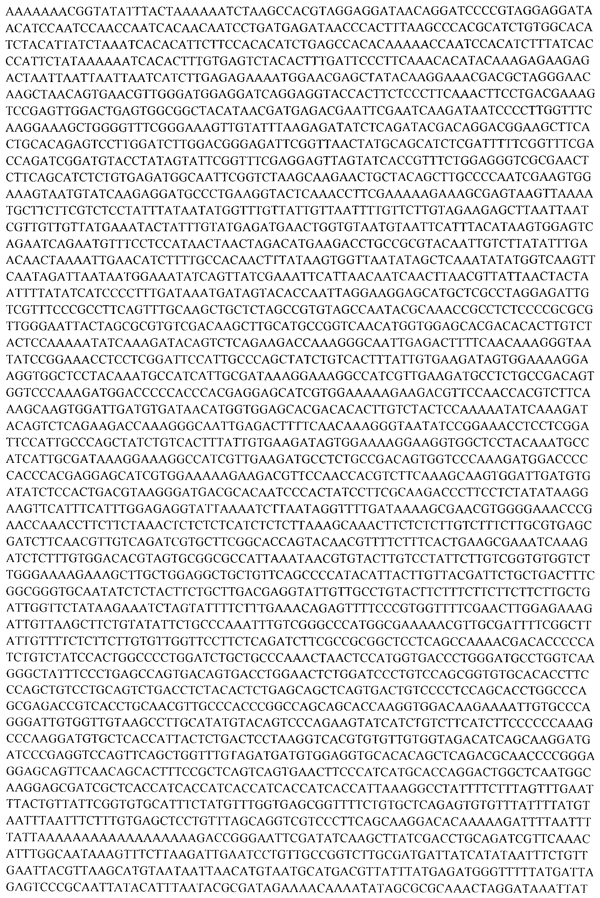

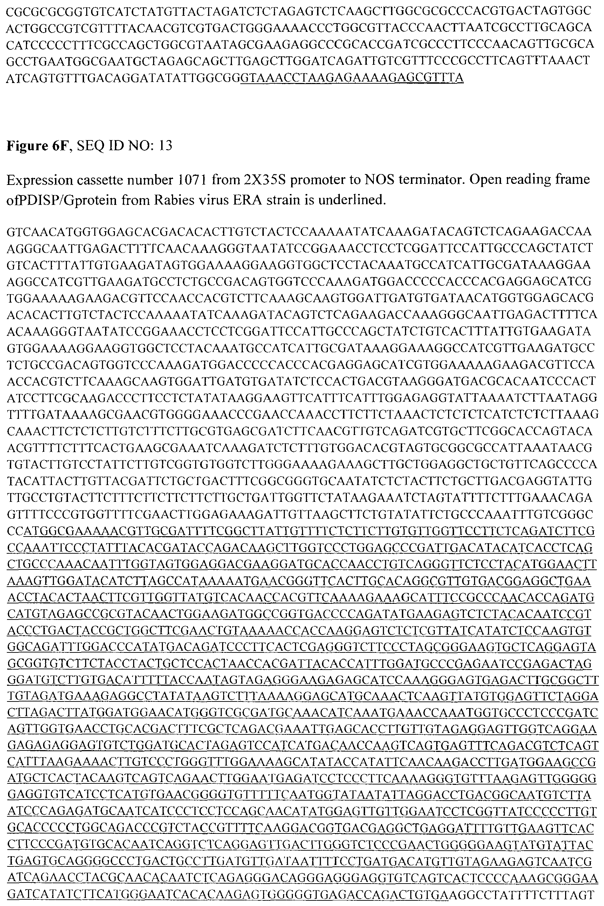

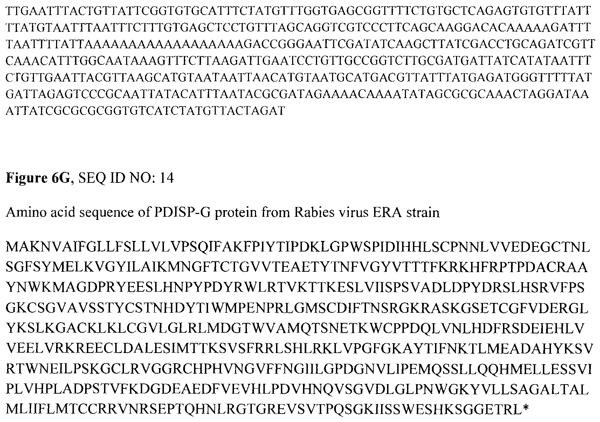

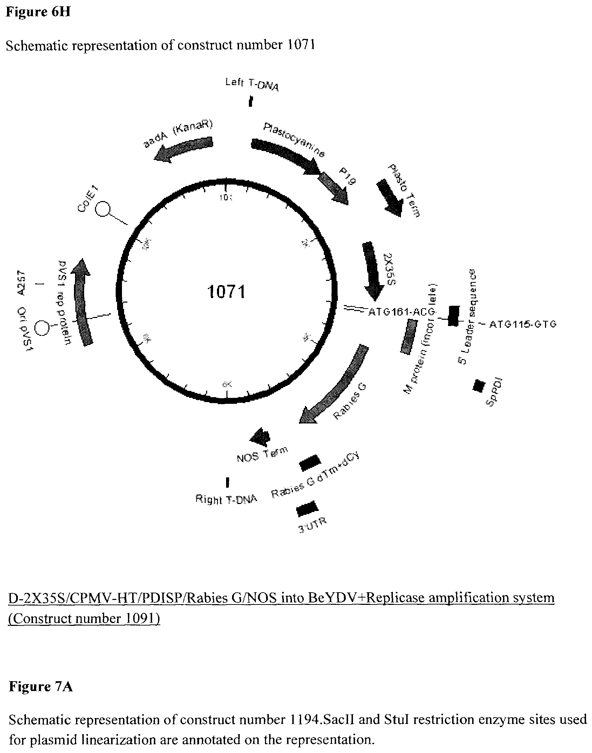

**Figure 6A** shows primer IF-RabG-S2+4.c (SEQ ID NO: 9). **Figure 6B** shows primer IF-RabG-S1-4.r (SEQ ID NO:10). **Figure 6C** shows synthesized Rabies G protein coding sequence (corresponding to nt 3317-4891 from Genbank accession number EF206707) (SEQ ID NO: 11). **Figure 6D** shows a schematic representation of construct number 1192. SacII and StuI restriction enzyme sites used for plasmid linearization are annotated on the representation. **Figure 6E** shows construct number 1192 from left to right t-DNA borders (underlined). 2X35S/CPMV-HT/PDISP/NOS with Plastocyanin-P19-Plastocyanin expression cassette (suppressor of silencing) (SEQ ID NO: 12). **Figure 6F** shows expression cassette number 1071 from 2X35S promoter to NOS terminator. Open reading frame of PDISP/G protein from Rabies virus ERA strain is underlined. (SEQ ID NO: 13). **Figure 6G** shows amino acid sequence of PDISP-G protein from Rabies virus ERA strain (SEQ ID NO: 14). **Figure 6H** shows a schematic representation of construct number 1071.

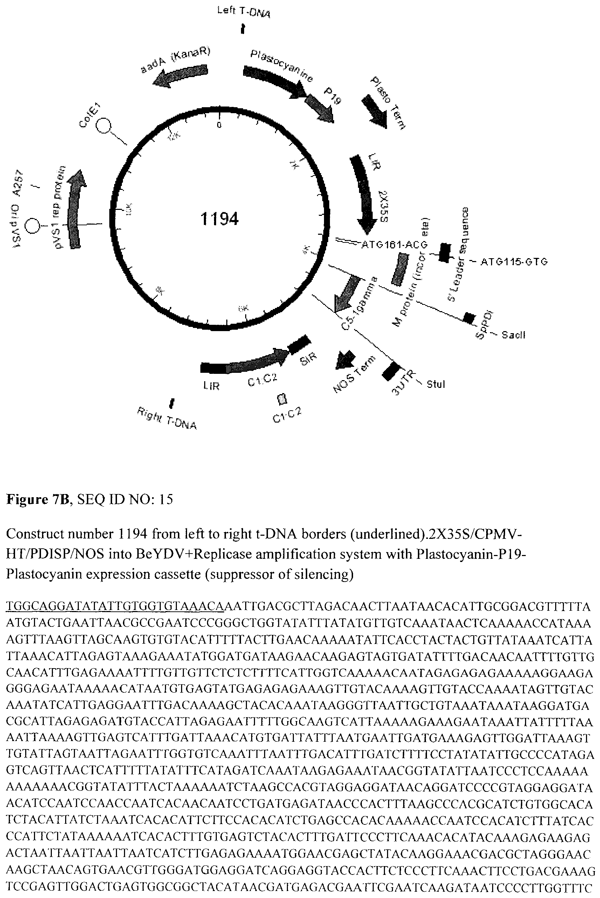

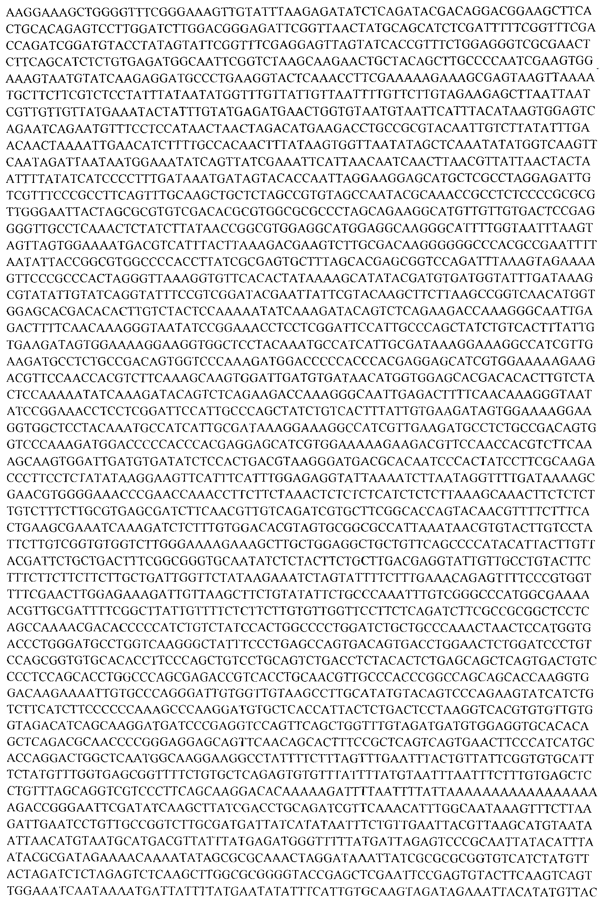

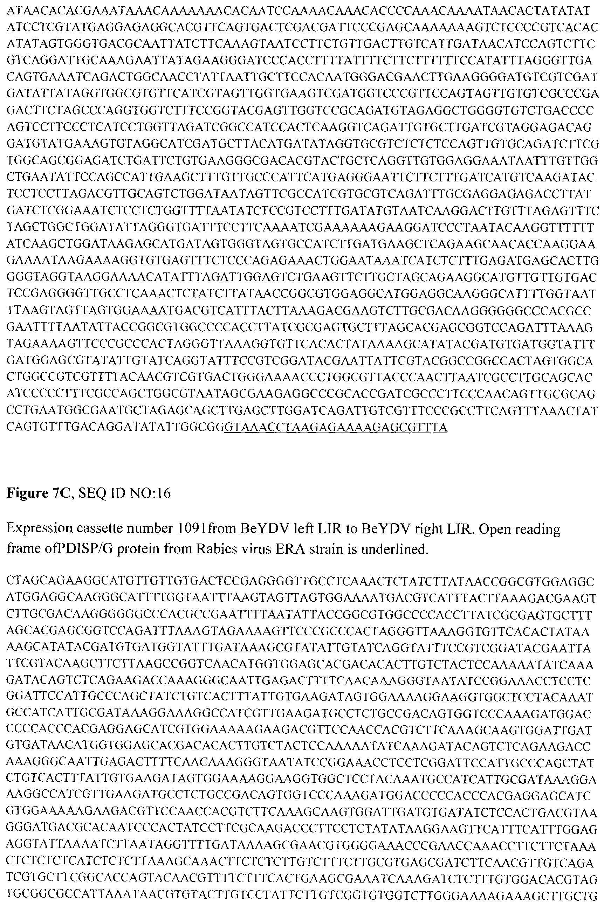

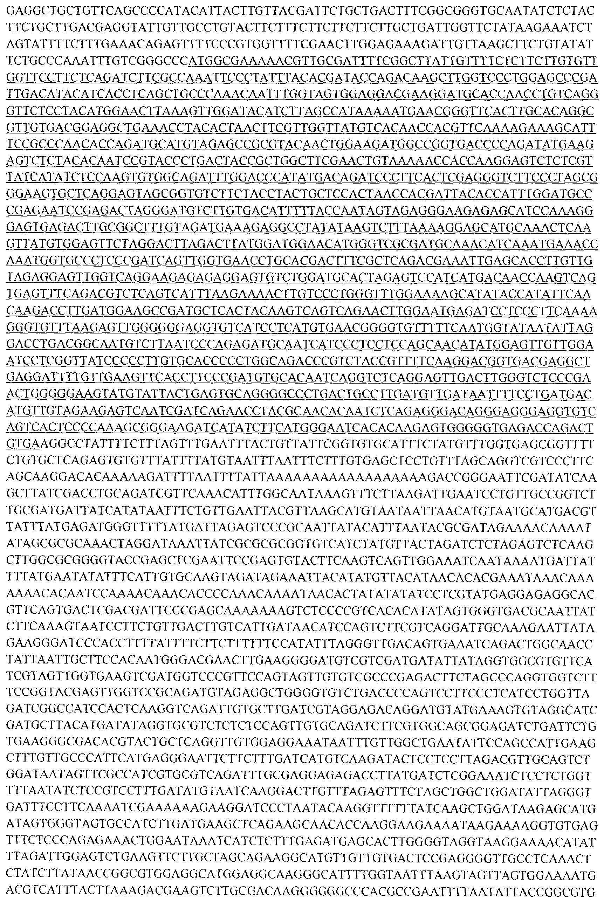

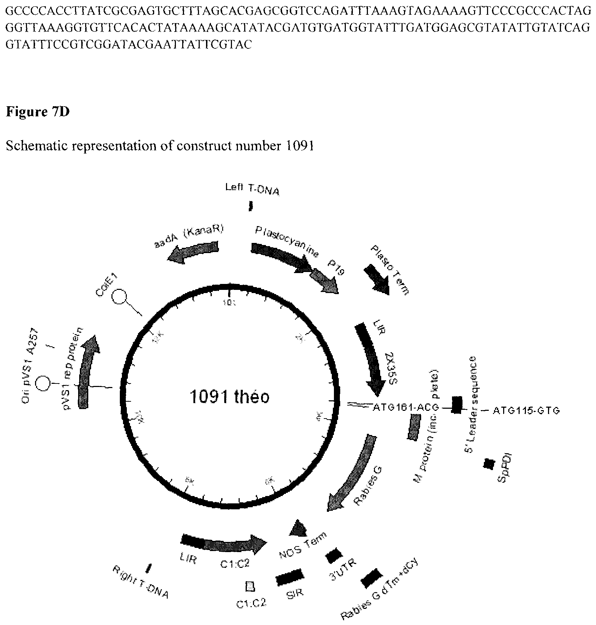

**Figure 7A** shows a schematic representation of construct number 1194. SacII and StuI restriction enzyme sites used for plasmid linearization are annotated on the representation. **Figure 7B** shows construct number 1194 from left to right t-DNA borders (underlined). 2X35S/CPMV-HT/PDISP/NOS into BeYDV+Replicase amplification system with Plastocyanin-P19-Plastocyanin expression cassette (suppressor of silencing) (SEQ ID NO: 15). **Figure 7C** shows expression cassette number 1091 from BeYDV left LIR to BeYDV right LIR. Open reading frame of PDISP/G protein from Rabies virus ERA strain is underlined. (SEQ ID NO:16). **Figure 7D** shows a schematic representation of construct number 1091.

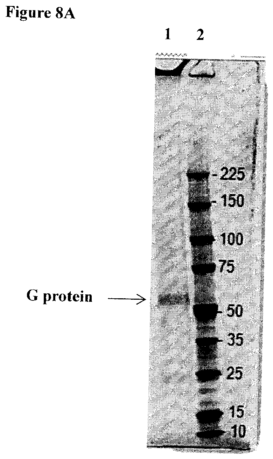

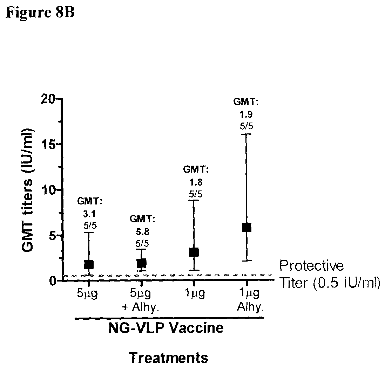

**Figure 8A** shows a coomassie stained SDS-PAGE (Precast 4-12%, from BioRad) showing G protein VLP preparation. 1) Rab-VLP preparation, 2) molecular weight marker. **Figure 8B** shows responses of neutralizing antibodies (IU/ml) in groups of mice (5 per group) immunized intramuscularly (i.m.) with three doses (D0, D7 and D28) of 0.1ml of the NG-VLP (Native G protein VLP) vaccine with or without adjuvant (Alhydrogel (Alhy)). Blood samples were taken on Day 44, 16 days after the 3rd doses. Method of testing used: RFFIT test (rapid fluorescent focus inhibition test): Geometric Mean Titers (GMT) was calculated, using the individual values obtained for each animal and positive responders are indicated. Bars represent GMT with 95% CI. Anova was performed between all treatment groups and no statistically significant differences were reported.

### DETAILED DESCRIPTION

The following description is of a preferred embodiment.

The present invention relates to virus-like particles (VLPs) comprising one or more native rabies virus structural protein, and methods of producing rabies VLPs in plants. The rabies VLPs comprise one or more native rabies virus structural protein, specifically one or more native rabies glycoprotein (G), and optionally one or more matrix protein. The VLP does not comprise virus proteins from a plant virus.

The present invention in part provides a method of producing a rabies virus like particle (VLP) in a plant. The method may comprise introducing a nucleic acid comprising a regulatory region active in the plant operatively linked to a nucleotide sequence encoding a native rabies virus glycoprotein (G) and one or more than one amplification element, into the plant, or portion of the plant. Followed by incubating the plant or portion of the plant under conditions that permit the expression of the nucleic acids, thereby producing the VLP.

The native rabies structural virus protein (also referred to as native rabies structural viral protein) may refer to all or a portion of a native rabies virus structural protein sequence isolated from rabies virus, present in any naturally occurring or variant rabies virus strain or isolate. Thus, the term native rabies virus structural protein and the like include naturally occurring native rabies virus structural protein sequence variants produced by mutation during the virus life-cycle or produced in response to selective pressure (e.g., drug therapy, expansion of host cell tropism or infectivity, etc.). As one of skill in the art appreciates, such native rabies virus structural protein sequences and variants thereof may be also produced using recombinant techniques. The native rabies structural virus protein does not include chimeric proteins wherein for example a transmembrane domain and/or a cytoplasmic tail have been replaced with a heterologous transmembrane domain and/or a cytoplasmic tail with respect to the native rabies structural virus protein.

A non-limiting example of a native rabies virus structural protein is rabies glycoprotein (G) protein, a fragment of G protein, a matrix (M) protein, a fragment of M protein, or a combination thereof. Non-limiting examples of G protein, or fragments of G protein that may be used according to the present invention include those G protein from rabies ERA strain. An example of a G protein, which is not to be considered limiting, is set forth in the amino acid sequence of SEQ ID NO: 14. Furthermore, the native rabies structural virus protein may comprise the sequence set forth in SEQ ID NO: 14, or a sequence having at least about 90-100% sequence similarity thereto, including any percent similarity within these ranges, such as 91, 92, 93, 94, 95, 96, 97, 98, 99% sequence similarity thereto.

Amino acid sequence similarity or identity may be computed by using the BLASTP and TBLASTN programs which employ the BLAST (basic local alignment search tool) 2.0 algorithm. Techniques for computing amino acid sequence similarity or identity are well known to those skilled in the art, and the use of the BLAST algorithm is described in ALTSCHUL et al. (1990, J Mol. Biol. 215: 403- 410) and ALTSCHUL et al. (1997, Nucleic Acids Res. 25: 3389-3402).

The native structural viral protein may exist as a monomer, a dimer, a trimer, or a combination thereof. A trimer is a macromolecular complex formed by three, usually non-covalently bound proteins. Without wishing to be bound by theory, the trimerization domain of a protein may be important for the formation such trimers. Therefore the viral protein or fragment thereof may comprise a trimerization domain.

By "matrix protein" (also referred to as viral core protein) it is meant a protein that that organizes and maintains virion structure. Viral matrix proteins usually interact directly with cellular membranes and can be involved in the budding process. Viral core proteins are proteins that make up part of the nucelocapsid and typically are directly associated with the viral nucleic acid. Examples of viral matrix or core protein are rabies M protein, influenza M1, RSV M and retrovirus gag proteins. Examples of Matrix proteins that may be used as described herein include, but are not limited to rabies M protein. Non-limiting example of sequences that may be used with the present invention include M protein from rabies virus ERA strain. An exemplary M protein consists of the amino acid sequence as shown in SEQ ID NO: 6. Further more, the native rabies structural virus protein may comprise the sequence set forth in SEQ ID NO: 6 or sequences having at least about 90-100% sequence similarity thereto, including any percent similarity within these ranges, such as 91, 92, 93, 94, 95, 96, 97, 98, 99% sequence similarity thereto.

Both vesicular stomatitis virus (a rhabdovirus like rabies) and Herpes Simplex virus (a herpes virus like varicella-zoster virus) bud in a VSP4-dependent manner (Taylor et al. J. Virol 81:13631-13639, 2007; Crump et al., J. Virol 81:7380-7387, 2007). Since VSP4 interacts with the late domain of the matrix protein, this suggests that the matrix protein is required for budding and, as a corollary, for VLP production. However, as described herein, native rabies VLPs may be produced within plants with or without co-expression of a matrix protein. Therefore, native rabies VLPs produced in plants from virus derived native structural proteins, in accordance with the present invention may or may not comprise viral matrix (or viral core) protein.

The present invention also provides for a method of producing rabies virus like protein VLPs in a plant, wherein a nucleic acid (a first nucleic acid) encoding a native rabies G protein is co-expressed with a second nucleic acid encoding a viral matrix protein, for example but not limited to rabies matrix protein. The nucleic acid, and second nucleic acid, may be introduced to the plant in the same step, or may be introduced to the plant sequentially.

As described in more detail below, VLPs may be produced in a plant by expressing a nucleic acid (a first nucleic acid) encoding one or more native rabies-virus G protein. A second nucleic acid encoding a matrix protein, for example but not limited to rabies matrix protein might be co-expressed in the plant. The nucleic acid and second nucleic acid may be introduced to the plant in the same step, or they may be introduced to the plant sequentially. The nucleic acid and second nucleic acid may be introduced in the plant in a transient manner, or in a stably manner.

Furthermore, a plant that expresses a first nucleic acid encoding one or more native rabies virus G protein, may be transformed with a matrix protein, for example but not limited to a rabies matrix
protein, (second nucleic acid) so that both the first and the second nucleic acids are co-expressed in the plant. Alternatively, a plant that expresses a matrix protein, for example but not limited to a rabies matrix protein, (second nucleic acid) may be transformed with a first nucleic acid encoding one or more native rabies virus structural protein, for example a rabies G protein, so that both the first and the second nucleic acids are co-expressed in the plant.

The present invention also provides a method of producing rabies virus VLPs in a plant that involves introducing one or more nucleic acid encoding one or more native rabies virus G protein operatively linked to a regulatory region active in the plant, and one or more than one amplification elements, into the plant or portion of the plant. The plant or portion of the plant is then incubated under conditions that permit the expression of the one or more nucleic acid, thereby producing the rabies virus VLPs.

The present invention further provides for a VLP comprising one or more native rabies virus G protein produced by the method as provided by the present invention.

The term "virus like particle" (VLP), or "virus-like particles" or "VLPs" refers to structures that self-assemble and comprise one or more structural proteins such as for example native rabies virus structural protein for example but not limited to rabies G protein. VLPs are generally morphologically and antigenically similar to virions produced in an infection, but lack genetic information sufficient to replicate
and thus are non-infectious. VLPs may be produced in suitable host cells including plant host cells. Following extraction from the host cell and upon isolation and further purification under suitable conditions, VLPs may be purified as intact structures.

The size (i.e. the diameter) of the above-defined VLPs, maybe measures for example by dynamic light scattering (DLS) or electron microscope (EM) techniques, is usually between 40 to 300 nm, or any size therebetween. In some embodiments, the size of the intact VLP structure may range from about 40 nm to about 300 nm, or any size therebetween, such as 50 nm, 60 nm, 70 nm, 80 nm, 90 nm, 100 nm, 110 nm, 120 nm, 130 nm, 140 nm, 150 nm, 160 nm, 170 nm, 180 nm, 190 nm, 200 nm, 210 nm, 220 nm, 230 nm, 240 nm, 250 nm, 260 nm, 270 nm or 280 nm, or any size therebetween. In an embodiment the size of the intact VLP structure may range from about 170 nm to about 200 nm or any size therebetween, such as 175 nm, 180 nm,185 nm, 190 nm, 195 nm or any size therebetween.

There is also provided a nucleic acid comprising a nucleotide sequence encoding one or more native rabies virus structural protein operatively linked to a regulatory region active in a plant. Furthermore one or more native rabies virus structural protein may be operatively linked to one or more than one amplification elements. The one or more native rabies virus structural protein may be for example one or more rabies G protein one or more M protein, or both.

A nucleic acid sequence referred to in the present invention, may be "substantially homologous" or "substantially similar" to a sequence, or a compliment of the sequence if the nucleic acid sequence hybridise to one or more than one nucleotide sequence or a compliment of the nucleic acid sequence as defined herein under stringent hybridisation conditions. Sequences are "substantially homologous" "substantially similar" when at least about 70%, or between 70 to 100%, or any amount therebetween, for example 70, 72, 74, 76, 78, 80, 82, 84, 86, 88, 90, 92, 94, 96, 98, 100%, or any amount therebetween, of the nucleotides match over a defined length of the nucleotide sequence providing that such homologous sequences exhibit one or more than one of the properties of the sequence, or the encoded product as described herein. Correct folding of the protein may be important for stability of the protein, formation of multimers, formation of VLPs and function. Folding of a protein may be influenced by one or more factors, including, but not limited to, the sequence of the protein, the relative abundance of the protein, the degree of intracellular crowding, the availability of cofactors that may bind or be transiently associated with the folded, partially folded or unfolded protein.

Such a sequence similarity may be determined using a nucleotide sequence comparison program, such as that provided within DNASIS (using, for example but not limited to, the following parameters: GAP penalty 5, #of top diagonals 5, fixed GAP penalty 10, k-tuple 2, floating gap 10, and window size 5). However, other methods of alignment of sequences for comparison are well-known in the art for example the algorithms of Smith & Waterman (1981, Adv. Appl. Math. 2:482), Needleman & Wunsch (J. Mol. Biol. 48:443, 1970), Pearson & Lipman (1988, Proc. Nat'l. Acad. Sci. USA 85:2444), and by computerized implementations of these algorithms (GAP, BESTFIT, FASTA, and BLAST, available through the NIH.), or by manual alignment and visual inspection (see, e.g., Current Protocols in Molecular Biology, Ausubel et al., eds. 1995 supplement), or using Southern or Northern hybridization under stringent conditions (see Maniatis et al., in Molecular Cloning (A Laboratory Manual), Cold Spring Harbor Laboratory, 1982). Preferably, sequences that are substantially homologous exhibit at least about 80% and most preferably at least about 90% sequence similarity over a defined length of the molecule.

An example of one such stringent hybridization conditions may be overnight (from about 16-20 hours) hybridization in 4 X SSC at 65°C, followed by washing in 0.1 X SSC at 65°C for an hour, or 2 washes in 0.1 X SSC at 65°C each for 20 or 30 minutes. Alternatively an exemplary stringent hybridization condition could be overnight (16-20 hours) in 50% formamide, 4 X SSC at 42°C, followed by washing in 0.1 X SSC at 65°C for an hour, or 2 washes in 0.1 X SSC at 65°C each for 20 or 30 minutes, or overnight (16-20 hours), or hybridization in Church aqueous phosphate buffer (7% SDS; 0.5M NaPO₄ buffer pH 7.2; 10 mM EDTA) at 65°C, with 2 washes either at 50°C in 0.1 X SSC, 0.1% SDS for 20 or 30 minutes each, or 2 washes at 65°C in 2 X SSC, 0.1% SDS for 20 or 30 minutes each for unique sequence regions.

A nucleic acid encoding a native rabies structural polypeptide or a native rabies virus structural protein may be described as a "rabies nucleic acid", a "rabies nucleotide sequence", a "native rabies nucleic acid", or a "native rabies nucleotide sequence". For example, which is not to be considered limiting, a virus-like particle comprising one or more native rabies virus structural protein or native rabies virus structural polypeptide, may be described as a "rabies VLP" or "native rabies VLP".

The native rabies virus structural protein or polypeptide may include a signal peptide that is the same as, or heterologous with, the remainder of the polypeptide or protein. The term "signal peptide" is well known in the art and refers generally to a short (about 5-30 amino acids) sequence of amino acids, found generally at the N-terminus of a polypeptide that may direct translocation of the newly-translated polypeptide to a particular organelle, or aid in positioning of specific domains of the polypeptide chain relative to others. As a non-limiting example, the signal peptide may target the translocation of the protein into the endoplasmic reticulum and/or aid in positioning of the N-terminus proximal domain relative to a membrane-anchor domain of the nascent polypeptide to aid in cleavage and folding of the mature protein, for example which is not to be considered limiting, a native rabies virus structural protein.

A signal peptide (SP) may be native to the protein or virus protein, or a signal peptide may be heterologous with respect to the primary sequence of the protein or virus protein being expressed. For example the native signal peptide of native rabies G protein or native rabies M protein may be used to express the native rabies virus structural protein in a plant system.

A signal peptide may also be non-native, for example, from a protein, viral protein or native structural protein of a virus other than virus protein, or from a plant, animal or bacterial polypeptide. A non limiting example of a signal peptide that may be used is that of alfalfa protein disulfide isomerase (PDI SP; nucleotides 32-103 of Accession No. Z11499).

The present invention therefore provides for a native rabies virus structural protein comprising a native, or a non-native signal peptide, and nucleic acids encoding such rabies virus structural proteins.

### Amplification Element

The native rabies virus structural protein or polypeptide may be expressed in an expression system comprising a viral based, DNA or RNA, expression system, for example but not limited to, a comovirus-based expression cassette and geminivirus-based amplification element.

The expression system as described herein may comprise an expression cassette based on a bipartite virus, or a virus with a bipartite genome. For example, the bipartite viruses may be of the Comoviridae family. Genera of the Comoviridae family include Comovirus, Nepovirus, Fabavirus, Cheravirus and Sadwavirus. Comoviruses include Cowpea mosaic virus (CPMV), Cowpea severe mosaic virus (CPSMV), Squash mosaic virus (SqMV), Red clover mottle virus (RCMV), Bean pod mottle virus (BPMV), Turnip ringspot virus (TuRSV), Broad bean true mosaic virus (BBtMV), Broad bean stain virus (BBSV), Radish mosaic virus (RaMV). Examples of comoviruse RNA-2 sequences comprising enhancer elements that may be useful for various aspects of the invention include, but are not limited to: CPMV RNA-2 (GenBank Accession No. NC_003550), RCMV RNA-2 (GenBank Accession No. NC_003738), BPMV RNA-2 (GenBank Accession No. NC_003495), CPSMV RNA-2 (GenBank Accession No.NC_003544), SqMV RNA-2 (GenBank Accession No.NC_003800), TuRSV RNA-2 (GenBank Accession No. NC_013219.1). BBtMV RNA-2 (GenBank Accession No. GU810904), BBSV RNA2 (GenBank Accession No. FJ028650), RaMV (GenBank Accession No. NC_003800).

Segments of the bipartite comoviral RNA genome are referred to as RNA-1 and RNA-2. RNA-1 encodes the proteins involved in replication while RNA-2 encodes the proteins necessary for cell-to-cell movement and the two capsid proteins. Any suitable comovirus-based cassette may be used including CPMV, CPSMV, SqMV, RCMV, or BPMV, for example, the expression cassette may be based on CPMV.

"Expression cassette" refers to a nucleotide sequence comprising a nucleic acid of interest under the control of, and operably (or operatively) linked to, an appropriate promoter or other regulatory elements for transcription of the nucleic acid of interest in a host cell.

The expression systems comprise amplification elements from a geminivirus for example, an amplification element from the bean yellow dwarf virus (BeYDV). BeYDV belongs to the Mastreviruses genus adapted to dicotyledonous plants. BeYDV is monopartite having a single-strand circular DNA genome and can replicate to very high copy numbers by a rolling circle mechanism. BeYDV-derived DNA replicon vector systems have been used for rapid high-yield protein production in plants.

As used herein, the phrase "amplification elements" refers to a nucleic acid segment comprising at least a portion of one ore more long intergenic regions or long intergenic repeat (LIR) of a geminivirus genome. As used herein, "long intergenic region" or "long intergenic repeat" refers to a region of a long intergenic region that contains a rep binding site capable of mediating excision and replication by a geminivirus Rep protein. In some aspects, the nucleic acid segment comprising one or more LIRs, may further comprises a short intergenic region or small intergenic region (SIR) of a geminivirus genome. As used herein, "short intergenic region" or "small intergenic region" refers to the complementary strand (the short IR (SIR) of a Mastreviruses). Any suitable geminivirus-derived amplification element may be used herein. See, for example, WO2000/20557; WO2010/025285; Zhang X. et al. (2005, Biotechnology and Bioengineering, Vol. 93, 271-279), Huang Z. et al. (2009, Biotechnology and Bioengineering, Vol. 103, 706-714), Huang Z. et al.(2009, Biotechnology and Bioengineering, Vol. 106, 9-17); which are herein incorporated by reference). If more than one LIR is used in the construct, for example two LIRs, then the promoter, CMPV-HT regions and the nucleic acid sequence of interest and the terminator are bracketed by each of the two LIRs. Furthermore, the amplification element might for example originate from the sequence as disclosed in Halley-Stott et al. (2007) Archives of Virology 152: 1237-1240, deposited under Gen Bank accession number DQ458791. The nucleic acid segment comprising LIRs are joined nucleotides 2401 to 2566 and 1 to 128. The nucleic acid segment comprising SIRs are nucleotides 1154 to 1212.

As described herein, co-delivery of bean yellow dwarf virus (BeYDV)-derived vector and a Rep/RepA-supplying vector, by agroinfiltration of *Nicotiana benthamiana* leaves results in efficient replicon amplification and robust protein production.

A comovirus-based expression cassette and a geminivirus-derived amplification element may be comprised on separate vectors, or the component parts may be included in one vector. If two vectors are used, the first and second vectors may be introduced into a plant cell simultaneously, or separately.

A viral replicase may also be included in the expression system as described herein to increase expression of the nucleic acid of interest. An non-limiting example of a replicase is a BeYDV replicase (pREP110) encoding BeYDV Rep and RepA (C2/C1; Huang et al., 2009, Biotechnol. Bioeng. 103, 706-714). Another non-limiting example of a replicase is disclosed in Halley-Stott et al. (2007) Archives of Virology 152: 1237-1240, deposited under Gen Bank accession number DQ458791. The nucleic acid segment comprising C1:C2 gene are nucleotides 1310 to 2400.

By "co-expressed" it is meant that two or more than two nucleotide sequences are expressed at about the same time within the plant, and within the same tissue of the plant. However, the nucleotide sequences need not be expressed at exactly the same time. Rather, the two or more nucleotide sequences are expressed in a manner such that the encoded products have a chance to interact. The two or more than two nucleotide sequences can be co-expressed using a transient expression system, where the two or more sequences are introduced within the plant at about the same time under conditions that both sequences are expressed. Alternatively, a platform plant comprising one of the nucleotide sequences may be transformed in a stable manner, with an additional sequence encoding the protein of interest for example the native rabies virus structural protein, introduced into the platform plant in a transient manner.

### Chaperon

Correct folding of the expressed native rabies virus structural protein may be important for stability of the protein, formation of multimers, formation of VLPs, function of the native rabies virus structural protein and recognition of the native rabies virus structural protein by an antibody, among other characteristics. Folding and accumulation of a protein may be influenced by one or more factors, including, but not limited to, the sequence of the protein, the relative abundance of the protein, the degree of intracellular crowding, the pH in a cell compartment, the availability of cofactors that may bind or be transiently associated with the folded, partially folded or unfolded protein, the presence of one or more chaperone proteins, or the like.

Heat shock proteins (Hsp) or stress proteins are examples of chaperone proteins, which may participate in various cellular processes including protein synthesis, intracellular trafficking, prevention of misfolding, prevention of protein aggregation, assembly and disassembly of protein complexes, protein folding, and protein disaggregation. Examples of such chaperone proteins include, but are not limited to, Hsp60, Hsp65, Hsp 70, Hsp90, Hsp100, Hsp20-30, Hsp10, Hsp100-200, Hsp100, Hsp90, Lon, TF55, FKBPs, cyclophilins, ClpP, GrpE, ubiquitin, calnexin, and protein disulfide isomerases (see, for example, Macario, A.J.L., Cold Spring Harbor Laboratory Res. 25:59-70. 1995; Parsell, D.A. & Lindquist, S. Ann. Rev. Genet. 27:437-496 (1993); U.S. Patent No. 5,232,833). As described herein, chaperone proteins, for example but not limited to Hsp40 and Hsp70 may be used to ensure folding of a rabies virus protein.

Examples of Hsp70 include Hsp72 and Hsc73 from mammalian cells, DnaK from bacteria, particularly mycobacteria such as *Mycobacterium* leprae, *Mycobacterium tuberculosis,* and *Mycobacterium bovis* (such as Bacille-Calmette Guerin: referred to herein as Hsp71). DnaK from *Escherichia coli,* yeast and other prokaryotes, and BiP and Grp78 from eukaryotes, such as *A. thaliana* (Lin et al. 2001 (Cell Stress and Chaperones 6:201-208). A particular example of an Hsp70 is *A. thaliana* Hsp70 (encoded by Genbank ref: AY120747.1). Hsp70 is capable of specifically binding ATP as well as unfolded polypeptides and peptides, thereby
participating in protein folding and unfolding as well as in the assembly and disassembly of protein complexes.

Examples of Hsp40 include DnaJ from prokaryotes such as *E. coli* and mycobacteria and HSJ1, HDJ1 and Hsp40 from eukaryotes, such as alfalfa (Frugis et al., 1999. Plant Molecular Biology 40:397-408). A particular example of an Hsp40 is *M*. *sativa* MsJ1 (Genbank ref: AJ000995.1). Hsp40 plays a role as a molecular chaperone in protein folding, thermotolerance and DNA replication, among other cellular activities.

Among Hsps, Hsp70 and its co-chaperone, Hsp40, are involved in the stabilization of translating and newly synthesized polypeptides before the synthesis is complete. Without wishing to be bound by theory, Hsp40 binds to the hydrophobic patches of unfolded (nascent or newly transferred) polypeptides, thus facilitating the interaction of Hsp70-ATP complex with the polypeptide. ATP hydrolysis leads to the formation of a stable complex between the polypeptide, Hsp70 and ADP, and release of Hsp40. The association of Hsp70-ADP complex with the hydrophobic patches of the polypeptide prevents their interaction with other hydrophobic patches, preventing the incorrect folding and the formation of aggregates with other proteins (reviewed in Hartl, FU. 1996. Nature 381:571-579).

Native chaperone proteins may be able to facilitate correct folding of low levels of recombinant protein, but as the expression levels increase, the abundance of native chaperones may become a limiting factor. High levels of expression of native rabies virus structural protein in the agroinfiltrated leaves may lead to the accumulation of native rabies virus structural protein in the cytosol, and co-expression of one or more than one chaperone proteins such as Hsp70, Hsp40 or both Hsp70 and Hsp40 may reduce the level of misfolded or aggregated proteins, and increase the number of proteins exhibiting tertiary and quaternary structural characteristics that allow for formation of virus-like particles.

Therefore, the present invention also provides for a method of producing rabies VLPs in a plant, wherein a first nucleic acid encoding a native rabies virus glycoprotein (G)_is co-expressed with a second nucleic acid encoding a chaperone. The first and
second nucleic acids may be introduced to the plant in the same step, or may be introduced to the plant sequentially.

### Regulatory Element

The use of the terms "regulatory region", "regulatory element" or "promoter" in the present application is meant to reflect a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" may includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, may also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.

In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element comprises a basal promoter element, responsible for the initiation of transcription, as well as other regulatory elements (as listed above) that modify gene expression.

There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et aI., 1994, Plant Cell 14: 125-130). An example of a leaf-specific promoter includes the plastocyanin promoter (see US 7,125,978).

An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, LR.P., 1998, Trends Plant Sci. 3, 352-358). Examples, of potential inducible promoters include, but not limited to, tetracycline-inducible promoter (Gatz, C.,1997, Ann. Rev. Plant Physiol. Plant Mol. Biol. 48,89-108) steroid inducible promoter (Aoyama. T. and Chua, N.H.,1997, Plant 1. 2, 397-404; and ethanol-inducible promoter (Salter, M.G., et aI, 1998, Plant 10urnal 16, 127-132; Caddick, M.X., et al,1998, Nature Biotech. 16, 177-180, cytokinin inducible IB6 and CKI 1 genes (Brandstatter, I. and K.ieber, 1.1.,1998, Plant Cell 10, 1009-1019; Kakimoto, T., 1996, Science 274,982-985; and the auxin inducible element, DR5 (Ulmasov, T., et aI., 1997, Plant Cell 9, 1963-1971).

A constitutive regulatory region directs the expression of a gene throughout the various parts of a plant and continuously throughout plant development. Examples of known constitutive regulatory elements include promoters associated with the CaMV 35S transcript (Odell et aI., 1985, Nature, 313: 810-812), the rice actin 1 (Zhang et aI, 1991, Plant Cell, 3: 1155-1165), actin 2 (An et al., 1996, Plant J., 10: 107-121), or tms 2 (U.S. 5,428,147, and triosephosphate isomerase 1 (Xu et. aI., 1994, Plant Physiol. 106: 459-467) genes, the maize ubiquitin 1 gene (Cornejo et ai, 1993, Plant Mol. BioI. 29: 637-646), the *Arabidopsis* ubiquitin 1 and 6 genes (Holtorf et aI, 1995, Plant Mol. BioI. 29: 637-646), and the tobacco translational initiation factor 4A gene (Mandel et aI, 1995, Plant Mol. BioI. 29: 995-1004).

The term "constitutive" as used herein does not necessarily indicate that a gene under control of the constitutive regulatory region is expressed at the same level in all cell types, but that the gene is expressed in a wide range of cell types even though variation in abundance is often observed. Constitutive regulatory elements may be coupled with other sequences to further enhance the transcription and/or translation of the nucleotide sequence to which they are operatively linked. For example, the CPMV-HT system is derived from the untranslated regions of the Cowpea mosaic virus (CPMV) and demonstrates enhanced translation of the associated coding sequence. By "native" it is meant that the nucleic acid or amino acid sequence is naturally occurring, or "wild type". By "operatively linked" it is meant that the particular sequences, for example a regulatory element and a coding region of interest, interact either directly or indirectly to carry out an intended function, such as mediation or modulation of gene expression. The interaction of operatively linked sequences may, for example, be mediated by proteins that interact with the operatively linked sequences.

The invention provides VLPs that obtain a lipid envelope from the plasma membrane of the cell in which the VLPs are expressed. For example, if the one or more native rabies virus structural protein is expressed in a plant-based system, the resulting VLP may obtain a lipid envelope from the plasma membrane of the plant cell.

Generally, the term "lipid" refers to a fat-soluble (lipophilic), naturally-occurring molecule. A VLP produced in a plant according to the invention may be complexed with plant-derived lipids. The plant-derived lipids may be in the form of a lipid bilayer, and may further comprise an envelope surrounding the VLP. The plant-derived lipids may comprise lipid components of the plasma membrane of the plant where the VLP is produced, including phospholipids, tri-, di- and monoglycerides, as well as fat-soluble sterol or metabolites comprising sterols. Examples include phosphatidylcholine (PC), phosphatidylethanolamine (PE), phosphatidylinositol, phosphatidylserine, glycosphingolipids, phytosterols or a combination thereof. A plant-derived lipid may alternately be referred to as a 'plant lipid'. Examples of phytosterols include campesterol, stigmasterol, ergosterol, brassicasterol, delta-7-stigmasterol, delta-7-avenasterol, daunosterol, sitosterol, 24-methylcholesterol, cholesterol or beta-sitosterol - see, for example, Mongrand et ai., 2004. As one of skill in the art would understand, the lipid composition of the plasma membrane of a cell may vary with the culture or growth conditions of the cell or organism, or species, from which the cell is obtained. Generally, beta-sitosterol is the most abundant phytosterol.

Cell membranes generally comprise lipid bilayers, as well as proteins for various functions. Localized concentrations of particular lipids may be found in the lipid bilayer, referred to as 'lipid rafts'. These lipid raft microdomains may be enriched in sphingolipids and sterols. Without wishing to be bound by theory, lipid rafts may have significant roles in endo and exocytosis, entry or egress of viruses or other infectious agents, inter-cell signal transduction, interaction with other structural components of the cell or organism, such as intracellular and extracellular matrices.

The VLP produced within a plant may induce a native rabies virus structural protein comprising plant-specific N-glycans. Therefore, this invention also provides for a VLP comprising native rabies virus structural protein having plant specific N-glycans.

Furthermore, modification of N-glycan in plants is known (see for example U.S. 60/944,344; which is incorporated herein by reference) and native rabies virus structural proteins having modified N-glycans may be produced. Native rabies virus structural proteins comprising a modified glycosylation pattern, for example with reduced fucosylated, xylosylated, or both, fucosylated and xylosylated, N-glycans may be obtained, or native rabies virus structural proteins having a modified glycosylation pattern may be obtained, wherein the protein lacks fucosylation, xylosylation, or both, and comprises increased galatosylation. Furthermore, modulation of post-translational modifications, for example, the addition of terminal galactose may result in a reduction of fucosylation and xylosylation of the expressed native rabies virus structural proteins when compared to a wild-type plant expressing native rabies virus structural proteins.

For example, which is not to be considered limiting, the synthesis of native rabies virus structural proteins having a modified glycosylation pattern may be achieved by co-expressing the native rabies virus structural protein along with a nucleotide sequence encoding beta-1.4 galactosyltransferase (GalT), for example, but not limited to mammalian GalT, or human GalT however GalT from another sources may also be used. The catalytic domain of GalT may also be fused to a CTS domain (i.e. the cytoplasmic tail, transmembrane domain, stem region) of N-acetylglucosaminyl transferase (GNT1), to produce a GNT1-GalT hybrid enzyme, and the hybrid enzyme may be co-expressed with native rabies virus structural protein. The native rabies virus structural protein may also be co-expressed along with a nucleotide sequence encoding N-acetylglucosaminyltrasnferase III (GnT-III), for example but not limited to mammalian GnT-III or human GnT-III, GnT-III from other sources may also be used. Additionally, a GNT1-GnT-III hybrid enzyme, comprising the CTS of GNT1 fused to GnT-III may also be used.

Therefore the present invention also provides VLP's comprising native rabies virus structural protein having modified N-glycans.

Without wishing to be bound by theory, the presence of plant N-glycans on native rabies virus structural protein may stimulate the immune response by promoting the binding of native rabies virus structural protein by antigen presenting cells. Stimulation of the immune response using plant N glycan has been proposed by Saint-Jore-Dupas et al. (2007). Furthermore, the conformation of the VLP may be advantageous for the presentation of the antigen, and enhance the adjuvant effect of VLP when complexed with a plant derived lipid layer.

The occurrence of VLPs may be detected using any suitable method for example, sucrose gradients, or size exclusion chromatography. VLPs may be assessed for structure and size by, for example electron microscopy, or by size exclusion chromatography.

For size exclusion chromatography, total soluble proteins may be extracted from plant tissue by homogenizing (Polytron) sample of frozen-crushed plant material in extraction buffer, and insoluble material removed by centrifugation. Precipitation with ice cold acetone or PEG may also be of benefit. The soluble protein is quantified, and the extract passed through a Sephacryl™ column, for example a Sephacryl™ S500 column. Blue Dextran 2000 may be used as a calibration standard. Following chromatography, fractions may be further analyzed by immunoblot to determine the protein complement of the fraction.

The separated fraction may be for example a supernatant (if centrifuged, sedimented, or precipitated), or a filtrate (if filtered), and is enriched for proteins, or suprastructure proteins, such as for example rosette-like structures or higher-order, higher molecular weight, particles such as VLPs. The separated fraction may be further processed to isolate, purify, concentrate or a combination thereof, the proteins, or suprastructure proteins, by, for example, additional centrifugation steps, precipitation, chromatographic steps (e.g. size exclusion, ion exchange, affinity chromatography), tangential flow filtration, or a combination thereof. The presence of purified proteins, or suprastructure proteins, may be confirmed by, for example, native or SDS-PAGE, Western analysis using an appropriate detection antibody, capillary electrophoresis, electron microscopy, or any other method as would be evident to one of skill in the art.

Figures 3A and 3B, show an example of an elution profile of a size exclusion chromatography analysis of a plant extract comprising VLPs. In this case, VLPs comprising native rabies virus structural proteins elute in fractions 8 to approx. 11, rosettes and high molecular weight structures elute from about fractions 12 to about 14, and lower molecular weight, or soluble form of the native rabies virus structural proteins elute in fractions from about 15 to about 17.

The VLPs may be purified or extracted using any suitable method for example chemical or biochemical extraction. VLPs are relatively sensitive to desiccation, heat, pH, surfactants and detergents. Therefore it may be useful to use methods that maximize yields, minimize contamination of the VLP fraction with cellular proteins, maintain the integrity of the proteins, or VLPs, and, where required, the associated lipid envelope or membrane, methods of loosening the cell wall to release the proteins, or VLP. For example, methods that produce protoplast and/or spheroplasts may be used (see for example WO 2011/035422) to obtain VLPs as described herein. Minimizing or eliminating the use of detergence or surfactants such for example SDS or Triton X-100 may be beneficial for improving the yield of VLP extraction. VLPs may be then assessed for structure and size by, for example, electron microscopy, or by size exclusion chromatography as mentioned above.

The one or more than one or more genetic constructs of the present invention may be expressed in any suitable plant host that is transformed by the nucleotide sequence, or constructs, or vectors of the present invention. Examples of suitable hosts include, but are not limited to, agricultural crops including alfalfa, canola, *Brassica* spp., maize, *Nicotiana* spp., alfalfa, potato, ginseng, pea, oat, rice, soybean, wheat, barley, sunflower, cotton and the like.

The one or more genetic constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. Non-limiting examples of suitable 3' regions are the 3' transcribed nontranslated regions containing a polyadenylation signal of *Agrobacterium* tumor inducing (Ti) plasmid genes, such as the nopaline synthase (NOS) gene, plant genes such as the soybean storage protein genes, the small subunit of the ribulose-I, 5-bisphosphate carboxylase gene (ssRUBISCO; US 4,962,028; the promoter used in regulating plastocyanin expression, described in US 7,125,978.

One or more of the genetic constructs of the present invention may also include further enhancers, either translation or transcription enhancers, as may be required. Enhancers may be located 5' or 3' to the sequence being transcribed. Enhancer regions are well known to persons skilled in the art, and may include an ATG initiation codon, adjacent sequences or the like. The initiation codon, if present, may be in phase with the reading frame ("in frame") of the coding sequence to provide for correct translation of the transcribed sequence.

The constructs of the present invention can be introduced into plant cells using Ti plasmids, Ri plasmids, plant virus vectors, direct DNA transformation, micro-injection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff (J Virol Meth, 105:343-348, 2002,), U.S. Pat. Nos. 4,945,050; 5,036,006; and 5,100,792, U.S. patent application Ser. Nos. 08/438,666, filed May 10, 1995, and 07/951,715, filed Sep. 25, 1992.

As described below, transient expression methods may be used to express the constructs of the present invention (see Liu and Lomonossoff, 2002, Journal of Virological Methods, 105:343-348). Alternatively, a vacuum-based transient expression method, as described by Kapila et al., 1997, may be used. These methods may include, for example, but are not limited to, a method of Agro-inoculation or Agro-infiltration, syringe infiltration, however, other transient methods may also be used as noted above. With Agro-inoculation, Agro-infiltration, or syringe infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacteria* infect and transfer t-DNA copies into the cells. The t-DNA is episomally transcribed and the mRNA translated, leading to the production of the protein of interest in infected cells, however, the passage of t-DNA inside the nucleus is transient.

To aid in identification of transformed plant cells, the constructs of this invention may be further manipulated to include plant selectable markers. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

Also provided are transgenic plants, plant cells or seeds containing the gene construct of the present invention. Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue cultures.

The present disclosure includes nucleotide sequences:

**Table 1. List of Sequence Identification numbers.**

| **SEQ ID NO**: | **Description** | **Table/Figure** |
|---|---|---|
| 1 | IF-RabM-S3.c | Figure 4A |
| 2 | IF-RabM-S1-4.r | Figure 4B |
| 3 | Synthesized M protein coding sequence (corresponding to nt2496-3104 from Genbank accession number FJ913470) | Figure 4C |
| 4 | Construct number 1191 from left to right t-DNA borders (underlined).2X35S/CPMV-HT/NOS with Plastocyanin-P19-Plastocyanin expression cassette (suppressor of silencing) | Figure 4E |
| 5 | Expression cassette number 1066 from 2X35S promoter to NOS terminator.Open reading frame of M protein from Rabies virus ERA strainis underlined. | Figure 4F |
| 6 | Amino acid sequence of M protein from Rabies virus ERA strain | Figure 4G |
| 7 | Construct number 1193 from left to right t-DNA borders (underlined). 2X35S/CPMV-HT/NOS into BeYDV+Replicase amplification system with Plastocyanin-TBSV P19-Plastocyanin expression cassette (suppressor of silencing) | Figure 5B |
| 8 | Expression cassette number 1086 from BeYDV left LIR to BeYDV right LIR. Open reading frame ofPDISP/G protein from Rabies virus ERA strain is underlined. | Figure 5C |
| 9 | IF-RabG-S2+4.c | Figure 6A |
| 10 | IF-RabG-S1-4.r | Figure 6B |
| 11 | Synthesized Rabies G protein coding sequence (corresponding to nt 3317-4891 from Genbank accession number EF206707) | Figure 6C |
| 12 | Construct number 1192 from left to right t-DNA borders (underlined).2X35S/CPMV-HT/PDISP/NOS with Plastocyanin-P19-Plastocyanin expression cassette (suppressor of silencing) | Figure 6E |
| 13 | Expression cassette number 1071 from 2X35S promoter to NOS terminator. Open reading frame ofPDISP/Gprotein from Rabies virus ERA strain is underlined. | Figure 6F |
| 14 | Amino acid sequence of PDISP-G protein from Rabies virus ERA strain | Figure 6G |
| 15 | Construct number 1194 from left to right t-DNA borders (underlined).2X35S/CPMV-HT/PDISP/NOS into BeYDV+Replicase amplification system with Plastocyanin-P19-Plastocyanin expression cassette (suppressor of silencing) | Figure 7B |
| 16 | Expression cassette number 1091 from BeYDV left LIR to BeYDV right LIR. Open reading frame ofPDISP/G protein from Rabies virus ERA strain is underlined. | Figure 7C |

The present invention will be further illustrated in the following examples.

### Examples

### Constructs

**Table 2. Constructs comprising sequences encoding native rabies virus structural protein**

| Expression system | Amplification system | Signal peptide | Structural Protein | Construct number |
|---|---|---|---|---|
| CPMV HT | - | - | Rabies M | **1066** |
| CPMV HT | BeYDV+rep | - | Rabies M | **1086** |
| CPMV HT | - | Sp PDI | Rabies G | **1071** |
| CPMV HT | BeYDV+rep | Sp PDI | Rabies G | **1091** |

Construct number 1091 encodes native rabies G protein and comprises an amplification element, which is in accordance with the nucleic acid of the claimed invention. Construct number 1066 encodes a rabies matrix protein (M) and construct number 1086 comprises an amplification element and encodes a rabies matrix protein (M). Construct numbers 1086 and 1066 might be used in conjunction with construct 1091 in accordance with an embodiment of the claimed invention. Construct number 1071 is relevant to the understanding of the invention.

### Example 1: Assembly of expression cassettes with rabies protein

### A-2X35S/CMPV-HT/Rabies M/NOS (Construct number 1066)

A sequence encoding M protein from Rabies virus ERA strain was cloned into 2X35S/CPMV-HT/NOS expression system in a plasmid containing Plasto_pro/P19/Plasto_terexpression cassette using the following PCR-based method. A fragment containing the complete M protein coding sequence was amplified using primers IF-RabM-S3.c (Figure 4A, SEQ ID NO:1) and IF-RabM-S1-4.r (Figure 4B, SEQ ID NO: 2) using synthesized M gene (corresponding to nt 2496-3104 from Genbank accession number FJ913470) (Figure 4C, SEQ ID NO: 3) as template. The PCR product was cloned in 2X35S/CPMV-HT/NOS expression system using In-Fusion cloning system (Clontech,Mountain View, CA). Construct 1191(Figure 4D) was digested with SacII and StuI restriction enzyme and the linearized plasmid was used for the In-Fusion assembly reaction. Construct number 1191 is an acceptor plasmid intended for "In Fusion" cloning of genes of interest in a CPMV-HT-based expression cassette. It also incorporates a gene construct for the co-expression of the TBSV P19 suppressor of silencing under the alfalfa Plastocyanin gene promoter and terminator. The backbone of construct number 1191 is a pCAMBIA binary plasmid and the sequence from left to right t-DNA borders is presented in Figure 4E (SEQ ID NO:4). The resulting construct was given number 1066 (Figure 4F, SEQ ID NO: 5). The amino acid sequence of M protein from Rabies virus ERA strain is presented in Figure 4G (SEQ ID NO: 6). A representation of plasmid 1066 is presented in Figure 4H.

### B-2X35S/CMPV-HT/Rabies M/NOS intoBeYDV+Replicase amplification system (Construct number 1086)

A sequence encoding M protein from Rabies virus ERA strain was cloned into 2X35S/CPMV-HT/NOS expression system comprising the BeYDV+replicase amplification system in a plasmid containing Plasto_pro/P19/Plasto_ter expression cassette using the following PCR-based method. A fragment containing the complete M protein coding sequence was amplified using primers IF-RabM-S3.c (Figure 4A, SEQ ID NO: 1) and IF-RabM-S1-4.r (Figure 4B, SEQ ID NO: 2) using synthesized M gene (corresponding to nt 2496-3104 from Genbank accession number FJ913470) (Figure 4C, SEQ ID NO: 3) as template. The PCR product was cloned in 2X35S/CPMV-HT/NOS expression system using In-Fusion cloning system (Clontech, Mountain View, CA). Construct 1193 (Figure 5A, SEQ ID NO: B1) was digested with SacII and StuI restriction enzyme and the linearized plasmid was used for the In-Fusion assembly reaction. Construct number 1193 is an acceptor plasmid intended for "In Fusion" cloning of genes of interest in a CPMV-HT-based expression cassette into the BeYDV amplification system. It also incorporates a gene construct for the co-expression of the TBSV P19 suppressor of silencing under the alfalfa Plastocyanin gene promoter and terminator. The backbone of construct number 1193 is a pCAMBIA binary plasmid and the sequence from left to right t-DNA borders is presented in Figure 5B (SEQ ID NO: 7). The resulting construct was given number 1086 (Figure 5C, SEQ ID NO: 8). The amino acid sequence of M protein from Rabies virus ERA strain is presented in Figure 4G (SEQ ID NO: 6). A representation of plasmid 1086 is presented in Figure 5D.

### C-2X35S/CPMV-HT/PDISP/Rabies G/NOS (Construct number 1071)

A sequence encoding G protein from Rabies virus ERA strain was cloned into 2X35S-CPMV-HT-PDISP-NOS expression system in a plasmid containing Plasto_pro/P19/Plasto_ter expression cassette using the following PCR-based method. A fragment containing the G protein coding sequence without its wild type signal peptide was amplified using primersIF-RabG-S2+4.c (Figure 6A, SEQ ID NO:9) and IF-RabG-S1-4.r (Figure 6B, SEQ ID NO: 10), using synthesized G gene (Corresponding to nt 3317-4891 from Genbank accession number EF206707) (Figure 6C, SEQ ID NO: 11) as template. The PCR product was cloned in-frame with alfalfa PDI signal peptide in 2X35S/CPMV-HT/NOS expression system using In-Fusion cloning system (Clontech, Mountain View, CA). Construct 1192 (Figure 6D) was digested with SacII and StuI restriction enzyme and the linearized plasmid was used for the In-Fusion assembly reaction. Construct number 1192 is an acceptor plasmid intended for "In Fusion" cloning of genes of interest in frame with an alfalfa PDI signal peptide in a CPMV-HT-based expression cassette. It also incorporates a gene construct for the co-expression of the TBSV P19 suppressor of silencing under the alfalfa Plastocyanin gene promoter and terminator. The backbone of construct 1192 is a pCAMBIA binary plasmid and the sequence from left to right t-DNA borders is presented in Figure 6E (SEQ ID NO: 12). The resulting construct was given number 1071(Figure 6F, SEQ ID NO: 13). The amino acid sequence of PDISP/G protein from Rabies virus ERA strain is presented in Figure 6G (SEQ ID NO: 14). A representation of plasmid 1071 is presented in Figure 6H.

### D-2X35S/CPMV-HT/PDISP/Rabies G/NOSintoBeYDV+Replicase amplification system (Construct number 1091)

A sequence encoding G protein from Rabies virus ERA strain was cloned into 2X35S/CPMV-HT/PDISP/NOS comprising the BeYDV+replicase amplification system in a plasmid containing Plasto_pro/P19/Plasto_ter expression cassette using the following PCR-based method. A fragment containing Rabies G protein coding sequence without his wild type signal peptide was amplified using primers IF-RabG-S2+4.c (Figure 6A, SEQ ID NO: 9) and IF-RabG-S1-4.r (Figure 6B, SEQ ID NO: 10), using synthesized G gene (Corresponding to nt 3317-4891 from Genbank accession number EF206707) (Figure 6C, SEQ ID NO: 11) as template. The PCR product was cloned in-frame with alfalfa PDI signal peptide in 2X35S/CPMV-HT/NOS expression cassette into the BeYDV amplification system using In-Fusion cloning system (Clontech, Mountain View, CA). Construct number 1194 (Figure 7A) was digested with SacII and StuI restriction enzyme and the linearized plasmid was used for the In-Fusion assembly reaction. Construct number 1194 is an acceptor plasmid intended for "In Fusion" cloning of genes of interest in frame with an alfalfa PDI signal peptide in a CPMV-HT-based expression cassette into the BeYDV amplification system. It also incorporates a gene construct for the co-expression of the TBSV P19 suppressor of silencing under the alfalfa Plastocyanin gene promoter and terminator. The backbone of construct number 1194 is a pCAMBIA binary plasmid and the sequence from left to right t-DNA borders is presented in Figure 7B (SEQ ID NO: 15). The resulting construct was given number 1091 (Figure 7C, SEQ ID NO: 16). The amino acid sequence of Influenza PDISP/G protein from Rabies virus ERA strain is presented in Figure 6G (SEQ ID NO: 14). A representation of plasmid1091 is presented in Figure 7D.

### Example 2: Preparation of plant biomass, inoculum and agro infiltration

The terms "biomass" and "plant matter" as used herein are meant to reflect any material derived from a plant. Biomass or plant matter may comprise an entire plant, tissue, cells, or any fraction thereof. Further, biomass or plant matter may comprise intracellular plant components, extracellular plant components, liquid or solid extracts of plants, or a combination thereof. Further, biomass or plant matter may comprise plants, plant cells, tissue, a liquid extract, or a combination thereof, from plant leaves, stems, fruit, roots or a combination thereof. A portion of a plant may comprise plant matter or biomass.

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* transfected with each construct were grown in a YEB medium supplemented with 10 mM 2-(N-morpholino)ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl₂ and 10 mM MES pH 5.6) and stored overnight at 4°C. On the day of infiltration, culture batches were diluted in 2.5 culture volumes and allowed to warm before use. Whole plants of *N. benthamiana* were placed upside down in the bacterial suspension in an air-tight stainless steel tank under a vacuum of 20-40 Torr for 2-min. Plants were returned to the greenhouse for a 2-6 day incubation period until harvest.

*A. tumefaciens* strains comprising the various constructs as described herein are referred to by using an "AGL1"prefix. For example *A. tumefaciens* comprising construct number 1091 is termed "AGL1/1091".

### Leaf harvest and total protein extraction (mechanical extraction)

Following incubation, the aerial part of plants was harvested, frozen at -80°C and crushed into pieces. Total soluble proteins were extracted by homogenizing (Polytron) each sample of frozen-crushed plant material in 3 volumes of cold 50 mM Tris pH 8.0, 150 mM NaCl, 0.1% Triton X-100 and 1 mM phenylmethanesulfonyl fluoride. After homogenization, the slurries were centrifuged at 10,000 g for 10 min at 4°C and these clarified crude extracts (supernatant) kept for analysis.

### Leaf harvest and total protein extraction (biochemical extraction)

Following incubation, the aerial part of plants was harvested and cut into small pieces (3 mm square) with a rolling cutter and special care was taken not to crush the leaves. Cut biomass was incubated 15-17 hours in 2.5 volumes of 200 mM mannitol, 125 mM citrate, 75 mM NaPO₄, 500 mM NaCl, 25 mM ethylenediaminetetraacetic acid, 1% (v/v) Multifect CX CG (Genencor, Cat. No. A03140G190), 1% (v/v) Multifect CX B (Genencor, Cat. No. A03042G190) and 1% (v/v) Multifect Pectinase FE (Genencor, Cat. No. A02080G190) pH 6.5 at 21°C under agitation at 100-200 rpm. Digested biomass was then filtered on Miracloth (Calbiochem, Cat 475855) and centrifuged at 10,000 g for 10 min at 4°C. The supernatant was transferred to a clean tube and centrifuged again under the same conditions and the supernatant was kept for analysis.

### Protein analysis and immunoblotting

The total protein content of clarified crude extracts was determined by the Bradford assay (Bio-Rad, Hercules, CA) using bovine serum albumin as the reference standard. Proteins were separated by SDS-PAGE and electrotransferred onto polyvinylene difluoride (PVDF) membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C. Immunoblotting was performed by incubation with 0.25 µg/µl of a Santa Cruz SC-57995 primary antibody in 2% skim milk in TBS-Tween 20 0.1%. Chemiluminescence detection was carried on after incubation with peroxidase-conjugated goat anti mouse, (JIR, 115-035-146) secondary antibody diluted 1:10,000 in 2% skim milk in TBS-Tween 20 0.1%. Immunoreactive complexes were detected by chemiluminescence using luminol as the substrate (Roche Diagnostics Corporation).

### Size exclusion chromatography of protein extract

For size exclusion chromatography (SEC), supernatant from biochemically extracted biomass was concentrated by centrifugation at 70,000 g for 20 min at 4°C and the resulting pellet was resuspended in 1/50 volume of cold 50 mM Tris pH 8.0, 150 mM NaCl. Size exclusion chromatography columns of 32 ml Sephacryl™ S-500 high resolution beads (S-500 HR: GE Healthcare, Uppsala, Sweden, Cat. No. 17-0613-10) were packed and equilibrated with equilibration/elution buffer (50 mM Tris pH8, 150 mM NaCl). One and a half millilitre of concentrated extract was loaded onto the column followed by an elution step with 45 mL of equilibration/elution buffer. The elution was collected in fractions of 1.5 mL relative protein content of eluted fractions was monitored by mixing 10 µL of the fraction with 200 µL of diluted Bio-Rad protein dye reagent (Bio-Rad, Hercules, CA). Two hundred microliters from each fraction were precipitated by addition of 5 volumes of ice cold acetone followed by freezing overnight at -20°C. Precipitated proteins were pelleted by centrifugation at 20000 g for 10 min. (4°C) and resuspended in 50 µl of hot SDS-PAGE sample loading buffer. The column was washed with 2 column volumes of 0.2N NaOH followed by 10 column volumes of 50 mM Tris pH8, 150 mM NaCl, 20% ethanol. Each separation was followed by a calibration of the column with Blue Dextran 2000 (GE Healthcare Bio-Science Corp., Piscataway, NJ, USA). Elution profiles of Blue Dextran 2000 and host soluble proteins were compared between each separation to ensure uniformity of the elution profiles between the columns used.

### Example 3: Evaluation of rabies G protein expression and co-expression strategies.

*Nicotiana benthamiana* plants were agro-infiltrated with AGL1/1071 (with or without AGL1/1066) or AGL1/1091 (with or without AGL1/1086) inoculums at different concentration and leaves were harvested after 5 days post infiltration (DPI) for 1071- and 1071 +1066-infiltrated plants or 3 to 4 DPI for 1091- and 1091+1086-infiltrated plants. Western blot analysis of leaf protein extracts from transformed plants showed that the BeYDV elements were required to reach a detectable G protein accumulation level (compare 1071- and 1091-infiltrated plants in figure 1). Maximum accumulation level was reached at 3 DPI for plants infiltrated with AGL1/1091 with or without co-expression of M protein (AGL1/1086) (Figure 1).

The biochemical extraction method was compared to the mechanical extraction for its capacity to release rabies G protein from the leaves of transformed plants. Western blot analysis of extracts obtained by biochemical extraction and mechanical extraction on 1091- and 1091+1086-infiltrated plants showed that the protein extracts from biochemical extraction contained significantly more G protein than the extract from mechanical extraction (Figure 2).

Protein extracts from 1091- and 1091+1086-infiltrated plants were subjected to size exclusion chromatography to assess their assembly into high molecular weight structures. Elution fractions were collected and an aliquot of each fraction was concentrated by acetone precipitation and analyzed for protein G content by western blot. As shown in figure 3A, protein G content peaked in SEC elution fractions 8 to 10, corresponding to the void volume of the column where enveloped VLPs are expected to be found. A similar elution profile was obtained when separating, by SEC, protein extracts from plants expressing rabies protein G with protein M (1091+1086-infiltrated plants, figure 3B).

### Example 4: Animal studies

### Rapid purification of Rab-VLP from plants

*N. benthamiana* plants were agroinfiltrated with AGL1/1091 as described for example in WO/2011/035422 which is incorporated herein by reference. Extraction of Rab-VLP (also referred to as NG-VLP, Native G protein VLP, G-VLP or G protein VLP) was undertaken as described above. Briefly, leaves were collected on day 4 post-infiltration, cut into ∼1 cm² pieces and digested for 15h at room temperature in an orbital shaker. The digestion buffer contained 1.0% (v/v) Multifect Pectinase FE, 1.0% (v/v) Multifect CX CG and/or 1.0% (v/v) Multifect CX B (all from Genencor), each in a solution of 200 mM Mannitol, 75 mM Citrate, 0.04%, 500 mM NaCl sodium bisulfite pH 6.0 buffer using a biomass : digestion buffer ratio of 1:2.5 (w/v).

Following digestion, the apoplastic fraction was filtered through a 400 µm nylon filter to remove coarse undigested vegetal tissue (<5% of starting biomass). The filtered extract was then centrifuged at room temperature for 15 min at 5000xg to remove protoplasts and intracellular contaminants (proteins, DNA, membranes, vesicles, pigments, etc). Next, the supernatant was depth-filtered (for clarification) using a 1.2 µm glass fiber filter (Sartorius Stedim) and a 0.45/0.2µm filter (Sartorium Stedim), before being subjected to chromatography.

In order to prepare for chromatography, the extract may be concentrated by suitable methods known in the art, for example the extract may be centrifuge and the pellet resuspended in appropriate buffer and volume or the extract may be concentrated and diafiltrated on tangential flow filtration (TFF) systems, equipped with appropriate membrane prior to loading on chromatographic media. Membranes may be flat-sheet or hollow fibres, pore size may range between 100, 300, 500, 750 kDa or 1 µ molecular weight cut-off. Lumen size may be chosen according to supplier line of product, usually 0.75 to 1 mm lumen size is preferable. Shear rates may be adjusted between 2000 s⁻¹ to 10 000 s⁻¹ with appropriate retentate and permeate flow rates.

The clarified apoplastic fraction was loaded over a cation exchange column (Poros HS Applied Biosystems) equilibrated with an equilibration/elution buffer (50 mM NaPO₄, 100 mM NaCl, 0.005% Tween 80 pH 6.0). Once the UV was back to zero, the extract was step-eluted with the equilibration/elution buffer containing increasing concentrations of NaCl (500 mM). Where necessary, the chromatographic fractions were concentrated 10 times using Amicon™ devices equipped with 10 kDa MWCO. Protein analysis was performed as described in previous examples.

Finally, a last formulation approach may be employ to buffer exchange the final candidate vaccine, by methods known in the art. For example a centrifugation or a TFF step may be used for such purpose, as described above.

Under conditions described as above, purity of 75% or greater for Rab-VLP vaccines preparation with sufficient quality for immunogenicity study may be obtained. As illustrated in figure 8A, the G protein migrates to the expected molecular weight of ca. 55 kDa. The size of the Rab-VLP is estimated to be between 175-190 nm by dymamic light scattering (Zeta sizer 90, Malvern instrument), a particle size similar to that described for the Rabies virus.

### Immunization studies in mice

Immunogenicity of the Rab-VLP was evaluated in the Balb/c mouse model. Briefly, groups of female BALB/c mice (8-10 weeks old; Charles River Laboratories, USA) were vaccinated with a total of three injections (0.05 mL/site) on each immunization day (Study Days 0, 7 and 28) in the left and right hind limb musculature intramuscularly (i.m.) for a total of 0.1 ml volume injected that contained various doses of Rab-VLP vaccine (1 or 5µg- with or without adjuvants). The doses were based upon G protein concentrations determined by bicinchoninic acid (BCA) assay combined with purity assessment by densitometry. Alhydrogel® at final concemtration of 1% was used as the adjuvant. The placebo group was immunized by the same route and regimen as the candidate vaccine. Fifteen mice per group were used to provide adequate statistical power to the study. Serum samples were collected prior vaccination (pre-immune sera) and on day 7, 21 and 44. Five animals per group were sacrificed on day 7, 21 and 44 in order to perform rapid fluorescent focus inhibition test (RFFIT) to evaluate the protective antibody in the sera. The protective dose established by the World Health Organization (WHO) for Rabies vaccine is 0.5 international unit (IU) per ml. As shown in figure 8b, the non-adjuvanted Rab-VLP vaccine, with doses as low as 1 µg, allows for achieving higher titers than the standard titers established by the WHO.

## Claims

1. A method of producing a rabies virus like particle (VLP) in a plant comprising,
a) providing a plant or portion of the plant comprising a nucleic acid comprising a regulatory region active in the plant operatively linked to a nucleotide sequence encoding one or more native rabies glycoprotein (G), wherein the nucleotide sequence further encodes, comprises, or both encodes and comprises, one or more than one amplification element comprising one or more long intergenic regions or long intergenic repeat of a geminivirus genome,
b) incubating the plant or portion of the plant under conditions that permit the expression of the nucleic acid, thereby producing the rabies VLP, the rabies VLP produced by self-assembly following expression of the native rabies glycoprotein and budding of the rabies VLP from a plasma membrane,
c) harvesting the plant, and
d) extracting the VLPs, wherein the VLPs range in size from 40-300 nm, and
wherein the VLP comprises a lipid envelope comprising lipids obtained from the plasma membrane of the plant,
optionally the plant or portion of the plant further comprises a second nucleic acid comprising a second regulatory region active in the plant and operatively linked to a second nucleotide sequence encoding a rabies matrix protein (M) or fragment thereof and is expressed when incubating the plant or portion of the plant in step b), and/or
the VLP does not contain a viral matrix or a core protein.

2. The method of claim 1, wherein prior to the step of providing, the nucleic acid comprising the regulatory region active in the plant and operatively linked to the nucleotide sequence encoding the native rabies glycoprotein (G) is introduced by transformation into the plant, or portion of the plant.

3. The method of claim 1 or 2, wherein the one or more than one amplification element is from a Bean Yellow Dwarf Virus.

4. The method of claim 2, wherein in the step of introducing, the nucleic acid is transiently expressed in the plant or the nucleic acid is stably expressed in the plant.

5. The method of any one of claims 1-4, wherein the VLP is extracted by biochemical extraction.

6. A plant derived VLP produced by the method of any one of claims 1-5, the envelope of the VLP comprising a native rabies glycoprotein (G) and lipids obtained from the plasma membrane of the plant.

7. The plant derived VLP of claim 6, wherein the lipid comprises one or more than one lipid derived from the plant and/or wherein one or more glycoprotein (G) comprises plant-specific N-glycans, or modified N-glycans.

8. A composition comprising an effective dose of the plant derived VLP of claim 6 or 7 for use in inducing an immune response in a subject, and a pharmaceutically acceptable carrier.

9. The plant derived VLP of claim 6 or 7 for use in inducing immunity to a rabies virus infection in a subject.

10. A food supplement comprising the plant derived VLP of claim 6 or 7.

11. The method of claim 2, further comprising introducing by transformation an additional nucleic acid, the additional nucleic acid comprising a nucleotide sequence encoding a suppressor of silencing, a geminivirus replicase or both, preferably the suppressor of silencing is selected from the group HCPro and p19 and the suppressor of silencing and the geminivirus replicase are encoded by two different additional nucleic acids that are introduced into the plant, or portion of the plant.

12. The method of claim 1, wherein the native rabies glycoprotein (G) has at least 90% sequence identity to SEQ ID NO: 14 or wherein the native rabies glycoprotein (G) has the amino acid sequence shown as in SEQ ID NO: 14.

13. The method of claim 1, wherein the rabies matrix protein (M) has at least 90% sequence identity to SEQ ID NO: 6 or wherein the rabies matrix protein (M) has the amino acid sequence shown as in SEQ ID NO: 6.

14. The method of claim 1, wherein the nucleic acid comprising the regulatory region is further operatively linked with one or more than one comovirus enhancer, a comovirus UTR, or a Cowpea Mosaic Virus (CPMV) UTR.

## Patentansprüche

1. Verfahren zum Herstellen eines Rabiesvirus-ähnlichen Partikels (Virus Like Particle - VLP) in einer Pflanze, umfassend:
a) Bereitstellen einer Pflanze oder eines Teils der Pflanze, die eine Nukleinsäure umfasst, die eine regulatorische Region umfasst, die in der Pflanze aktiv ist, die operativ mit einer Nukleotidsequenz verbunden ist, die ein oder mehrere native Rabies-Glycoproteine (G) codiert, wobei die Nukleotidsequenz ferner ein oder mehr als ein Verstärkungselement codiert, umfasst oder sowohl codiert als auch umfasst, das eine oder mehrere lange intergene Regionen oder lange intergene Wiederholungen eines Geminivirusgenoms umfasst,
b) Inkubieren der Pflanze oder eines Teils der Pflanze unter Bedingungen, die die Expression der Nukleinsäure erlauben, wodurch das Rabies-VLP erzeugt wird, wobei das Rabies-VLP, das durch Selbstzusammensetzung erzeugt wird, der Expression des nativen Rabies-Glycoproteins folgt und sich aus dem Rabies-VLP von einer Plasmamembran neubildet,
c) Ernten der Pflanze, und
d) Extrahieren der VLPs, wobei die Größe der VLPs von 40-300 nm reicht, und
wobei das VLP eine Lipidhülle umfasst, die Lipide umfasst, die von der Plasmamembran der Pflanze erhalten werden,
wobei die Pflanze oder ein Teil der Pflanze optional ferner eine zweite Nukleinsäure umfasst, die eine zweite regulatorische Region umfasst, die in der Pflanze aktiv ist, und operativ mit einer zweiten Nukleotidsequenz verbunden ist, die ein Rabies-Matrixprotein (M) oder Fragmente davon codiert, und exprimiert wird, wenn die Pflanze oder ein Teil der Pflanze in Schritt b) inkubiert wird, und/oder das VLP weder eine virale Matrix noch ein Kernprotein enthält.

2. Verfahren nach Anspruch 1, wobei die Nukleinsäure, die die regulatorische Region umfasst, die in der Pflanze aktiv ist, und operativ mit der Nukleotidsequenz verbunden ist, die das native Rabies-Glycoprotein (G) codiert, vor dem Schritt des Bereitstellens durch Transformation in die Pflanze oder einen Teil der Pflanze eingeführt wird.

3. Verfahren nach Anspruch 1 oder 2, wobei das eine oder mehr als ein Verstärkungselement aus einem Bohnengelbverzwergungsvirus stammt.

4. Verfahren nach Anspruch 2, wobei die Nukleinsäure im Schritt des Einführens transient in der Pflanze exprimiert wird oder die Nukleinsäure stabil in der Pflanze exprimiert wird.

5. Verfahren nach einem der Ansprüche 1-4, wobei das VLP durch biochemische Extraktion extrahiert wird.

6. Aus Pflanzen stammendes VLP, das durch das Verfahren nach einem der Ansprüche 1-5 hergestellt wird, wobei die Hülle des VLP ein natives Rabies-Glycoprotein (G) und Lipide, die aus der Plasmamembran der Pflanze erhalten wurden, umfasst.

7. Aus Pflanzen stammendes VLP nach Anspruch 6, wobei das Lipid ein oder mehr als ein Lipid umfasst, das aus der Pflanze stammt, und/oder wobei ein oder mehrere Glycoproteine (G) pflanzenspezifische N-Glykane oder modifizierte N-Glykane umfasst.

8. Zusammensetzung, umfassend eine effektive Dosis des aus Pflanzen stammenden VLP nach Anspruch 6 oder 7 zur Verwendung beim Induzieren einer Immunreaktion in einem Subjekt und einen pharmazeutisch akzeptablen Träger.

9. Aus Pflanzen stammendes VLP nach Anspruch 6 oder 7 zur Verwendung beim Induzieren von Immunität gegen eine Rabiesvirus-Infektion in einem Subjekt.

10. Nahrungsergänzungsmittel, umfassend das von Pflanzen stammende VLP nach Anspruch 6 oder 7.

11. Verfahren nach Anspruch 2, ferner umfassend das Einführen einer zusätzlichen Nukleinsäure durch Transformation, wobei die zusätzliche Nukleinsäure eine Nukleotidsequenz umfasst, die einen Abschaltungssuppressor, eine Geminivirus-Replikase oder beides codiert, wobei der Abschaltungssuppressor vorzugsweise aus der Gruppe HCPro und p19 ausgewählt wird und der Abschaltungssuppressor und die Geminivirus-Replikase von zwei unterschiedlichen zusätzlichen Nukleinsäuren codiert werden, die in die Pflanze oder einen Teil der Pflanze eingeführt werden.

12. Verfahren nach Anspruch 1, wobei das native Rabies-Glycoprotein (G) eine Sequenzidentität zu SEQ ID NO: 14 von mindestens 90 % aufweist oder wobei das native Rabies-Glycoprotein (G) die in SEQ ID NO: 14 gezeigte Aminosäuresequenz aufweist.

13. Verfahren nach Anspruch 1, wobei das Rabies-Matrixprotein (M) eine Sequenzidentität zu SEQ ID NO: 6 von mindestens 90 % aufweist oder wobei das Rabies-Matrixprotein (M) die in SEQ ID NO: 6 gezeigte Aminosäuresequenz aufweist.

14. Verfahren nach Anspruch 1, wobei die Nukleinsäure, die die regulatorische Region umfasst, ferner operativ mit einem oder mehr als einem Comovirus-Verstärker, einer Comovirus-UTR oder einer Cowpea-Mosaic-Virus-(CMPV)-UTR verbunden ist.

## Revendications

1. Procédé de production d'une particule analogue à un virus (PAV) de la rage dans une plante comprenant
a) la fourniture d'une plante ou d'une partie de la plante comprenant un acide nucléique comprenant une région régulatrice active dans la plante liée de manière fonctionnelle à une séquence nucléotidique codant une ou plusieurs glycoprotéines (G) de la rage natives, où, en outre, la séquence nucléotidique code, comprend, ou code et comprend, un ou plus d'un élément d'amplification comprenant une ou plusieurs longues régions intergéniques ou longues répétitions intergéniques d'un génome de geminivirus,
b) l'incubation de la plante ou de la partie de la plante dans des conditions qui permettent l'expression de l'acide nucléique, pour produire ainsi la PAV de la rage, la PAV de la rage produite par auto-assemblage après l'expression de la glycoprotéine de la rage native et bourgeonnement de la PAV de la rage depuis une membrane plasmique,
c) la récolte de la plante, et
d) l'extraction des PAV, où les PAV vont en dimension de 40-300 nm, et
où la PAV comprend une enveloppe lipidique comprenant des lipides obtenus à partir de la membrane plasmique de la plante,
éventuellement la plante ou la partie de la plante comprend en outre un second acide nucléique comprenant une seconde région régulatrice active dans la plante et liée de manière fonctionnelle à une seconde séquence nucléotidique codant une protéine de matrice (M) de la rage ou un fragment de celle-ci et est exprimé lors de l'incubation de la plante ou de la partie de la plante dans l'étape b), et/ou
la PAV ne contient pas de protéine de matrice ou de nucléocapside virale.

2. Procédé selon la revendication 1, où avant l'étape de fourniture, l'acide nucléique comprenant la région régulatrice active dans la plante et liée de manière fonctionnelle à la séquence nucléotidique codant la glycoprotéine (G) de la rage native est introduit par transformation dans la plante, ou la partie de la plante.

3. Procédé selon la revendication 1 ou 2, où le un ou plus d'un élément d'amplification provient d'un virus du nanisme jaune du haricot.

4. Procédé selon la revendication 2, où dans l'étape d'introduction, l'acide nucléique est exprimé de manière transitoire dans la plante ou l'acide nucléique est exprimé de manière stable dans la plante.

5. Procédé selon l'une quelconque des revendications 1 - 4, où la PAV est extraite par extraction biochimique.

6. PAV dérivée d'une plante produite par le procédé selon l'une quelconque des revendications 1-5, l'enveloppe de la PAV comprenant une glycoprotéine (G) de la rage native et des lipides obtenus à partir de la membrane plasmique de la plante.

7. PAV dérivée d'une plante selon la revendication 6, où le lipide comprend un ou plus d'un lipide dérivé de la plante et/ou où une ou plusieurs glycoprotéines (G) comprend des N-glycanes spécifiques de plante, ou des N-glycanes modifiés.

8. Composition comprenant une dose efficace de la PAV dérivée d'une plante selon la revendication 6 ou 7 destinée à être utilisée dans l'induction d'une réponse immunitaire chez un sujet, et un vecteur pharmaceutiquement acceptable.

9. PAV dérivée d'une plante selon la revendication 6 ou 7 destinée à être utilisée dans l'induction d'une immunité à l'égard d'une infection par un virus de la rage chez un sujet.

10. Complément alimentaire comprenant la PAV dérivée d'une plante selon la revendication 6 ou 7.

11. Procédé selon la revendication 2, comprenant en outre l'introduction par transformation d'un acide nucléique supplémentaire, l'acide nucléique supplémentaire comprenant une séquence nucléotidique codant un suppresseur d'inhibition, une réplicase de geminivirus ou les deux, de préférence le suppresseur d'inhibition est choisi dans le groupe HCPro et pl9 et le suppresseur d'inhibition et la réplicase de geminivirus sont codés par deux acides nucléiques supplémentaires différents qui sont introduits dans la plante, ou la partie de la plante.

12. Procédé selon la revendication 1, où la glycoprotéine (G) de la rage native a au moins 90 % d'identité de séquence avec SEQ ID NO: 14 ou bien où la glycoprotéine (G) de la rage native a la séquence d'acides aminés montrée dans SEQ ID NO: 14.

13. Procédé selon la revendication 1, où la protéine de matrice (M) de la rage a au moins 90 % d'identité de séquence avec SEQ ID NO: 6 ou bien où la protéine de matrice (M) de la rage a la séquence d'acides aminés montrée dans SEQ ID NO: 6.

14. Procédé selon la revendication 1, où l'acide nucléique comprenant la région régulatrice est en outre lié de manière fonctionnelle avec un ou plus d'un amplificateur de comovirus, une UTR de comovirus, ou une UTR de virus de la mosaïque du niébé (CPMV).


### SEQUENCE LISTING

<110> MEDICAGO INC.
   D'AOUST, Marc-Andre
   LAVOIE, Pierre-Olivier
   VEZINA, Louis-Philippe
   MANON, COUTURE
<120> Rabies Virus Like Production in Plants
<130> V83990WO
<150> US 61/496,371
   <151> 2011-06-13
<150> US 61/578,787
   <151> 2011-12-21
<160> 16
<170> PatentIn version 3.5
<210> 1
   <211> 37
   <212> DNA
   <213> Artificial Sequence
<220>
   <223> IF-RabM-S3.c
<400> 1
   aaatttgtcg ggcccatgaa ctttctacgt aagatag 37
<210> 2
   <211> 39
   <212> DNA
   <213> Artificial Sequence
<220>
   <223> IF-RabM-Sl-4.r
<400> 2
   actaaagaaa ataggccttt attctagaag cagagagga 39
<210> 3
   <211> 609
   <212> DNA
   <213> Artificial Sequence
<220>
   <223> Synthesized M protein coding sequence
<400> 3
   atgaactttc tacgtaagat agtgaaaaat tgcagggacg aggacactca aaaaccctct 60
   cccgtgtcag cccctctgga tgacgatgac ttgtggcttc caccccctga atacgtcccg 120
   ctgaaagaac ttacaagcaa gaagaacatg aggaactttt gtatcaacgg aggggttaaa 180
   gtgtgtagcc cgaatggtta ctcgttcagg atcctgcggc acattctgaa atcattcgac 240
   gagatatatt ctgggaatca taggatgatc gggttagtca aagtagttat tggactggct 300
   ttgtcaggat ctccagtccc tgagggcatg aactgggtat acaaattgag gagaaccttt 360
   atcttccagt gggctgattc caggggccct cttgaagggg aggagttgga atactctcag 420
   gagatcactt gggatgatga tactgagttc gtcggattgc aaataagagt gattgcaaaa 480
   cagtgtcata tccagggcag aatctggtgt atcaacatga acccgagagc atgtcaacta 540
   tggtctgaca tgtctcttca gacacaaagg tccgaagagg acaaagattc ctctctgctt 600
   ctagaataa 609
<210> 4
   <211> 4903
   <212> DNA
   <213> Artificial Sequence
<220>
   <223> Construct number 1191
<400> 4
   tggcaggata tattgtggtg taaacaaatt gacgcttaga caacttaata acacattgcg      60
   gacgttttta atgtactgaa ttaacgccga atcccgggct ggtatattta tatgttgtca     120
   aataactcaa aaaccataaa agtttaagtt agcaagtgtg tacattttta cttgaacaaa     180
   aatattcacc tactactgtt ataaatcatt attaaacatt agagtaaaga aatatggatg     240
   ataagaacaa gagtagtgat attttgacaa caattttgtt gcaacatttg agaaaatttt     300
   gttgttctct cttttcattg gtcaaaaaca atagagagag aaaaaggaag agggagaata     360
   aaaacataat gtgagtatga gagagaaagt tgtacaaaag ttgtaccaaa atagttgtac     420
   aaatatcatt gaggaatttg acaaaagcta cacaaataag ggttaattgc tgtaaataaa     480
   taaggatgac gcattagaga gatgtaccat tagagaattt ttggcaagtc attaaaaaga     540
   aagaataaat tatttttaaa attaaaagtt gagtcatttg attaaacatg tgattattta     600
   atgaattgat gaaagagttg gattaaagtt gtattagtaa ttagaatttg gtgtcaaatt     660
   taatttgaca tttgatcttt tcctatatat tgccccatag agtcagttaa ctcattttta     720
   tatttcatag atcaaataag agaaataacg gtatattaat ccctccaaaa aaaaaaaacg     780
   gtatatttac taaaaaatct aagccacgta ggaggataac aggatccccg taggaggata     840
   acatccaatc caaccaatca caacaatcct gatgagataa cccactttaa gcccacgcat     900
   ctgtggcaca tctacattat ctaaatcaca cattcttcca cacatctgag ccacacaaaa     960
   accaatccac atctttatca cccattctat aaaaaatcac actttgtgag tctacacttt    1020
   gattcccttc aaacacatac aaagagaaga gactaattaa ttaattaatc atcttgagag    1080
   aaaatggaac gagctataca aggaaacgac gctagggaac aagctaacag tgaacgttgg    1140
   gatggaggat caggaggtac cacttctccc ttcaaacttc ctgacgaaag tccgagttgg    1200
   actgagtggc ggctacataa cgatgagacg aattcgaatc aagataatcc ccttggtttc 1260
   aaggaaagct ggggtttcgg gaaagttgta tttaagagat atctcagata cgacaggacg 1320
   gaagcttcac tgcacagagt ccttggatct tggacgggag attcggttaa ctatgcagca 1380
   tctcgatttt tcggtttcga ccagatcgga tgtacctata gtattcggtt tcgaggagtt 1440
   agtatcaccg tttctggagg gtcgcgaact cttcagcatc tctgtgagat ggcaattcgg 1500
   tctaagcaag aactgctaca gcttgcccca atcgaagtgg aaagtaatgt atcaagagga 1560
   tgccctgaag gtactcaaac cttcgaaaaa gaaagcgagt aagttaaaat gcttcttcgt 1620
   ctcctattta taatatggtt tgttattgtt aattttgttc ttgtagaaga gcttaattaa 1680
   tcgttgttgt tatgaaatac tatttgtatg agatgaactg gtgtaatgta attcatttac 1740
   ataagtggag tcagaatcag aatgtttcct ccataactaa ctagacatga agacctgccg 1800
   cgtacaattg tcttatattt gaacaactaa aattgaacat cttttgccac aactttataa 1860
   gtggttaata tagctcaaat atatggtcaa gttcaataga ttaataatgg aaatatcagt 1920
   tatcgaaatt cattaacaat caacttaacg ttattaacta ctaattttat atcatcccct 1980
   ttgataaatg atagtacacc aattaggaag gagcatgctc gcctaggaga ttgtcgtttc 2040
   ccgccttcag tttgcaagct gctctagccg tgtagccaat acgcaaaccg cctctccccg 2100
   cgcgttggga attactagcg cgtgtcgaca agcttgcatg ccggtcaaca tggtggagca 2160
   cgacacactt gtctactcca aaaatatcaa agatacagtc tcagaagacc aaagggcaat 2220
   tgagactttt caacaaaggg taatatccgg aaacctcctc ggattccatt gcccagctat 2280
   ctgtcacttt attgtgaaga tagtggaaaa ggaaggtggc tcctacaaat gccatcattg 2340
   cgataaagga aaggccatcg ttgaagatgc ctctgccgac agtggtccca aagatggacc 2400
   cccacccacg aggagcatcg tggaaaaaga agacgttcca accacgtctt caaagcaagt 2460
   ggattgatgt gataacatgg tggagcacga cacacttgtc tactccaaaa atatcaaaga 2520
   tacagtctca gaagaccaaa gggcaattga gacttttcaa caaagggtaa tatccggaaa 2580
   cctcctcgga ttccattgcc cagctatctg tcactttatt gtgaagatag tggaaaagga 2640
   aggtggctcc tacaaatgcc atcattgcga taaaggaaag gccatcgttg aagatgcctc 2700
   tgccgacagt ggtcccaaag atggaccccc acccacgagg agcatcgtgg aaaaagaaga 2760
   cgttccaacc acgtcttcaa agcaagtgga ttgatgtgat atctccactg acgtaaggga 2820
   tgacgcacaa tcccactatc cttcgcaaga cccttcctct atataaggaa gttcatttca 2880
   tttggagagg tattaaaatc ttaataggtt ttgataaaag cgaacgtggg gaaacccgaa 2940
   ccaaaccttc ttctaaactc tctctcatct ctcttaaagc aaacttctct cttgtctttc 3000
   ttgcgtgagc gatcttcaac gttgtcagat cgtgcttcgg caccagtaca acgttttctt 3060
   tcactgaagc gaaatcaaag atctctttgt ggacacgtag tgcggcgcca ttaaataacg 3120
   tgtacttgtc ctattcttgt cggtgtggtc ttgggaaaag aaagcttgct ggaggctgct 3180
   gttcagcccc atacattact tgttacgatt ctgctgactt tcggcgggtg caatatctct 3240
   acttctgctt gacgaggtat tgttgcctgt acttctttct tcttcttctt gctgattggt 3300
   tctataagaa atctagtatt ttctttgaaa cagagttttc ccgtggtttt cgaacttgga 3360
   gaaagattgt taagcttctg tatattctgc ccaaatttgt cgggcccgcg gatggcgaaa 3420
   aacgttgcga ttttcggctt attgttttct cttcttgtgt tggttccttc tcagatcttc 3480
   gcctgcaggc tcctcagcca aaacgacacc cccatctgtc tatccactgg cccctggatc 3540
   tgctgcccaa actaactcca tggtgaccct gggatgcctg gtcaagggct atttccctga 3600
   gccagtgaca gtgacctgga actctggatc cctgtccagc ggtgtgcaca ccttcccagc 3660
   tgtcctgcag tctgacctct acactctgag cagctcagtg actgtcccct ccagcacctg 3720
   gcccagcgag accgtcacct gcaacgttgc ccacccggcc agcagcacca aggtggacaa 3780
   gaaaattgtg cccagggatt gtggttgtaa gccttgcata tgtacagtcc cagaagtatc 3840
   atctgtcttc atcttccccc caaagcccaa ggatgtgctc accattactc tgactcctaa 3900
   ggtcacgtgt gttgtggtag acatcagcaa ggatgatccc gaggtccagt tcagctggtt 3960
   tgtagatgat gtggaggtgc acacagctca gacgcaaccc cgggaggagc agttcaacag 4020
   cactttccgc tcagtcagtg aacttcccat catgcaccag gactggctca atggcaagga 4080
   gcgatcgctc accatcacca tcaccatcac catcaccatt aaaggcctat tttctttagt 4140
   ttgaatttac tgttattcgg tgtgcatttc tatgtttggt gagcggtttt ctgtgctcag 4200
   agtgtgttta ttttatgtaa tttaatttct ttgtgagctc ctgtttagca ggtcgtccct 4260
   tcagcaagga cacaaaaaga ttttaatttt attaaaaaaa aaaaaaaaaa agaccgggaa 4320
   ttcgatatca agcttatcga cctgcagatc gttcaaacat ttggcaataa agtttcttaa 4380
   gattgaatcc tgttgccggt cttgcgatga ttatcatata atttctgttg aattacgtta 4440
   agcatgtaat aattaacatg taatgcatga cgttatttat gagatgggtt tttatgatta 4500
   gagtcccgca attatacatt taatacgcga tagaaaacaa aatatagcgc gcaaactagg 4560
   ataaattatc gcgcgcggtg tcatctatgt tactagatct ctagagtctc aagcttggcg 4620
   cgcccacgtg actagtggca ctggccgtcg ttttacaacg tcgtgactgg gaaaaccctg 4680
   gcgttaccca acttaatcgc cttgcagcac atcccccttt cgccagctgg cgtaatagcg 4740
   aagaggcccg caccgatcgc ccttcccaac agttgcgcag cctgaatggc gaatgctaga 4800
   gcagcttgag cttggatcag attgtcgttt cccgccttca gtttaaacta tcagtgtttg 4860
   acaggatata ttggcgggta aacctaagag aaaagagcgt tta 4903
<210> 5
   <211> 2349
   <212> DNA
   <213> Artificial Sequence
<220>
   <223> Expression cassette number 1066
<400> 5
   gtcaacatgg tggagcacga cacacttgtc tactccaaaa atatcaaaga tacagtctca 60
   gaagaccaaa gggcaattga gacttttcaa caaagggtaa tatccggaaa cctcctcgga 120
   ttccattgcc cagctatctg tcactttatt gtgaagatag tggaaaagga aggtggctcc 180
   tacaaatgcc atcattgcga taaaggaaag gccatcgttg aagatgcctc tgccgacagt 240
   ggtcccaaag atggaccccc acccacgagg agcatcgtgg aaaaagaaga cgttccaacc 300
   acgtcttcaa agcaagtgga ttgatgtgat aacatggtgg agcacgacac acttgtctac 360
   tccaaaaata tcaaagatac agtctcagaa gaccaaaggg caattgagac ttttcaacaa 420
   agggtaatat ccggaaacct cctcggattc cattgcccag ctatctgtca ctttattgtg 480
   aagatagtgg aaaaggaagg tggctcctac aaatgccatc attgcgataa aggaaaggcc 540
   atcgttgaag atgcctctgc cgacagtggt cccaaagatg gacccccacc cacgaggagc 600
   atcgtggaaa aagaagacgt tccaaccacg tcttcaaagc aagtggattg atgtgatatc 660
   tccactgacg taagggatga cgcacaatcc cactatcctt cgcaagaccc ttcctctata 720
   taaggaagtt catttcattt ggagaggtat taaaatctta ataggttttg ataaaagcga 780
   acgtggggaa acccgaacca aaccttcttc taaactctct ctcatctctc ttaaagcaaa 840
   cttctctctt gtctttcttg cgtgagcgat cttcaacgtt gtcagatcgt gcttcggcac 900
   cagtacaacg ttttctttca ctgaagcgaa atcaaagatc tctttgtgga cacgtagtgc 960
   ggcgccatta aataacgtgt acttgtccta ttcttgtcgg tgtggtcttg ggaaaagaaa 1020
   gcttgctgga ggctgctgtt cagccccata cattacttgt tacgattctg ctgactttcg 1080
   gcgggtgcaa tatctctact tctgcttgac gaggtattgt tgcctgtact tctttcttct 1140
   tcttcttgct gattggttct ataagaaatc tagtattttc tttgaaacag agttttcccg 1200
   tggttttcga acttggagaa agattgttaa gcttctgtat attctgccca aatttgtcgg 1260
   gcccatgaac tttctacgta agatagtgaa aaattgcagg gacgaggaca ctcaaaaacc 1320
   ctctcccgtg tcagcccctc tggatgacga tgacttgtgg cttccacccc ctgaatacgt 1380
   cccgctgaaa gaacttacaa gcaagaagaa catgaggaac ttttgtatca acggaggggt 1440
   taaagtgtgt agcccgaatg gttactcgtt caggatcctg cggcacattc tgaaatcatt 1500
   cgacgagata tattctggga atcataggat gatcgggtta gtcaaagtag ttattggact 1560
   ggctttgtca ggatctccag tccctgaggg catgaactgg gtatacaaat tgaggagaac 1620
   ctttatcttc cagtgggctg attccagggg ccctcttgaa ggggaggagt tggaatactc 1680
   tcaggagatc acttgggatg atgatactga gttcgtcgga ttgcaaataa gagtgattgc 1740
   aaaacagtgt catatccagg gcagaatctg gtgtatcaac atgaacccga gagcatgtca 1800
   actatggtct gacatgtctc ttcagacaca aaggtccgaa gaggacaaag attcctctct 1860
   gcttctagaa taaaggccta ttttctttag tttgaattta ctgttattcg gtgtgcattt 1920
   ctatgtttgg tgagcggttt tctgtgctca gagtgtgttt attttatgta atttaatttc 1980
   tttgtgagct cctgtttagc aggtcgtccc ttcagcaagg acacaaaaag attttaattt 2040
   tattaaaaaa aaaaaaaaaa aagaccggga attcgatatc aagcttatcg acctgcagat 2100
   cgttcaaaca tttggcaata aagtttctta agattgaatc ctgttgccgg tcttgcgatg 2160
   attatcatat aatttctgtt gaattacgtt aagcatgtaa taattaacat gtaatgcatg 2220
   acgttattta tgagatgggt ttttatgatt agagtcccgc aattatacat ttaatacgcg 2280
   atagaaaaca aaatatagcg cgcaaactag gataaattat cgcgcgcggt gtcatctatg 2340
   ttactagat                                                         2349
<210> 6
   <211> 202
   <212> PRT
   <213> Rabies virus
<400> 6
   Met Asn Phe Leu Arg Lys Ile Val Lys Asn Cys Arg Asp Glu Asp Thr
   1               5                   10                  15
   Gln Lys Pro Ser Pro Val Ser Ala Pro Leu Asp Asp Asp Asp Leu Trp
               20                  25                  30
   Leu Pro Pro Pro Glu Tyr Val Pro Leu Lys Glu Leu Thr Ser Lys Lys
           35                  40                  45
   Asn Met Arg Asn Phe Cys Ile Asn Gly Gly Val Lys Val Cys Ser Pro
       50                  55                  60
   Asn Gly Tyr Ser Phe Arg Ile Leu Arg His Ile Leu Lys Ser Phe Asp
   65                  70                  75                  80
   Glu Ile Tyr Ser Gly Asn His Arg Met Ile Gly Leu Val Lys Val Val
                   85                  90                  95
   Ile Gly Leu Ala Leu Ser Gly Ser Pro Val Pro Glu Gly Met Asn Trp
               100                 105                 110
   Val Tyr Lys Leu Arg Arg Thr Phe Ile Phe Gln Trp Ala Asp Ser Arg
           115                 120                 125
   Gly Pro Leu Glu Gly Glu Glu Leu Glu Tyr Ser Gln Glu Ile Thr Trp
       130                 135                 140
   Asp Asp Asp Thr Glu Phe Val Gly Leu Gln Ile Arg Val Ile Ala Lys
   145                 150                 155                 160
   Gln Cys His Ile Gln Gly Arg Ile Trp Cys Ile Asn Met Asn Pro Arg
                   165                 170                 175
   Ala Cys Gln Leu Trp Ser Asp Met Ser Leu Gln Thr Gln Arg Ser Glu
               180                 185                 190
   Glu Asp Lys Asp Ser Ser Leu Leu Leu Glu
           195                 200
<210> 7
   <211> 6789
   <212> DNA
   <213> Artificial Sequence
<220>
   <223> Construct No. 1193
<400> 7
   tggcaggata tattgtggtg taaacaaatt gacgcttaga caacttaata acacattgcg    60
   gacgttttta atgtactgaa ttaacgccga atcccgggct ggtatattta tatgttgtca   120
   aataactcaa aaaccataaa agtttaagtt agcaagtgtg tacattttta cttgaacaaa   180
   aatattcacc tactactgtt ataaatcatt attaaacatt agagtaaaga aatatggatg   240
   ataagaacaa gagtagtgat attttgacaa caattttgtt gcaacatttg agaaaatttt   300
   gttgttctct cttttcattg gtcaaaaaca atagagagag aaaaaggaag agggagaata   360
   aaaacataat gtgagtatga gagagaaagt tgtacaaaag ttgtaccaaa atagttgtac   420
   aaatatcatt gaggaatttg acaaaagcta cacaaataag ggttaattgc tgtaaataaa   480
   taaggatgac gcattagaga gatgtaccat tagagaattt ttggcaagtc attaaaaaga   540
   aagaataaat tatttttaaa attaaaagtt gagtcatttg attaaacatg tgattattta   600
   atgaattgat gaaagagttg gattaaagtt gtattagtaa ttagaatttg gtgtcaaatt   660
   taatttgaca tttgatcttt tcctatatat tgccccatag agtcagttaa ctcattttta   720
   tatttcatag atcaaataag agaaataacg gtatattaat ccctccaaaa aaaaaaaacg   780
   gtatatttac taaaaaatct aagccacgta ggaggataac aggatccccg taggaggata   840
   acatccaatc caaccaatca caacaatcct gatgagataa cccactttaa gcccacgcat   900
   ctgtggcaca tctacattat ctaaatcaca cattcttcca cacatctgag ccacacaaaa   960
   accaatccac atctttatca cccattctat aaaaaatcac actttgtgag tctacacttt 1020
   gattcccttc aaacacatac aaagagaaga gactaattaa ttaattaatc atcttgagag 1080
   aaaatggaac gagctataca aggaaacgac gctagggaac aagctaacag tgaacgttgg 1140
   gatggaggat caggaggtac cacttctccc ttcaaacttc ctgacgaaag tccgagttgg 1200
   actgagtggc ggctacataa cgatgagacg aattcgaatc aagataatcc ccttggtttc 1260
   aaggaaagct ggggtttcgg gaaagttgta tttaagagat atctcagata cgacaggacg 1320
   gaagcttcac tgcacagagt ccttggatct tggacgggag attcggttaa ctatgcagca 1380
   tctcgatttt tcggtttcga ccagatcgga tgtacctata gtattcggtt tcgaggagtt 1440
   agtatcaccg tttctggagg gtcgcgaact cttcagcatc tctgtgagat ggcaattcgg 1500
   tctaagcaag aactgctaca gcttgcccca atcgaagtgg aaagtaatgt atcaagagga 1560
   tgccctgaag gtactcaaac cttcgaaaaa gaaagcgagt aagttaaaat gcttcttcgt 1620
   ctcctattta taatatggtt tgttattgtt aattttgttc ttgtagaaga gcttaattaa 1680
   tcgttgttgt tatgaaatac tatttgtatg agatgaactg gtgtaatgta attcatttac 1740
   ataagtggag tcagaatcag aatgtttcct ccataactaa ctagacatga agacctgccg 1800
   cgtacaattg tcttatattt gaacaactaa aattgaacat cttttgccac aactttataa 1860
   gtggttaata tagctcaaat atatggtcaa gttcaataga ttaataatgg aaatatcagt 1920
   tatcgaaatt cattaacaat caacttaacg ttattaacta ctaattttat atcatcccct 1980
   ttgataaatg atagtacacc aattaggaag gagcatgctc gcctaggaga ttgtcgtttc 2040
   ccgccttcag tttgcaagct gctctagccg tgtagccaat acgcaaaccg cctctccccg 2100
   cgcgttggga attactagcg cgtgtcgaca cgcgtggcgc gccctagcag aaggcatgtt 2160
   gttgtgactc cgaggggttg cctcaaactc tatcttataa ccggcgtgga ggcatggagg 2220
   caagggcatt ttggtaattt aagtagttag tggaaaatga cgtcatttac ttaaagacga 2280
   agtcttgcga caaggggggc ccacgccgaa ttttaatatt accggcgtgg ccccacctta 2340
   tcgcgagtgc tttagcacga gcggtccaga tttaaagtag aaaagttccc gcccactagg 2400
   gttaaaggtg ttcacactat aaaagcatat acgatgtgat ggtatttgat aaagcgtata 2460
   ttgtatcagg tatttccgtc ggatacgaat tattcgtaca agcttcttaa gccggtcaac 2520
   atggtggagc acgacacact tgtctactcc aaaaatatca aagatacagt ctcagaagac 2580
   caaagggcaa ttgagacttt tcaacaaagg gtaatatccg gaaacctcct cggattccat 2640
   tgcccagcta tctgtcactt tattgtgaag atagtggaaa aggaaggtgg ctcctacaaa 2700
   tgccatcatt gcgataaagg aaaggccatc gttgaagatg cctctgccga cagtggtccc 2760
   aaagatggac ccccacccac gaggagcatc gtggaaaaag aagacgttcc aaccacgtct 2820
   tcaaagcaag tggattgatg tgataacatg gtggagcacg acacacttgt ctactccaaa 2880
   aatatcaaag atacagtctc agaagaccaa agggcaattg agacttttca acaaagggta 2940
   atatccggaa acctcctcgg attccattgc ccagctatct gtcactttat tgtgaagata 3000
   gtggaaaagg aaggtggctc ctacaaatgc catcattgcg ataaaggaaa ggccatcgtt 3060
   gaagatgcct ctgccgacag tggtcccaaa gatggacccc cacccacgag gagcatcgtg 3120
   gaaaaagaag acgttccaac cacgtcttca aagcaagtgg attgatgtga tatctccact 3180
   gacgtaaggg atgacgcaca atcccactat ccttcgcaag acccttcctc tatataagga 3240
   agttcatttc atttggagag gtattaaaat cttaataggt tttgataaaa gcgaacgtgg 3300
   ggaaacccga accaaacctt cttctaaact ctctctcatc tctcttaaag caaacttctc 3360
   tcttgtcttt cttgcgtgag cgatcttcaa cgttgtcaga tcgtgcttcg gcaccagtac 3420
   aacgttttct ttcactgaag cgaaatcaaa gatctctttg tggacacgta gtgcggcgcc 3480
   attaaataac gtgtacttgt cctattcttg tcggtgtggt cttgggaaaa gaaagcttgc 3540
   tggaggctgc tgttcagccc catacattac ttgttacgat tctgctgact ttcggcgggt 3600
   gcaatatctc tacttctgct tgacgaggta ttgttgcctg tacttctttc ttcttcttct 3660
   tgctgattgg ttctataaga aatctagtat tttctttgaa acagagtttt cccgtggttt 3720
   tcgaacttgg agaaagattg ttaagcttct gtatattctg cccaaatttg tcgggcccgc 3780
   ggctcctcag ccaaaacgac acccccatct gtctatccac tggcccctgg atctgctgcc 3840
   caaactaact ccatggtgac cctgggatgc ctggtcaagg gctatttccc tgagccagtg 3900
   acagtgacct ggaactctgg atccctgtcc agcggtgtgc acaccttccc agctgtcctg 3960
   cagtctgacc tctacactct gagcagctca gtgactgtcc cctccagcac ctggcccagc 4020
   gagaccgtca cctgcaacgt tgcccacccg gccagcagca ccaaggtgga caagaaaatt 4080
   gtgcccaggg attgtggttg taagccttgc atatgtacag tcccagaagt atcatctgtc 4140
   ttcatcttcc ccccaaagcc caaggatgtg ctcaccatta ctctgactcc taaggtcacg 4200
   tgtgttgtgg tagacatcag caaggatgat cccgaggtcc agttcagctg gtttgtagat 4260
   gatgtggagg tgcacacagc tcagacgcaa ccccgggagg agcagttcaa cagcactttc 4320
   cgctcagtca gtgaacttcc catcatgcac caggactggc tcaatggcaa ggaaggccta 4380
   ttttctttag tttgaattta ctgttattcg gtgtgcattt ctatgtttgg tgagcggttt 4440
   tctgtgctca gagtgtgttt attttatgta atttaatttc tttgtgagct cctgtttagc 4500
   aggtcgtccc ttcagcaagg acacaaaaag attttaattt tattaaaaaa aaaaaaaaaa 4560
   aagaccggga attcgatatc aagcttatcg acctgcagat cgttcaaaca tttggcaata 4620
   aagtttctta agattgaatc ctgttgccgg tcttgcgatg attatcatat aatttctgtt 4680
   gaattacgtt aagcatgtaa taattaacat gtaatgcatg acgttattta tgagatgggt 4740
   ttttatgatt agagtcccgc aattatacat ttaatacgcg atagaaaaca aaatatagcg 4800
   cgcaaactag gataaattat cgcgcgcggt gtcatctatg ttactagatc tctagagtct 4860
   caagcttggc gcggggtacc gagctcgaat tccgagtgta cttcaagtca gttggaaatc 4920
   aataaaatga ttattttatg aatatatttc attgtgcaag tagatagaaa ttacatatgt 4980
   tacataacac acgaaataaa caaaaaaaca caatccaaaa caaacacccc aaacaaaata 5040
   acactatata tatcctcgta tgaggagagg cacgttcagt gactcgacga ttcccgagca 5100
   aaaaaagtct ccccgtcaca catatagtgg gtgacgcaat tatcttcaaa gtaatccttc 5160
   tgttgacttg tcattgataa catccagtct tcgtcaggat tgcaaagaat tatagaaggg 5220
   atcccacctt ttattttctt cttttttcca tatttagggt tgacagtgaa atcagactgg 5280
   caacctatta attgcttcca caatgggacg aacttgaagg ggatgtcgtc gatgatatta 5340
   taggtggcgt gttcatcgta gttggtgaag tcgatggtcc cgttccagta gttgtgtcgc 5400
   ccgagacttc tagcccaggt ggtctttccg gtacgagttg gtccgcagat gtagaggctg 5460
   gggtgtctga ccccagtcct tccctcatcc tggttagatc ggccatccac tcaaggtcag 5520
   attgtgcttg atcgtaggag acaggatgta tgaaagtgta ggcatcgatg cttacatgat 5580
   ataggtgcgt ctctctccag ttgtgcagat cttcgtggca gcggagatct gattctgtga 5640
   agggcgacac gtactgctca ggttgtggag gaaataattt gttggctgaa tattccagcc 5700
   attgaagctt tgttgcccat tcatgaggga attcttcttt gatcatgtca agatactcct 5760
   ccttagacgt tgcagtctgg ataatagttc gccatcgtgc gtcagatttg cgaggagaga 5820
   ccttatgatc tcggaaatct cctctggttt taatatctcc gtcctttgat atgtaatcaa 5880
   ggacttgttt agagtttcta gctggctgga tattagggtg atttccttca aaatcgaaaa 5940
   aagaaggatc cctaatacaa ggttttttat caagctggat aagagcatga tagtgggtag 6000
   tgccatcttg atgaagctca gaagcaacac caaggaagaa aataagaaaa ggtgtgagtt 6060
   tctcccagag aaactggaat aaatcatctc tttgagatga gcacttgggg taggtaagga 6120
   aaacatattt agattggagt ctgaagttct tgctagcaga aggcatgttg ttgtgactcc 6180
   gaggggttgc ctcaaactct atcttataac cggcgtggag gcatggaggc aagggcattt 6240
   tggtaattta agtagttagt ggaaaatgac gtcatttact taaagacgaa gtcttgcgac 6300
   aaggggggcc cacgccgaat tttaatatta ccggcgtggc cccaccttat cgcgagtgct 6360
   ttagcacgag cggtccagat ttaaagtaga aaagttcccg cccactaggg ttaaaggtgt 6420
   tcacactata aaagcatata cgatgtgatg gtatttgatg gagcgtatat tgtatcaggt 6480
   atttccgtcg gatacgaatt attcgtacgg ccggccacta gtggcactgg ccgtcgtttt 6540
   acaacgtcgt gactgggaaa accctggcgt tacccaactt aatcgccttg cagcacatcc 6600
   ccctttcgcc agctggcgta atagcgaaga ggcccgcacc gatcgccctt cccaacagtt 6660
   gcgcagcctg aatggcgaat gctagagcag cttgagcttg gatcagattg tcgtttcccg 6720
   ccttcagttt aaactatcag tgtttgacag gatatattgg cgggtaaacc taagagaaaa 6780
   gagcgttta 6789
<210> 8
   <211> 4379
   <212> DNA
   <213> Artificial Sequence
<220>
   <223> Expression cassette number 1086
<400> 8
   ctagcagaag gcatgttgtt gtgactccga ggggttgcct caaactctat cttataaccg 60
   gcgtggaggc atggaggcaa gggcattttg gtaatttaag tagttagtgg aaaatgacgt 120
   catttactta aagacgaagt cttgcgacaa ggggggccca cgccgaattt taatattacc 180
   ggcgtggccc caccttatcg cgagtgcttt agcacgagcg gtccagattt aaagtagaaa 240
   agttcccgcc cactagggtt aaaggtgttc acactataaa agcatatacg atgtgatggt 300
   atttgataaa gcgtatattg tatcaggtat ttccgtcgga tacgaattat tcgtacaagc 360
   ttcttaagcc ggtcaacatg gtggagcacg acacacttgt ctactccaaa aatatcaaag 420
   atacagtctc agaagaccaa agggcaattg agacttttca acaaagggta atatccggaa 480
   acctcctcgg attccattgc ccagctatct gtcactttat tgtgaagata gtggaaaagg 540
   aaggtggctc ctacaaatgc catcattgcg ataaaggaaa ggccatcgtt gaagatgcct 600
   ctgccgacag tggtcccaaa gatggacccc cacccacgag gagcatcgtg gaaaaagaag 660
   acgttccaac cacgtcttca aagcaagtgg attgatgtga taacatggtg gagcacgaca 720
   cacttgtcta ctccaaaaat atcaaagata cagtctcaga agaccaaagg gcaattgaga 780
   cttttcaaca aagggtaata tccggaaacc tcctcggatt ccattgccca gctatctgtc 840
   actttattgt gaagatagtg gaaaaggaag gtggctccta caaatgccat cattgcgata 900
   aaggaaaggc catcgttgaa gatgcctctg ccgacagtgg tcccaaagat ggacccccac 960
   ccacgaggag catcgtggaa aaagaagacg ttccaaccac gtcttcaaag caagtggatt 1020
   gatgtgatat ctccactgac gtaagggatg acgcacaatc ccactatcct tcgcaagacc 1080
   cttcctctat ataaggaagt tcatttcatt tggagaggta ttaaaatctt aataggtttt 1140
   gataaaagcg aacgtgggga aacccgaacc aaaccttctt ctaaactctc tctcatctct 1200
   cttaaagcaa acttctctct tgtctttctt gcgtgagcga tcttcaacgt tgtcagatcg 1260
   tgcttcggca ccagtacaac gttttctttc actgaagcga aatcaaagat ctctttgtgg 1320
   acacgtagtg cggcgccatt aaataacgtg tacttgtcct attcttgtcg gtgtggtctt 1380
   gggaaaagaa agcttgctgg aggctgctgt tcagccccat acattacttg ttacgattct 1440
   gctgactttc ggcgggtgca atatctctac ttctgcttga cgaggtattg ttgcctgtac 1500
   ttctttcttc ttcttcttgc tgattggttc tataagaaat ctagtatttt ctttgaaaca 1560
   gagttttccc gtggttttcg aacttggaga aagattgtta agcttctgta tattctgccc 1620
   aaatttgtcg ggcccatgaa ctttctacgt aagatagtga aaaattgcag ggacgaggac 1680
   actcaaaaac cctctcccgt gtcagcccct ctggatgacg atgacttgtg gcttccaccc 1740
   cctgaatacg tcccgctgaa agaacttaca agcaagaaga acatgaggaa cttttgtatc 1800
   aacggagggg ttaaagtgtg tagcccgaat ggttactcgt tcaggatcct gcggcacatt 1860
   ctgaaatcat tcgacgagat atattctggg aatcatagga tgatcgggtt agtcaaagta 1920
   gttattggac tggctttgtc aggatctcca gtccctgagg gcatgaactg ggtatacaaa 1980
   ttgaggagaa cctttatctt ccagtgggct gattccaggg gccctcttga aggggaggag 2040
   ttggaatact ctcaggagat cacttgggat gatgatactg agttcgtcgg attgcaaata 2100
   agagtgattg caaaacagtg tcatatccag ggcagaatct ggtgtatcaa catgaacccg 2160
   agagcatgtc aactatggtc tgacatgtct cttcagacac aaaggtccga agaggacaaa 2220
   gattcctctc tgcttctaga ataaaggcct attttcttta gtttgaattt actgttattc 2280
   ggtgtgcatt tctatgtttg gtgagcggtt ttctgtgctc agagtgtgtt tattttatgt 2340
   aatttaattt ctttgtgagc tcctgtttag caggtcgtcc cttcagcaag gacacaaaaa 2400
   gattttaatt ttattaaaaa aaaaaaaaaa aaagaccggg aattcgatat caagcttatc 2460
   gacctgcaga tcgttcaaac atttggcaat aaagtttctt aagattgaat cctgttgccg 2520
   gtcttgcgat gattatcata taatttctgt tgaattacgt taagcatgta ataattaaca 2580
   tgtaatgcat gacgttattt atgagatggg tttttatgat tagagtcccg caattataca 2640
   tttaatacgc gatagaaaac aaaatatagc gcgcaaacta ggataaatta tcgcgcgcgg 2700
   tgtcatctat gttactagat ctctagagtc tcaagcttgg cgcggggtac cgagctcgaa 2760
   ttccgagtgt acttcaagtc agttggaaat caataaaatg attattttat gaatatattt 2820
   cattgtgcaa gtagatagaa attacatatg ttacataaca cacgaaataa acaaaaaaac 2880
   acaatccaaa acaaacaccc caaacaaaat aacactatat atatcctcgt atgaggagag 2940
   gcacgttcag tgactcgacg attcccgagc aaaaaaagtc tccccgtcac acatatagtg 3000
   ggtgacgcaa ttatcttcaa agtaatcctt ctgttgactt gtcattgata acatccagtc 3060
   ttcgtcagga ttgcaaagaa ttatagaagg gatcccacct tttattttct tcttttttcc 3120
   atatttaggg ttgacagtga aatcagactg gcaacctatt aattgcttcc acaatgggac 3180
   gaacttgaag gggatgtcgt cgatgatatt ataggtggcg tgttcatcgt agttggtgaa 3240
   gtcgatggtc ccgttccagt agttgtgtcg cccgagactt ctagcccagg tggtctttcc 3300
   ggtacgagtt ggtccgcaga tgtagaggct ggggtgtctg accccagtcc ttccctcatc 3360
   ctggttagat cggccatcca ctcaaggtca gattgtgctt gatcgtagga gacaggatgt 3420
   atgaaagtgt aggcatcgat gcttacatga tataggtgcg tctctctcca gttgtgcaga 3480
   tcttcgtggc agcggagatc tgattctgtg aagggcgaca cgtactgctc aggttgtgga 3540
   ggaaataatt tgttggctga atattccagc cattgaagct ttgttgccca ttcatgaggg 3600
   aattcttctt tgatcatgtc aagatactcc tccttagacg ttgcagtctg gataatagtt 3660
   cgccatcgtg cgtcagattt gcgaggagag accttatgat ctcggaaatc tcctctggtt 3720
   ttaatatctc cgtcctttga tatgtaatca aggacttgtt tagagtttct agctggctgg 3780
   atattagggt gatttccttc aaaatcgaaa aaagaaggat ccctaataca aggtttttta 3840
   tcaagctgga taagagcatg atagtgggta gtgccatctt gatgaagctc agaagcaaca 3900
   ccaaggaaga aaataagaaa aggtgtgagt ttctcccaga gaaactggaa taaatcatct 3960
   ctttgagatg agcacttggg gtaggtaagg aaaacatatt tagattggag tctgaagttc 4020
   ttgctagcag aaggcatgtt gttgtgactc cgaggggttg cctcaaactc tatcttataa 4080
   ccggcgtgga ggcatggagg caagggcatt ttggtaattt aagtagttag tggaaaatga 4140
   cgtcatttac ttaaagacga agtcttgcga caaggggggc ccacgccgaa ttttaatatt 4200
   accggcgtgg ccccacctta tcgcgagtgc tttagcacga gcggtccaga tttaaagtag 4260
   aaaagttccc gcccactagg gttaaaggtg ttcacactat aaaagcatat acgatgtgat 4320
   ggtatttgat ggagcgtata ttgtatcagg tatttccgtc ggatacgaat tattcgtac 4379
<210> 9
   <211> 42
   <212> DNA
   <213> Artificial Sequence
<220>
   <223> IF-RabG-S2+4.c
<400> 9
   tctcagatct tcgccaaatt ccctatttac acgataccag ac 42
<210> 10
   <211> 48
   <212> DNA
   <213> Artificial Sequence
<220>
   <223> IF-RabG-Sl-4.r
<400> 10
   actaaagaaa ataggccttc acagtctggt ctcaccccca ctcttgtg 48
<210> 11
   <211> 1575
   <212> DNA
   <213> Artificial Sequence
<220>
   <223> Synthesized Rabies G protein coding sequence
<400> 11
   atggttcctc aggctctcct gtttgtaccc cttctggttt ttccattgtg ttttgggaaa 60
   ttccctattt acacgatacc agacaagctt ggtccctgga gcccgattga catacatcac 120
   ctcagctgcc caaacaattt ggtagtggag gacgaaggat gcaccaacct gtcagggttc 180
   tcctacatgg aacttaaagt tggatacatc ttagccataa aaatgaacgg gttcacttgc 240
   acaggcgttg tgacggaggc tgaaacctac actaacttcg ttggttatgt cacaaccacg 300
   ttcaaaagaa agcatttccg cccaacacca gatgcatgta gagccgcgta caactggaag 360
   atggccggtg accccagata tgaagagtct ctacacaatc cgtaccctga ctaccgctgg 420
   cttcgaactg taaaaaccac caaggagtct ctcgttatca tatctccaag tgtggcagat 480
   ttggacccat atgacagatc ccttcactcg agggtcttcc ctagcgggaa gtgctcagga 540
   gtagcggtgt cttctaccta ctgctccact aaccacgatt acaccatttg gatgcccgag 600
   aatccgagac tagggatgtc ttgtgacatt tttaccaata gtagagggaa gagagcatcc 660
   aaagggagtg agacttgcgg ctttgtagat gaaagaggcc tatataagtc tttaaaagga 720
   gcatgcaaac tcaagttatg tggagttcta ggacttagac ttatggatgg aacatgggtc 780
   gcgatgcaaa catcaaatga aaccaaatgg tgccctcccg atcagttggt gaacctgcac 840
   gactttcgct cagacgaaat tgagcacctt gttgtagagg agttggtcag gaagagagag 900
   gagtgtctgg atgcactaga gtccatcatg acaaccaagt cagtgagttt cagacgtctc 960
   agtcatttaa gaaaacttgt ccctgggttt ggaaaagcat ataccatatt caacaagacc 1020
   ttgatggaag ccgatgctca ctacaagtca gtcagaactt ggaatgagat cctcccttca 1080
   aaagggtgtt taagagttgg ggggaggtgt catcctcatg tgaacggggt gtttttcaat 1140
   ggtataatat taggacctga cggcaatgtc ttaatcccag agatgcaatc atccctcctc 1200
   cagcaacata tggagttgtt ggaatcctcg gttatccccc ttgtgcaccc cctggcagac 1260
   ccgtctaccg ttttcaagga cggtgacgag gctgaggatt ttgttgaagt tcaccttccc 1320
   gatgtgcaca atcaggtctc aggagttgac ttgggtctcc cgaactgggg gaagtatgta 1380
   ttactgagtg caggggccct gactgccttg atgttgataa ttttcctgat gacatgttgt 1440
   agaagagtca atcgatcaga acctacgcaa cacaatctca gagggacagg gagggaggtg 1500
   tcagtcactc cccaaagcgg gaagatcata tcttcatggg aatcacacaa gagtgggggt 1560
   gagaccagac tgtga 1575
<210> 12
   <211> 4897
   <212> DNA
   <213> Artificial Sequence
<220>
   <223> Construct number 1192
<400> 12
   tggcaggata tattgtggtg taaacaaatt gacgcttaga caacttaata acacattgcg      60
   gacgttttta atgtactgaa ttaacgccga atcccgggct ggtatattta tatgttgtca     120
   aataactcaa aaaccataaa agtttaagtt agcaagtgtg tacattttta cttgaacaaa     180
   aatattcacc tactactgtt ataaatcatt attaaacatt agagtaaaga aatatggatg     240
   ataagaacaa gagtagtgat attttgacaa caattttgtt gcaacatttg agaaaatttt     300
   gttgttctct cttttcattg gtcaaaaaca atagagagag aaaaaggaag agggagaata     360
   aaaacataat gtgagtatga gagagaaagt tgtacaaaag ttgtaccaaa atagttgtac     420
   aaatatcatt gaggaatttg acaaaagcta cacaaataag ggttaattgc tgtaaataaa     480
   taaggatgac gcattagaga gatgtaccat tagagaattt ttggcaagtc attaaaaaga     540
   aagaataaat tatttttaaa attaaaagtt gagtcatttg attaaacatg tgattattta     600
   atgaattgat gaaagagttg gattaaagtt gtattagtaa ttagaatttg gtgtcaaatt     660
   taatttgaca tttgatcttt tcctatatat tgccccatag agtcagttaa ctcattttta     720
   tatttcatag atcaaataag agaaataacg gtatattaat ccctccaaaa aaaaaaaacg     780
   gtatatttac taaaaaatct aagccacgta ggaggataac aggatccccg taggaggata     840
   acatccaatc caaccaatca caacaatcct gatgagataa cccactttaa gcccacgcat     900
   ctgtggcaca tctacattat ctaaatcaca cattcttcca cacatctgag ccacacaaaa     960
   accaatccac atctttatca cccattctat aaaaaatcac actttgtgag tctacacttt    1020
   gattcccttc aaacacatac aaagagaaga gactaattaa ttaattaatc atcttgagag    1080
   aaaatggaac gagctataca aggaaacgac gctagggaac aagctaacag tgaacgttgg    1140
   gatggaggat caggaggtac cacttctccc ttcaaacttc ctgacgaaag tccgagttgg    1200
   actgagtggc ggctacataa cgatgagacg aattcgaatc aagataatcc ccttggtttc    1260
   aaggaaagct ggggtttcgg gaaagttgta tttaagagat atctcagata cgacaggacg    1320
   gaagcttcac tgcacagagt ccttggatct tggacgggag attcggttaa ctatgcagca    1380
   tctcgatttt tcggtttcga ccagatcgga tgtacctata gtattcggtt tcgaggagtt    1440
   agtatcaccg tttctggagg gtcgcgaact cttcagcatc tctgtgagat ggcaattcgg    1500
   tctaagcaag aactgctaca gcttgcccca atcgaagtgg aaagtaatgt atcaagagga    1560
   tgccctgaag gtactcaaac cttcgaaaaa gaaagcgagt aagttaaaat gcttcttcgt    1620
   ctcctattta taatatggtt tgttattgtt aattttgttc ttgtagaaga gcttaattaa 1680
   tcgttgttgt tatgaaatac tatttgtatg agatgaactg gtgtaatgta attcatttac 1740
   ataagtggag tcagaatcag aatgtttcct ccataactaa ctagacatga agacctgccg 1800
   cgtacaattg tcttatattt gaacaactaa aattgaacat cttttgccac aactttataa 1860
   gtggttaata tagctcaaat atatggtcaa gttcaataga ttaataatgg aaatatcagt 1920
   tatcgaaatt cattaacaat caacttaacg ttattaacta ctaattttat atcatcccct 1980
   ttgataaatg atagtacacc aattaggaag gagcatgctc gcctaggaga ttgtcgtttc 2040
   ccgccttcag tttgcaagct gctctagccg tgtagccaat acgcaaaccg cctctccccg 2100
   cgcgttggga attactagcg cgtgtcgaca agcttgcatg ccggtcaaca tggtggagca 2160
   cgacacactt gtctactcca aaaatatcaa agatacagtc tcagaagacc aaagggcaat 2220
   tgagactttt caacaaaggg taatatccgg aaacctcctc ggattccatt gcccagctat 2280
   ctgtcacttt attgtgaaga tagtggaaaa ggaaggtggc tcctacaaat gccatcattg 2340
   cgataaagga aaggccatcg ttgaagatgc ctctgccgac agtggtccca aagatggacc 2400
   cccacccacg aggagcatcg tggaaaaaga agacgttcca accacgtctt caaagcaagt 2460
   ggattgatgt gataacatgg tggagcacga cacacttgtc tactccaaaa atatcaaaga 2520
   tacagtctca gaagaccaaa gggcaattga gacttttcaa caaagggtaa tatccggaaa 2580
   cctcctcgga ttccattgcc cagctatctg tcactttatt gtgaagatag tggaaaagga 2640
   aggtggctcc tacaaatgcc atcattgcga taaaggaaag gccatcgttg aagatgcctc 2700
   tgccgacagt ggtcccaaag atggaccccc acccacgagg agcatcgtgg aaaaagaaga 2760
   cgttccaacc acgtcttcaa agcaagtgga ttgatgtgat atctccactg acgtaaggga 2820
   tgacgcacaa tcccactatc cttcgcaaga cccttcctct atataaggaa gttcatttca 2880
   tttggagagg tattaaaatc ttaataggtt ttgataaaag cgaacgtggg gaaacccgaa 2940
   ccaaaccttc ttctaaactc tctctcatct ctcttaaagc aaacttctct cttgtctttc 3000
   ttgcgtgagc gatcttcaac gttgtcagat cgtgcttcgg caccagtaca acgttttctt 3060
   tcactgaagc gaaatcaaag atctctttgt ggacacgtag tgcggcgcca ttaaataacg 3120
   tgtacttgtc ctattcttgt cggtgtggtc ttgggaaaag aaagcttgct ggaggctgct 3180
   gttcagcccc atacattact tgttacgatt ctgctgactt tcggcgggtg caatatctct 3240
   acttctgctt gacgaggtat tgttgcctgt acttctttct tcttcttctt gctgattggt 3300
   tctataagaa atctagtatt ttctttgaaa cagagttttc ccgtggtttt cgaacttgga 3360
   gaaagattgt taagcttctg tatattctgc ccaaatttgt cgggcccatg gcgaaaaacg 3420
   ttgcgatttt cggcttattg ttttctcttc ttgtgttggt tccttctcag atcttcgccg 3480
   cggctcctca gccaaaacga cacccccatc tgtctatcca ctggcccctg gatctgctgc 3540
   ccaaactaac tccatggtga ccctgggatg cctggtcaag ggctatttcc ctgagccagt 3600
   gacagtgacc tggaactctg gatccctgtc cagcggtgtg cacaccttcc cagctgtcct 3660
   gcagtctgac ctctacactc tgagcagctc agtgactgtc ccctccagca cctggcccag 3720
   cgagaccgtc acctgcaacg ttgcccaccc ggccagcagc accaaggtgg acaagaaaat 3780
   tgtgcccagg gattgtggtt gtaagccttg catatgtaca gtcccagaag tatcatctgt 3840
   cttcatcttc cccccaaagc ccaaggatgt gctcaccatt actctgactc ctaaggtcac 3900
   gtgtgttgtg gtagacatca gcaaggatga tcccgaggtc cagttcagct ggtttgtaga 3960
   tgatgtggag gtgcacacag ctcagacgca accccgggag gagcagttca acagcacttt 4020
   ccgctcagtc agtgaacttc ccatcatgca ccaggactgg ctcaatggca aggagcgatc 4080
   gctcaccatc accatcacca tcaccatcac cattaaaggc ctattttctt tagtttgaat 4140
   ttactgttat tcggtgtgca tttctatgtt tggtgagcgg ttttctgtgc tcagagtgtg 4200
   tttattttat gtaatttaat ttctttgtga gctcctgttt agcaggtcgt cccttcagca 4260
   aggacacaaa aagattttaa ttttattaaa aaaaaaaaaa aaaaagaccg ggaattcgat 4320
   atcaagctta tcgacctgca gatcgttcaa acatttggca ataaagtttc ttaagattga 4380
   atcctgttgc cggtcttgcg atgattatca tataatttct gttgaattac gttaagcatg 4440
   taataattaa catgtaatgc atgacgttat ttatgagatg ggtttttatg attagagtcc 4500
   cgcaattata catttaatac gcgatagaaa acaaaatata gcgcgcaaac taggataaat 4560
   tatcgcgcgc ggtgtcatct atgttactag atctctagag tctcaagctt ggcgcgccca 4620
   cgtgactagt ggcactggcc gtcgttttac aacgtcgtga ctgggaaaac cctggcgtta 4680
   cccaacttaa tcgccttgca gcacatcccc ctttcgccag ctggcgtaat agcgaagagg 4740
   cccgcaccga tcgcccttcc caacagttgc gcagcctgaa tggcgaatgc tagagcagct 4800
   tgagcttgga tcagattgtc gtttcccgcc ttcagtttaa actatcagtg tttgacagga 4860
   tatattggcg ggtaaaccta agagaaaaga gcgttta 4897
<210> 13
   <211> 3330
   <212> DNA
   <213> Artificial Sequence
<220>
   <223> Expression cassette No. 1071
<400> 13
   gtcaacatgg tggagcacga cacacttgtc tactccaaaa atatcaaaga tacagtctca 60
   gaagaccaaa gggcaattga gacttttcaa caaagggtaa tatccggaaa cctcctcgga 120
   ttccattgcc cagctatctg tcactttatt gtgaagatag tggaaaagga aggtggctcc 180
   tacaaatgcc atcattgcga taaaggaaag gccatcgttg aagatgcctc tgccgacagt 240
   ggtcccaaag atggaccccc acccacgagg agcatcgtgg aaaaagaaga cgttccaacc 300
   acgtcttcaa agcaagtgga ttgatgtgat aacatggtgg agcacgacac acttgtctac 360
   tccaaaaata tcaaagatac agtctcagaa gaccaaaggg caattgagac ttttcaacaa 420
   agggtaatat ccggaaacct cctcggattc cattgcccag ctatctgtca ctttattgtg 480
   aagatagtgg aaaaggaagg tggctcctac aaatgccatc attgcgataa aggaaaggcc 540
   atcgttgaag atgcctctgc cgacagtggt cccaaagatg gacccccacc cacgaggagc 600
   atcgtggaaa aagaagacgt tccaaccacg tcttcaaagc aagtggattg atgtgatatc 660
   tccactgacg taagggatga cgcacaatcc cactatcctt cgcaagaccc ttcctctata 720
   taaggaagtt catttcattt ggagaggtat taaaatctta ataggttttg ataaaagcga 780
   acgtggggaa acccgaacca aaccttcttc taaactctct ctcatctctc ttaaagcaaa 840
   cttctctctt gtctttcttg cgtgagcgat cttcaacgtt gtcagatcgt gcttcggcac 900
   cagtacaacg ttttctttca ctgaagcgaa atcaaagatc tctttgtgga cacgtagtgc 960
   ggcgccatta aataacgtgt acttgtccta ttcttgtcgg tgtggtcttg ggaaaagaaa 1020
   gcttgctgga ggctgctgtt cagccccata cattacttgt tacgattctg ctgactttcg 1080
   gcgggtgcaa tatctctact tctgcttgac gaggtattgt tgcctgtact tctttcttct 1140
   tcttcttgct gattggttct ataagaaatc tagtattttc tttgaaacag agttttcccg 1200
   tggttttcga acttggagaa agattgttaa gcttctgtat attctgccca aatttgtcgg 1260
   gcccatggcg aaaaacgttg cgattttcgg cttattgttt tctcttcttg tgttggttcc 1320
   ttctcagatc ttcgccaaat tccctattta cacgatacca gacaagcttg gtccctggag 1380
   cccgattgac atacatcacc tcagctgccc aaacaatttg gtagtggagg acgaaggatg 1440
   caccaacctg tcagggttct cctacatgga acttaaagtt ggatacatct tagccataaa 1500
   aatgaacggg ttcacttgca caggcgttgt gacggaggct gaaacctaca ctaacttcgt 1560
   tggttatgtc acaaccacgt tcaaaagaaa gcatttccgc ccaacaccag atgcatgtag 1620
   agccgcgtac aactggaaga tggccggtga ccccagatat gaagagtctc tacacaatcc 1680
   gtaccctgac taccgctggc ttcgaactgt aaaaaccacc aaggagtctc tcgttatcat 1740
   atctccaagt gtggcagatt tggacccata tgacagatcc cttcactcga gggtcttccc 1800
   tagcgggaag tgctcaggag tagcggtgtc ttctacctac tgctccacta accacgatta 1860
   caccatttgg atgcccgaga atccgagact agggatgtct tgtgacattt ttaccaatag 1920
   tagagggaag agagcatcca aagggagtga gacttgcggc tttgtagatg aaagaggcct 1980
   atataagtct ttaaaaggag catgcaaact caagttatgt ggagttctag gacttagact 2040
   tatggatgga acatgggtcg cgatgcaaac atcaaatgaa accaaatggt gccctcccga 2100
   tcagttggtg aacctgcacg actttcgctc agacgaaatt gagcaccttg ttgtagagga 2160
   gttggtcagg aagagagagg agtgtctgga tgcactagag tccatcatga caaccaagtc 2220
   agtgagtttc agacgtctca gtcatttaag aaaacttgtc cctgggtttg gaaaagcata 2280
   taccatattc aacaagacct tgatggaagc cgatgctcac tacaagtcag tcagaacttg 2340
   gaatgagatc ctcccttcaa aagggtgttt aagagttggg gggaggtgtc atcctcatgt 2400
   gaacggggtg tttttcaatg gtataatatt aggacctgac ggcaatgtct taatcccaga 2460
   gatgcaatca tccctcctcc agcaacatat ggagttgttg gaatcctcgg ttatccccct 2520
   tgtgcacccc ctggcagacc cgtctaccgt tttcaaggac ggtgacgagg ctgaggattt 2580
   tgttgaagtt caccttcccg atgtgcacaa tcaggtctca ggagttgact tgggtctccc 2640
   gaactggggg aagtatgtat tactgagtgc aggggccctg actgccttga tgttgataat 2700
   tttcctgatg acatgttgta gaagagtcaa tcgatcagaa cctacgcaac acaatctcag 2760
   agggacaggg agggaggtgt cagtcactcc ccaaagcggg aagatcatat cttcatggga 2820
   atcacacaag agtgggggtg agaccagact gtgaaggcct attttcttta gtttgaattt 2880
   actgttattc ggtgtgcatt tctatgtttg gtgagcggtt ttctgtgctc agagtgtgtt 2940
   tattttatgt aatttaattt ctttgtgagc tcctgtttag caggtcgtcc cttcagcaag 3000
   gacacaaaaa gattttaatt ttattaaaaa aaaaaaaaaa aaagaccggg aattcgatat 3060
   caagcttatc gacctgcaga tcgttcaaac atttggcaat aaagtttctt aagattgaat 3120
   cctgttgccg gtcttgcgat gattatcata taatttctgt tgaattacgt taagcatgta 3180
   ataattaaca tgtaatgcat gacgttattt atgagatggg tttttatgat tagagtcccg 3240
   caattataca tttaatacgc gatagaaaac aaaatatagc gcgcaaacta ggataaatta 3300
   tcgcgcgcgg tgtcatctat gttactagat 3330
<210> 14
   <211> 529
   <212> PRT
   <213> Rabies virus
<400> 14
   Met Ala Lys Asn Val Ala Ile Phe Gly Leu Leu Phe Ser Leu Leu Val
   1               5                   10                  15
   Leu Val Pro Ser Gln Ile Phe Ala Lys Phe Pro Ile Tyr Thr Ile Pro
               20                  25                  30
   Asp Lys Leu Gly Pro Trp Ser Pro Ile Asp Ile His His Leu Ser Cys
           35                  40                  45
   Pro Asn Asn Leu Val Val Glu Asp Glu Gly Cys Thr Asn Leu Ser Gly
       50                  55                  60
   Phe Ser Tyr Met Glu Leu Lys Val Gly Tyr Ile Leu Ala Ile Lys Met
   65                  70                  75                  80
   Asn Gly Phe Thr Cys Thr Gly Val Val Thr Glu Ala Glu Thr Tyr Thr
                   85                  90                  95
   Asn Phe Val Gly Tyr Val Thr Thr Thr Phe Lys Arg Lys His Phe Arg
               100                 105                 110
   Pro Thr Pro Asp Ala Cys Arg Ala Ala Tyr Asn Trp Lys Met Ala Gly
           115                 120                 125
   Asp Pro Arg Tyr Glu Glu Ser Leu His Asn Pro Tyr Pro Asp Tyr Arg
       130                 135                 140
   Trp Leu Arg Thr Val Lys Thr Thr Lys Glu Ser Leu Val Ile Ile Ser
   145                 150                 155                 160
   Pro Ser Val Ala Asp Leu Asp Pro Tyr Asp Arg Ser Leu His Ser Arg
                   165                 170                 175
   Val Phe Pro Ser Gly Lys Cys Ser Gly Val Ala Val Ser Ser Thr Tyr
               180                 185                 190
   Cys Ser Thr Asn His Asp Tyr Thr Ile Trp Met Pro Glu Asn Pro Arg
           195                 200                 205
   Leu Gly Met Ser Cys Asp Ile Phe Thr Asn Ser Arg Gly Lys Arg Ala
       210                 215                 220
   Ser Lys Gly Ser Glu Thr Cys Gly Phe Val Asp Glu Arg Gly Leu Tyr
   225                 230                 235                 240
   Lys Ser Leu Lys Gly Ala Cys Lys Leu Lys Leu Cys Gly Val Leu Gly
                   245                 250                 255
   Leu Arg Leu Met Asp Gly Thr Trp Val Ala Met Gln Thr Ser Asn Glu
               260                 265                 270
   Thr Lys Trp Cys Pro Pro Asp Gln Leu Val Asn Leu His Asp Phe Arg
           275                 280                 285
   Ser Asp Glu Ile Glu His Leu Val Val Glu Glu Leu Val Arg Lys Arg
       290                 295                 300
   Glu Glu Cys Leu Asp Ala Leu Glu Ser Ile Met Thr Thr Lys Ser Val
   305                 310                 315                 320
   Ser Phe Arg Arg Leu Ser His Leu Arg Lys Leu Val Pro Gly Phe Gly
                   325                 330                 335
   Lys Ala Tyr Thr Ile Phe Asn Lys Thr Leu Met Glu Ala Asp Ala His
               340                 345                 350
   Tyr Lys Ser Val Arg Thr Trp Asn Glu Ile Leu Pro Ser Lys Gly Cys
           355                 360                 365
   Leu Arg Val Gly Gly Arg Cys His Pro His Val Asn Gly Val Phe Phe
       370                 375                 380
   Asn Gly Ile Ile Leu Gly Pro Asp Gly Asn Val Leu Ile Pro Glu Met
   385                 390                 395                 400
   Gln Ser Ser Leu Leu Gln Gln His Met Glu Leu Leu Glu Ser Ser Val
                   405                 410                 415
   Ile Pro Leu Val His Pro Leu Ala Asp Pro Ser Thr Val Phe Lys Asp
               420                 425                 430
   Gly Asp Glu Ala Glu Asp Phe Val Glu Val His Leu Pro Asp Val His
           435                 440                 445
   Asn Gln Val Ser Gly Val Asp Leu Gly Leu Pro Asn Trp Gly Lys Tyr
       450                 455                 460
   Val Leu Leu Ser Ala Gly Ala Leu Thr Ala Leu Met Leu Ile Ile Phe
   465                 470                 475                 480
   Leu Met Thr Cys Cys Arg Arg Val Asn Arg Ser Glu Pro Thr Gln His
                   485                 490                 495
   Asn Leu Arg Gly Thr Gly Arg Glu Val Ser Val Thr Pro Gln Ser Gly
               500                 505                 510
   Lys Ile Ile Ser Ser Trp Glu Ser His Lys Ser Gly Gly Glu Thr Arg
           515                 520                 525
   Leu
<210> 15
   <211> 6861
   <212> DNA
   <213> Artificial Sequence
<220>
   <223> Construct No. 1194
<400> 15
   tggcaggata tattgtggtg taaacaaatt gacgcttaga caacttaata acacattgcg     60
   gacgttttta atgtactgaa ttaacgccga atcccgggct ggtatattta tatgttgtca    120
   aataactcaa aaaccataaa agtttaagtt agcaagtgtg tacattttta cttgaacaaa    180
   aatattcacc tactactgtt ataaatcatt attaaacatt agagtaaaga aatatggatg    240
   ataagaacaa gagtagtgat attttgacaa caattttgtt gcaacatttg agaaaatttt    300
   gttgttctct cttttcattg gtcaaaaaca atagagagag aaaaaggaag agggagaata    360
   aaaacataat gtgagtatga gagagaaagt tgtacaaaag ttgtaccaaa atagttgtac    420
   aaatatcatt gaggaatttg acaaaagcta cacaaataag ggttaattgc tgtaaataaa    480
   taaggatgac gcattagaga gatgtaccat tagagaattt ttggcaagtc attaaaaaga    540
   aagaataaat tatttttaaa attaaaagtt gagtcatttg attaaacatg tgattattta    600
   atgaattgat gaaagagttg gattaaagtt gtattagtaa ttagaatttg gtgtcaaatt    660
   taatttgaca tttgatcttt tcctatatat tgccccatag agtcagttaa ctcattttta    720
   tatttcatag atcaaataag agaaataacg gtatattaat ccctccaaaa aaaaaaaacg    780
   gtatatttac taaaaaatct aagccacgta ggaggataac aggatccccg taggaggata    840
   acatccaatc caaccaatca caacaatcct gatgagataa cccactttaa gcccacgcat    900
   ctgtggcaca tctacattat ctaaatcaca cattcttcca cacatctgag ccacacaaaa    960
   accaatccac atctttatca cccattctat aaaaaatcac actttgtgag tctacacttt   1020
   gattcccttc aaacacatac aaagagaaga gactaattaa ttaattaatc atcttgagag   1080
   aaaatggaac gagctataca aggaaacgac gctagggaac aagctaacag tgaacgttgg   1140
   gatggaggat caggaggtac cacttctccc ttcaaacttc ctgacgaaag tccgagttgg   1200
   actgagtggc ggctacataa cgatgagacg aattcgaatc aagataatcc ccttggtttc   1260
   aaggaaagct ggggtttcgg gaaagttgta tttaagagat atctcagata cgacaggacg   1320
   gaagcttcac tgcacagagt ccttggatct tggacgggag attcggttaa ctatgcagca   1380
   tctcgatttt tcggtttcga ccagatcgga tgtacctata gtattcggtt tcgaggagtt   1440
   agtatcaccg tttctggagg gtcgcgaact cttcagcatc tctgtgagat ggcaattcgg   1500
   tctaagcaag aactgctaca gcttgcccca atcgaagtgg aaagtaatgt atcaagagga   1560
   tgccctgaag gtactcaaac cttcgaaaaa gaaagcgagt aagttaaaat gcttcttcgt   1620
   ctcctattta taatatggtt tgttattgtt aattttgttc ttgtagaaga gcttaattaa   1680
   tcgttgttgt tatgaaatac tatttgtatg agatgaactg gtgtaatgta attcatttac 1740
   ataagtggag tcagaatcag aatgtttcct ccataactaa ctagacatga agacctgccg 1800
   cgtacaattg tcttatattt gaacaactaa aattgaacat cttttgccac aactttataa 1860
   gtggttaata tagctcaaat atatggtcaa gttcaataga ttaataatgg aaatatcagt 1920
   tatcgaaatt cattaacaat caacttaacg ttattaacta ctaattttat atcatcccct 1980
   ttgataaatg atagtacacc aattaggaag gagcatgctc gcctaggaga ttgtcgtttc 2040
   ccgccttcag tttgcaagct gctctagccg tgtagccaat acgcaaaccg cctctccccg 2100
   cgcgttggga attactagcg cgtgtcgaca cgcgtggcgc gccctagcag aaggcatgtt 2160
   gttgtgactc cgaggggttg cctcaaactc tatcttataa ccggcgtgga ggcatggagg 2220
   caagggcatt ttggtaattt aagtagttag tggaaaatga cgtcatttac ttaaagacga 2280
   agtcttgcga caaggggggc ccacgccgaa ttttaatatt accggcgtgg ccccacctta 2340
   tcgcgagtgc tttagcacga gcggtccaga tttaaagtag aaaagttccc gcccactagg 2400
   gttaaaggtg ttcacactat aaaagcatat acgatgtgat ggtatttgat aaagcgtata 2460
   ttgtatcagg tatttccgtc ggatacgaat tattcgtaca agcttcttaa gccggtcaac 2520
   atggtggagc acgacacact tgtctactcc aaaaatatca aagatacagt ctcagaagac 2580
   caaagggcaa ttgagacttt tcaacaaagg gtaatatccg gaaacctcct cggattccat 2640
   tgcccagcta tctgtcactt tattgtgaag atagtggaaa aggaaggtgg ctcctacaaa 2700
   tgccatcatt gcgataaagg aaaggccatc gttgaagatg cctctgccga cagtggtccc 2760
   aaagatggac ccccacccac gaggagcatc gtggaaaaag aagacgttcc aaccacgtct 2820
   tcaaagcaag tggattgatg tgataacatg gtggagcacg acacacttgt ctactccaaa 2880
   aatatcaaag atacagtctc agaagaccaa agggcaattg agacttttca acaaagggta 2940
   atatccggaa acctcctcgg attccattgc ccagctatct gtcactttat tgtgaagata 3000
   gtggaaaagg aaggtggctc ctacaaatgc catcattgcg ataaaggaaa ggccatcgtt 3060
   gaagatgcct ctgccgacag tggtcccaaa gatggacccc cacccacgag gagcatcgtg 3120
   gaaaaagaag acgttccaac cacgtcttca aagcaagtgg attgatgtga tatctccact 3180
   gacgtaaggg atgacgcaca atcccactat ccttcgcaag acccttcctc tatataagga 3240
   agttcatttc atttggagag gtattaaaat cttaataggt tttgataaaa gcgaacgtgg 3300
   ggaaacccga accaaacctt cttctaaact ctctctcatc tctcttaaag caaacttctc 3360
   tcttgtcttt cttgcgtgag cgatcttcaa cgttgtcaga tcgtgcttcg gcaccagtac 3420
   aacgttttct ttcactgaag cgaaatcaaa gatctctttg tggacacgta gtgcggcgcc 3480
   attaaataac gtgtacttgt cctattcttg tcggtgtggt cttgggaaaa gaaagcttgc 3540
   tggaggctgc tgttcagccc catacattac ttgttacgat tctgctgact ttcggcgggt 3600
   gcaatatctc tacttctgct tgacgaggta ttgttgcctg tacttctttc ttcttcttct 3660
   tgctgattgg ttctataaga aatctagtat tttctttgaa acagagtttt cccgtggttt 3720
   tcgaacttgg agaaagattg ttaagcttct gtatattctg cccaaatttg tcgggcccat 3780
   ggcgaaaaac gttgcgattt tcggcttatt gttttctctt cttgtgttgg ttccttctca 3840
   gatcttcgcc gcggctcctc agccaaaacg acacccccat ctgtctatcc actggcccct 3900
   ggatctgctg cccaaactaa ctccatggtg accctgggat gcctggtcaa gggctatttc 3960
   cctgagccag tgacagtgac ctggaactct ggatccctgt ccagcggtgt gcacaccttc 4020
   ccagctgtcc tgcagtctga cctctacact ctgagcagct cagtgactgt cccctccagc 4080
   acctggccca gcgagaccgt cacctgcaac gttgcccacc cggccagcag caccaaggtg 4140
   gacaagaaaa ttgtgcccag ggattgtggt tgtaagcctt gcatatgtac agtcccagaa 4200
   gtatcatctg tcttcatctt ccccccaaag cccaaggatg tgctcaccat tactctgact 4260
   cctaaggtca cgtgtgttgt ggtagacatc agcaaggatg atcccgaggt ccagttcagc 4320
   tggtttgtag atgatgtgga ggtgcacaca gctcagacgc aaccccggga ggagcagttc 4380
   aacagcactt tccgctcagt cagtgaactt cccatcatgc accaggactg gctcaatggc 4440
   aaggaaggcc tattttcttt agtttgaatt tactgttatt cggtgtgcat ttctatgttt 4500
   ggtgagcggt tttctgtgct cagagtgtgt ttattttatg taatttaatt tctttgtgag 4560
   ctcctgttta gcaggtcgtc ccttcagcaa ggacacaaaa agattttaat tttattaaaa 4620
   aaaaaaaaaa aaaagaccgg gaattcgata tcaagcttat cgacctgcag atcgttcaaa 4680
   catttggcaa taaagtttct taagattgaa tcctgttgcc ggtcttgcga tgattatcat 4740
   ataatttctg ttgaattacg ttaagcatgt aataattaac atgtaatgca tgacgttatt 4800
   tatgagatgg gtttttatga ttagagtccc gcaattatac atttaatacg cgatagaaaa 4860
   caaaatatag cgcgcaaact aggataaatt atcgcgcgcg gtgtcatcta tgttactaga 4920
   tctctagagt ctcaagcttg gcgcggggta ccgagctcga attccgagtg tacttcaagt 4980
   cagttggaaa tcaataaaat gattatttta tgaatatatt tcattgtgca agtagataga 5040
   aattacatat gttacataac acacgaaata aacaaaaaaa cacaatccaa aacaaacacc 5100
   ccaaacaaaa taacactata tatatcctcg tatgaggaga ggcacgttca gtgactcgac 5160
   gattcccgag caaaaaaagt ctccccgtca cacatatagt gggtgacgca attatcttca 5220
   aagtaatcct tctgttgact tgtcattgat aacatccagt cttcgtcagg attgcaaaga 5280
   attatagaag ggatcccacc ttttattttc ttcttttttc catatttagg gttgacagtg 5340
   aaatcagact ggcaacctat taattgcttc cacaatggga cgaacttgaa ggggatgtcg 5400
   tcgatgatat tataggtggc gtgttcatcg tagttggtga agtcgatggt cccgttccag 5460
   tagttgtgtc gcccgagact tctagcccag gtggtctttc cggtacgagt tggtccgcag 5520
   atgtagaggc tggggtgtct gaccccagtc cttccctcat cctggttaga tcggccatcc 5580
   actcaaggtc agattgtgct tgatcgtagg agacaggatg tatgaaagtg taggcatcga 5640
   tgcttacatg atataggtgc gtctctctcc agttgtgcag atcttcgtgg cagcggagat 5700
   ctgattctgt gaagggcgac acgtactgct caggttgtgg aggaaataat ttgttggctg 5760
   aatattccag ccattgaagc tttgttgccc attcatgagg gaattcttct ttgatcatgt 5820
   caagatactc ctccttagac gttgcagtct ggataatagt tcgccatcgt gcgtcagatt 5880
   tgcgaggaga gaccttatga tctcggaaat ctcctctggt tttaatatct ccgtcctttg 5940
   atatgtaatc aaggacttgt ttagagtttc tagctggctg gatattaggg tgatttcctt 6000
   caaaatcgaa aaaagaagga tccctaatac aaggtttttt atcaagctgg ataagagcat 6060
   gatagtgggt agtgccatct tgatgaagct cagaagcaac accaaggaag aaaataagaa 6120
   aaggtgtgag tttctcccag agaaactgga ataaatcatc tctttgagat gagcacttgg 6180
   ggtaggtaag gaaaacatat ttagattgga gtctgaagtt cttgctagca gaaggcatgt 6240
   tgttgtgact ccgaggggtt gcctcaaact ctatcttata accggcgtgg aggcatggag 6300
   gcaagggcat tttggtaatt taagtagtta gtggaaaatg acgtcattta cttaaagacg 6360
   aagtcttgcg acaagggggg cccacgccga attttaatat taccggcgtg gccccacctt 6420
   atcgcgagtg ctttagcacg agcggtccag atttaaagta gaaaagttcc cgcccactag 6480
   ggttaaaggt gttcacacta taaaagcata tacgatgtga tggtatttga tggagcgtat 6540
   attgtatcag gtatttccgt cggatacgaa ttattcgtac ggccggccac tagtggcact 6600
   ggccgtcgtt ttacaacgtc gtgactggga aaaccctggc gttacccaac ttaatcgcct 6660
   tgcagcacat ccccctttcg ccagctggcg taatagcgaa gaggcccgca ccgatcgccc 6720
   ttcccaacag ttgcgcagcc tgaatggcga atgctagagc agcttgagct tggatcagat 6780
   tgtcgtttcc cgccttcagt ttaaactatc agtgtttgac aggatatatt ggcgggtaaa 6840
   cctaagagaa aagagcgttt a 6861
<210> 16
   <211> 5360
   <212> DNA
   <213> Artificial Sequence
<220>
   <223> Expression cassette number 1091
<400> 16
   ctagcagaag gcatgttgtt gtgactccga ggggttgcct caaactctat cttataaccg 60
   gcgtggaggc atggaggcaa gggcattttg gtaatttaag tagttagtgg aaaatgacgt 120
   catttactta aagacgaagt cttgcgacaa gggggcccca cgccgaattt taatattacc 180
   ggcgtggccc caccttatcg cgagtgcttt agcacgagcg gtccagattt aaagtagaaa 240
   agttcccgcc cactagggtt aaaggtgttc acactataaa agcatatacg atgtgatggt 300
   atttgataaa gcgtatattg tatcaggtat ttccgtcgga tacgaattat tcgtacaagc 360
   ttcttaagcc ggtcaacatg gtggagcacg acacacttgt ctactccaaa aatatcaaag 420
   atacagtctc agaagaccaa agggcaattg agacttttca acaaagggta atatccggaa 480
   acctcctcgg attccattgc ccagctatct gtcactttat tgtgaagata gtggaaaagg 540
   aaggtggctc ctacaaatgc catcattgcg ataaaggaaa ggccatcgtt gaagatgcct 600
   ctgccgacag tggtcccaaa gatggacccc cacccacgag gagcatcgtg gaaaaagaag 660
   acgttccaac cacgtcttca aagcaagtgg attgatgtga taacatggtg gagcacgaca 720
   cacttgtcta ctccaaaaat atcaaagata cagtctcaga agaccaaagg gcaattgaga 780
   cttttcaaca aagggtaata tccggaaacc tcctcggatt ccattgccca gctatctgtc 840
   actttattgt gaagatagtg gaaaaggaag gtggctccta caaatgccat cattgcgata 900
   aaggaaaggc catcgttgaa gatgcctctg ccgacagtgg tcccaaagat ggacccccac 960
   ccacgaggag catcgtggaa aaagaagacg ttccaaccac gtcttcaaag caagtggatt 1020
   gatgtgatat ctccactgac gtaagggatg acgcacaatc ccactatcct tcgcaagacc 1080
   cttcctctat ataaggaagt tcatttcatt tggagaggta ttaaaatctt aataggtttt 1140
   gataaaagcg aacgtgggga aacccgaacc aaaccttctt ctaaactctc tctcatctct 1200
   cttaaagcaa acttctctct tgtctttctt gcgtgagcga tcttcaacgt tgtcagatcg 1260
   tgcttcggca ccagtacaac gttttctttc actgaagcga aatcaaagat ctctttgtgg 1320
   acacgtagtg cggcgccatt aaataacgtg tacttgtcct attcttgtcg gtgtggtctt 1380
   gggaaaagaa agcttgctgg aggctgctgt tcagccccat acattacttg ttacgattct 1440
   gctgactttc ggcgggtgca atatctctac ttctgcttga cgaggtattg ttgcctgtac 1500
   ttctttcttc ttcttcttgc tgattggttc tataagaaat ctagtatttt ctttgaaaca 1560
   gagttttccc gtggttttcg aacttggaga aagattgtta agcttctgta tattctgccc 1620
   aaatttgtcg ggcccatggc gaaaaacgtt gcgattttcg gcttattgtt ttctcttctt 1680
   gtgttggttc cttctcagat cttcgccaaa ttccctattt acacgatacc agacaagctt 1740
   ggtccctgga gcccgattga catacatcac ctcagctgcc caaacaattt ggtagtggag 1800
   gacgaaggat gcaccaacct gtcagggttc tcctacatgg aacttaaagt tggatacatc 1860
   ttagccataa aaatgaacgg gttcacttgc acaggcgttg tgacggaggc tgaaacctac 1920
   actaacttcg ttggttatgt cacaaccacg ttcaaaagaa agcatttccg cccaacacca 1980
   gatgcatgta gagccgcgta caactggaag atggccggtg accccagata tgaagagtct 2040
   ctacacaatc cgtaccctga ctaccgctgg cttcgaactg taaaaaccac caaggagtct 2100
   ctcgttatca tatctccaag tgtggcagat ttggacccat atgacagatc ccttcactcg 2160
   agggtcttcc ctagcgggaa gtgctcagga gtagcggtgt cttctaccta ctgctccact 2220
   aaccacgatt acaccatttg gatgcccgag aatccgagac tagggatgtc ttgtgacatt 2280
   tttaccaata gtagagggaa gagagcatcc aaagggagtg agacttgcgg ctttgtagat 2340
   gaaagaggcc tatataagtc tttaaaagga gcatgcaaac tcaagttatg tggagttcta 2400
   ggacttagac ttatggatgg aacatgggtc gcgatgcaaa catcaaatga aaccaaatgg 2460
   tgccctcccg atcagttggt gaacctgcac gactttcgct cagacgaaat tgagcacctt 2520
   gttgtagagg agttggtcag gaagagagag gagtgtctgg atgcactaga gtccatcatg 2580
   acaaccaagt cagtgagttt cagacgtctc agtcatttaa gaaaacttgt ccctgggttt 2640
   ggaaaagcat ataccatatt caacaagacc ttgatggaag ccgatgctca ctacaagtca 2700
   gtcagaactt ggaatgagat cctcccttca aaagggtgtt taagagttgg ggggaggtgt 2760
   catcctcatg tgaacggggt gtttttcaat ggtataatat taggacctga cggcaatgtc 2820
   ttaatcccag agatgcaatc atccctcctc cagcaacata tggagttgtt ggaatcctcg 2880
   gttatccccc ttgtgcaccc cctggcagac ccgtctaccg ttttcaagga cggtgacgag 2940
   gctgaggatt ttgttgaagt tcaccttccc gatgtgcaca atcaggtctc aggagttgac 3000
   ttgggtctcc cgaactgggg gaagtatgta ttactgagtg caggggccct gactgccttg 3060
   atgttgataa ttttcctgat gacatgttgt agaagagtca atcgatcaga acctacgcaa 3120
   cacaatctca gagggacagg gagggaggtg tcagtcactc cccaaagcgg gaagatcata 3180
   tcttcatggg aatcacacaa gagtgggggt gagaccagac tgtgaaggcc tattttcttt 3240
   agtttgaatt tactgttatt cggtgtgcat ttctatgttt ggtgagcggt tttctgtgct 3300
   cagagtgtgt ttattttatg taatttaatt tctttgtgag ctcctgttta gcaggtcgtc 3360
   ccttcagcaa ggacacaaaa agattttaat tttattaaaa aaaaaaaaaa aaaagaccgg 3420
   gaattcgata tcaagcttat cgacctgcag atcgttcaaa catttggcaa taaagtttct 3480
   taagattgaa tcctgttgcc ggtcttgcga tgattatcat ataatttctg ttgaattacg 3540
   ttaagcatgt aataattaac atgtaatgca tgacgttatt tatgagatgg gtttttatga 3600
   ttagagtccc gcaattatac atttaatacg cgatagaaaa caaaatatag cgcgcaaact 3660
   aggataaatt atcgcgcgcg gtgtcatcta tgttactaga tctctagagt ctcaagcttg 3720
   gcgcggggta ccgagctcga attccgagtg tacttcaagt cagttggaaa tcaataaaat 3780
   gattatttta tgaatatatt tcattgtgca agtagataga aattacatat gttacataac 3840
   acacgaaata aacaaaaaaa cacaatccaa aacaaacacc ccaaacaaaa taacactata 3900
   tatatcctcg tatgaggaga ggcacgttca gtgactcgac gattcccgag caaaaaaagt     3960
   ctccccgtca cacatatagt gggtgacgca attatcttca aagtaatcct tctgttgact     4020
   tgtcattgat aacatccagt cttcgtcagg attgcaaaga attatagaag ggatcccacc     4080
   ttttattttc ttcttttttc catatttagg gttgacagtg aaatcagact ggcaacctat     4140
   taattgcttc cacaatggga cgaacttgaa ggggatgtcg tcgatgatat tataggtggc     4200
   gtgttcatcg tagttggtga agtcgatggt cccgttccag tagttgtgtc gcccgagact     4260
   tctagcccag gtggtctttc cggtacgagt tggtccgcag atgtagaggc tggggtgtct     4320
   gaccccagtc cttccctcat cctggttaga tcggccatcc actcaaggtc agattgtgct     4380
   tgatcgtagg agacaggatg tatgaaagtg taggcatcga tgcttacatg atataggtgc     4440
   gtctctctcc agttgtgcag atcttcgtgg cagcggagat ctgattctgt gaagggcgac     4500
   acgtactgct caggttgtgg aggaaataat ttgttggctg aatattccag ccattgaagc     4560
   tttgttgccc attcatgagg gaattcttct ttgatcatgt caagatactc ctccttagac     4620
   gttgcagtct ggataatagt tcgccatcgt gcgtcagatt tgcgaggaga gaccttatga     4680
   tctcggaaat ctcctctggt tttaatatct ccgtcctttg atatgtaatc aaggacttgt     4740
   ttagagtttc tagctggctg gatattaggg tgatttcctt caaaatcgaa aaaagaagga     4800
   tccctaatac aaggtttttt atcaagctgg ataagagcat gatagtgggt agtgccatct     4860
   tgatgaagct cagaagcaac accaaggaag aaaataagaa aaggtgtgag tttctcccag     4920
   agaaactgga ataaatcatc tctttgagat gagcacttgg ggtaggtaag gaaaacatat     4980
   ttagattgga gtctgaagtt cttgctagca gaaggcatgt tgttgtgact ccgaggggtt     5040
   gcctcaaact ctatcttata accggcgtgg aggcatggag gcaagggcat tttggtaatt     5100
   taagtagtta gtggaaaatg acgtcattta cttaaagacg aagtcttgcg acaagggggg     5160
   cccacgccga attttaatat taccggcgtg gccccacctt atcgcgagtg ctttagcacg     5220
   agcggtccag atttaaagta gaaaagttcc cgcccactag ggttaaaggt gttcacacta     5280
   taaaagcata tacgatgtga tggtatttga tggagcgtat attgtatcag gtatttccgt     5340
   cggatacgaa ttattcgtac                                                 5360